# EUROPEAN PATENT APPLICATION

(11) **EP 4 813 381 A1**
(43) Date of publication of application: **30.09.2026**
(21) Application number: 24893315.2
(22) Date of filing: 14.11.2024
(51) Int. Cl.: A61K 31/365, A61P 35/00, A61P 29/00, A61P 1/16, A61P 31/04, A61P 31/12, A61P 13/12, A61P 3/10, A61P 17/00, A61K 31/40, A61K 31/4453, A61K 31/397, A61K 31/496, A61K 31/5377, A61K 31/55, A61K 31/438, A61K 31/4025, A61K 31/4525, A61K 31/454, A61K 31/453

(54) **PHARMACEUTICAL USE OF GUAIANE SESQUITERPENE DERIVATIVE**

(30) Priority: 24.11.2023 CN 202311592223; 19.04.2024 CN 202410477196; 19.04.2024 CN 202410477201
(71) Applicant: Nanjing University Of Chinese Medicine, Nanjing, Jiangsu 210023 (CN)
(72) Inventor: HU, Lihong, Nanjing, Jiangsu 210023 (CN); LIU, Jian, Nanjing, Jiangsu 210023 (CN); LV, Qi, Nanjing, Jiangsu 210023 (CN); KANG, Di, Nanjing, Jiangsu 210023 (CN); ZHU, Yujie, Nanjing, Jiangsu 210023 (CN); LIN, Weijiang, Nanjing, Jiangsu 210023 (CN); ZHENG, Zhe, Nanjing, Jiangsu 210023 (CN); WANG, Ping, Nanjing, Jiangsu 210023 (CN); CUI, Zhenzhen, Nanjing, Jiangsu 210023 (CN); SUN, Yu, Nanjing, Jiangsu 210023 (CN); XIE, Ying, Nanjing, Jiangsu 210023 (CN); WANG, Kuimao, Nanjing, Jiangsu 210023 (CN)
(74) Representative: Murgitroyd & Company
(86) International application number: PCT/CN2024/131911
(87) International publication number: WO 2025/108157

(57) **Abstract**

The present invention discloses the use of guaiacol sesquiterpene lactone derivatives or their pharmaceutically acceptable salts in the preparation of drugs for treating inflammatory diseases or tumors, or for soothing the skin. The present invention use chrysanthemum lactone as a starting material to prepare a class of guaiacol sesquiterpene lactone derivatives, prodrugs, or their pharmaceutically acceptable salts and compositions. Experimental validation demonstrates their effective therapeutic effects on inflammatory diseases such as acute lung injury, hepatitis, lupus nephritis, atopic dermatitis, and sepsis caused by bacterial and/or viral infections, as well as tumors.

## Description

### Technical Field

The present invention relates to the pharmaceutical use of guaiacol sesquiterpene lactone derivatives, specifically to the use of guaiacol sesquiterpene lactone derivatives and the pharmaceutically acceptable salts thereof in the treatment of inflammatory diseases or tumors.

### Background

Inflammatory diseases constitute a broad category of conditions characterized by tissue damage resulting from excessive or persistent inflammatory responses. They have emerged as a significant global health threat, encompassing conditions such as sepsis, rheumatoid arthritis, acute lung injury, non-alcoholic steatohepatitis, and drug-induced hepatitis. These diseases are spreading worldwide and have evolved into major public health challenges that severely jeopardize human health and socioeconomic sustainability. Consequently, they are receiving increasing attention from governments and communities globally. In China, the prevalence of such diseases is approximately 10%, affecting over 100 million patients. These conditions are often chronic, with high rates of complications and disability, severely impacting human health and life. The exact causes of inflammatory diseases remain incompletely understood, though immune dysregulation is currently recognized as the primary culprit behind these uncontrolled inflammatory responses. Clinically, treatments for inflammatory diseases primarily include nonsteroidal anti-inflammatory drugs (NSAIDs), glucocorticoids, immunosuppressants, and biologics. Long-term use of these medications can lead to multiple complications (such as osteoporosis and adrenal insufficiency), increased susceptibility to infections (e.g., gastrointestinal infections), and heightened cancer risks (e.g., lymphoma and melanoma). Additionally, biologic therapies carry substantial costs, imposing significant financial burdens on patients. Consequently, there is an urgent need to explore novel drugs or more effective treatment strategies.

Malignant tumors have long posed a serious threat to human health and life. Statistics indicate that at least 15-20% of cancer deaths worldwide are associated with persistent infections and chronic inflammation. Chronic inflammation ranks among the ten hallmark features of tumors, with the abundant inflammatory cells present in the tumor microenvironment playing a crucial role in tumor initiation and progression.

Natural products serve as a vital source of therapeutic agents for inflammatory diseases and malignant tumors. Among these, guaianic sesquiterpene lactones stand out as significant natural compounds exhibiting potent anti-inflammatory and antitumor activities. Arglabin (Arg), a guaiacol-type sesquiterpene lactone derived from Artemisia absinthium (wormwood) in Kazakhstan, has been reported to exhibit potent anti-inflammatory and antitumor activities. In a rat arthritis model, Arg significantly suppressed joint swelling, reduced the arthritis index, alleviated inflammatory lesions in ankle tissues along with cartilage and bone damage, lowered serum levels of pro-inflammatory factors TNF-α, IL-1β, and IL-6, ultimately achieving marked relief of rat arthritis. In clinical trials involving heart failure patients, agrabin inhibited cardiac inflammation during the mid-stage of myocardial hypertrophy, protected cardiac function, and reduced fibrosis [Circulation, 2020, 141: 1704-1719]. Concurrently, studies report Arg's antitumor effects by inhibiting farnesyltransferase (FTase) to block H-Ras protein farnesylation. However, in vitro experiments indicate Arg's IC₅₀ for inhibiting H-Ras protein farnesylation is only at the micromolar level. This mechanism cannot fully account for its in vivo antitumor efficacy, necessitating further optimization for tumor indications. Furthermore, Arg exhibits low natural abundance, approximately 0.27% [J Nat Prop, 1999, 62: 1068-1071]; its aqueous solubility is only 7.9 µg/mL; and it exhibits poor chemical stability in gastric fluid, with a degradation rate reaching 50% within 8 hours and oral bioavailability as low as 5%. These drawbacks-including low natural abundance and poor drug-like properties-limit Arg's clinical application, and its suitability for anti-inflammatory indications remains unconfirmed.

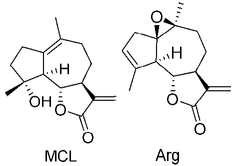

Another guaiacol-type sesquiterpene lactone, Micheliolide (MCL), is distributed in the root bark of Magnolia grandiflora and Michelia taiwanensis, and can also be synthesized from parthenolide. Modern pharmacological studies indicate that MCL exhibits significant anti-inflammatory effects. Furthermore, in vitro cell models report that MCL inhibits tumor cell growth by exerting cytotoxic effects, particularly targeting tumor stem cells, with an IC50 in the micromolar range. MCL promotes dendritic cell (DC) maturation, presenting DAMPs signals to immune cells such as T and B cells to mediate immunogenic cell death. However, its limited ability to activate T cells through DC maturation results in suboptimal anti-HCC activity. In vivo pharmacodynamic studies on HCC showed that a single dose of MCL (30 mg/kg) administered via intraperitoneal injection for 14 consecutive days achieved only a 50% tumor weight inhibition rate in the Hepa1-6 transplanted tumor model. Furthermore, in immunocompetent mice implanted with human-derived HepG2 and Huh7 HCC cell transplanted tumor models, the effective dose for intraperitoneal administration reached as high as 25 mg/kg.

Therefore, new derivatives with optimized structures, enhanced activity, and improved druggability are needed in the fields of treating inflammatory diseases and malignant tumors.

### Invention Summary

The present invention provides, in a first aspect, the use of guaiacol sesquiterpene lactone derivatives or their pharmaceutically acceptable salts, solvates, or stereoisomers in the preparation of antitumor drugs. Preferably, the tumors are hepatocellular carcinoma, colorectal cancer, or lymphoma.

A second aspect of the present invention provides for the use of guaiacol sesquiterpene lactone derivatives or their pharmaceutically acceptable salts, solvates, or stereoisomers in the preparation of drugs for treating inflammatory diseases or soothing the skin. Preferably, the inflammatory diseases are selected from (1) acute lung injury; (2) hepatitis; (3) sepsis caused by bacterial and/or viral infections; (4) acute and chronic kidney diseases; (5) renal fibrosis; (6) lupus nephritis; (7) diabetic nephropathy; (8) atopic dermatitis.

Preferably, the skin soothing is achieved by increasing skin hyaluronic acid levels and/or inhibiting mast cell degranulation.

In the first and second aspects of the present invention, the structure of the guaiacol sesquiterpene lactone derivative is shown by the general formula I:

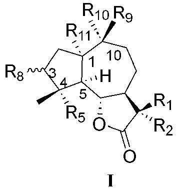

Wherein, R₁ and R₂ together form a double bond; or R₁ is hydrogen or deuterium, and R₂ is 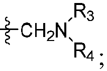 wherein R₃ and R₄ are each an alkyl group, or R₃, R₄, and the N atom to which they are attached form a 3- to 10-membered ring structure;
R₅ is selected from hydroxy, C1-C6 alkoxy, C1-C6 alkyl ester, or halogen, and - OCONR₆R₇, wherein R₆ is hydrogen or C1-C6 alkyl; R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, OH-(CH₂)n-O-substituted C1-C6 alkyl, amino-substituted C1-C6 alkyl, 5-6-membered nitrogen-containing heterocycle- substituted C1-C6 alkyl, or benzene ring- substituted C1-C6 alkyl; wherein the 5- to 6-membered nitrogen-containing heterocycle or benzene ring may optionally be substituted by one or more of C1-C6 alkyl; or R₆, R₇, and the N atom to which they are attached may form a 3- to 10-membered cyclic structure; or the 3rd position carbon atom may be bonded to the 4th position carbon atom to form a double bond; or the 4th position carbon atom may be bonded to the 5th position carbon atom to form a double bond;
n is selected from 1, 2, 3, 4, 5, or 6;
R₈ is hydrogen or hydroxyl;
R₉ is C1-C6 alkyl, R₁₀ and R₁₁ forming a cyclopropane; or R₉ is C1-C6 alkyl, 1st position carbon atom bonded to the 10th position carbon atom forming a double bond;

In some preferred embodiments, when R₉ is C1-C6 alkyl and the 1st carbon atom is bonded to the 10th carbon atom to form a double bond, R₅ is not a hydroxyl group.

In some preferred embodiments, R₁ and R₂ together form the double bond;

In some preferred embodiments, R₁ is hydrogen;

In some preferred embodiments, R₂ is 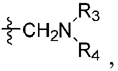 and R₃ and R₄ are each independently selected from C1-, C2-, C3-, C4-, C5-, C6-, C7-, C8-, C9-, or C10-alkyl;

In some preferred embodiments, R₂ is 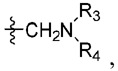 and R₃ and R₄ are each independently selected from C1-C6 alkyl;

In some preferred embodiments, R₂ is 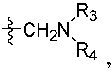 and R₃ and R₄ are each independently selected from methyl, ethyl, n-propyl, isopropyl, n-butyl, isobutyl; more preferably, R₃ and R₄ are both methyl;

In some preferred embodiments, R₂ is 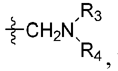 wherein R₃, R₄, and the N atom to which they are attached form a 3-, 4-, 5-, 6-, 7-, 8-, 9-, or 10-membered ring structure;

In some preferred embodiments, R₂ is 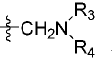 wherein R₃, R₄, and the N atom to which they are attached form a 5- to 8-membered ring structure;

In some preferred embodiments, R₂ is 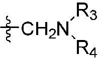 such that R₃, R₄, and the N atom to which they are attached form a 5- to 6-membered ring structure;

In some preferred embodiments, R₂ is 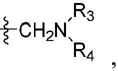 R₃, R₄, and the N atom to which they are attached form a 4- to 6-membered ring structure;

In the above preferred embodiments, R₃, R₄, and the N atom to which they are attached form a monocyclic, spirocyclic, or fused ring structure;

In some preferred embodiments, R₂ is 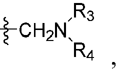 R₃, R₄, and the N atom to which they are attached form a ring structure, the ring structure is selected from azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

In the above-described preferred embodiments, the ring structure formed by R₃, R₄, and the N atom to which they are bonded is substituted by one or more substituents; preferably, said substituents are selected from C1-C6 alkyl, 5-6-membered nitrogen-containing heterocycle groups, and C1-C6 alkoxycarbonyl groups (e.g., ethoxycarbonyl). Further preferably, the 5- to 6-membered nitrogen-containing heterocyclic groups are selected from tetrahydropyrolidyl, piperidyl, piperazine, or morpholinyl.

In some preferred embodiments, R₅ is a hydroxyl group;
In some preferred embodiments, R₅ is selected from C1-C6 alkoxy or C1-C6 alkyl ester groups;
In some preferred embodiments, R₅ is selected from fluorine, chlorine, bromine, or iodine.

In some preferred embodiments, R₅ is -OCONR₆R₇, where R₆ is hydrogen and R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, amino-substituted C1-C6 alkyl, 5-6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by one or more C1-C6 alkyl groups.
In some preferred embodiments, R₅ is -OCONR₆R₇, where R₆ is C1-C6 alkyl and R₇ is C1-C6 alkyl;
In some preferred embodiments, R₅ is -OCONR₆R₇, wherein R₆, R₇, and the N atom to which they are attached form a 3-, 4-, 5-, 6-, 7-, 8-, 9-, or 10-membered ring structure;
In some preferred embodiments, R₅ is -OCONR₆R₇, wherein R₆, R₇, and the N atom to which they are bonded form a 5- or 6-membered ring structure;
In some preferred embodiments, R₅ is -OCONR₆R₇, wherein R₆, R₇, and the N atom to which they are attached form a 5- or 6-membered ring structure selected from tetrahydropyrole, piperidine, piperazine, and morpholine.

In the above-described preferred embodiments, the ring structure formed by R₆, R₇, and the connecting nitrogen atom is substituted by one or more substituents; Preferably, said substituents are selected from C1-C6 alkyl groups and 5-6-membered nitrogen-containing heterocyclic groups; further preferably, said 5-6-membered nitrogen-containing heterocyclic groups are selected from tetrahydropyrolidyl, piperidyl, piperazinyl, or morpholinyl groups.

In some preferred embodiments, the 3rd carbon atom is bonded to the 4th carbon atom to form a double bond; or the 4th carbon atom is bonded to the 5th carbon atom to form a double bond;
In some preferred embodiments, R₈ is hydrogen;
In some preferred embodiments, R₉ is methyl and R₁₀ and R₁₁ are connected to form a cyclopropane ring;
In some preferred embodiments, R₉ is methyl, and the 1st carbon atom is bonded to the 10th carbon atom to form a double bond.

In the first and second aspects of the present invention, the structure of the guaiacol sesquiterpene lactone derivative is shown by the general formula II: 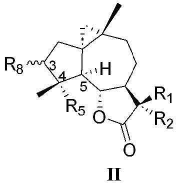
R₁ and R₂ together form a double bond;
or R₁ is hydrogen or deuterium, R₂ is 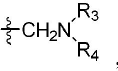 wherein R₃ and R₄ are each an alkyl group, or R₃, R₄, and N atom to which they attached form a 3- to 10-membered ring structure,
R₅ is a hydroxyl group, a C1-C6 alkoxy group, a C1-C6 alkyl ester group, or halogen; or the 3rd position carbon atom is bonded to the 4th position carbon atom to form a double bond; or the 4th position carbon atom is bonded to the 5th position carbon atom to form a double bond;
R₈ is hydrogen or a hydroxyl group.

In some preferred embodiments, R₁ and R₂ together form a double bond;
In some preferred embodiments, R₂ is 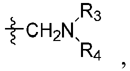 and R₃ and R₄ are each independently selected from C1, C2, C3, C4, C5, C6, C7, C8, C9, or C10 alkyl;
In some preferred embodiments, R₂ is 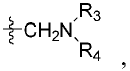 and R₃ and R₄ are each independently selected from C1-C6 alkyl;
In some preferred embodiments, R₂ is 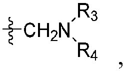 and R₃ and R₄ are each independently selected from methyl, ethyl, n-propyl, isopropyl, n-butyl, isobutyl; more preferably, R₃ and R₄ are both methyl;
In some preferred embodiments, R₂ is 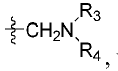 wherein R₃, R₄, and the N atom to which they are bonded form a 3-, 4-, 5-, 6-, 7-, 8-, 9-, or 10-membered ring structure;
In some preferred embodiments, R₂ is 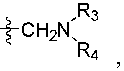 wherein R₃, R₄, and the N atom to which they are bonded form a 5- to 8-membered ring structure;
In some preferred embodiments, R₂ is 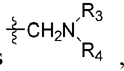 wherein R₃, R₄, and the N atom to which they are bonded form a 4- to 6-membered ring structure;
In some preferred embodiments, R₂ is 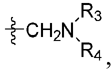 wherein R₃, R₄, and the N atom to which they are bonded form a 5- to 6-membered ring structure;
In the above preferred embodiments, the ring structure formed by R₃, R₄, and the N atom to which they are bonded is a monocyclic, spirocyclic, or fused ring structure;
In the above preferred embodiments, R₃, R₄, and the N atom to which they are attached form a ring structure, the ring structure is selected from azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

In the above preferred embodiments, the ring structure formed by R₃, R₄, and the N atom to which they are attached is substituted by one or more substituents; preferably, said substituents are selected from C1-C6 alkyl, 5-6-membered nitrogen-containing heterocyclic groups, and C1-C6 alkoxycarbonyl, further preferably, the 5-6-membered nitrogen-containing heterocyclic groups are selected from tetrahydropyrrole, piperidine, piperazine, or morpholine.
In some preferred embodiments, R₅ is a hydroxyl group;
In some preferred embodiments, R₈ is hydrogen;
In the first and second aspects of the present invention, the guaiacol-type sesquiterpene lactone derivative is represented by Formula III: 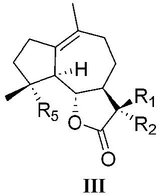
R₁ and R₂ together form a double bond;
or R₁ is hydrogen or deuterium, and R₂ is 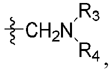 wherein R₃ and R₄ are each an alkyl group, or R₃, R₄, and an N atom form a 3- to 10-membered ring structure;
R₅ is selected from hydroxy, C1-C6 alkoxy, C1-C6 alkyl ester, or halogen, and - OCONR₆R₇, wherein R₆ is hydrogen or C1-C6 alkyl; R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, OH-(CH₂)n-O-substituted C1-C6 alkyl, amino-substituted C1-C6 alkyl, 5-6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by one or more C1-C6 alkyl groups, or R₆, R₇, and the connecting nitrogen atom may form a 3- to 10-membered ring structure;
n is selected from 1, 2, 3, 4, 5, or 6.

In some preferred embodiments, R₁ and R₂ together form a double bond;
In some preferred embodiments, R₁ is hydrogen;
In some preferred embodiments, R₂ is 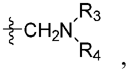 and R₃ and R₄ are each independently selected from C1, C2, C3, C4, C5, C6, C7, C8, C9, or C10 alkyl;
In some preferred embodiments, R₂ is 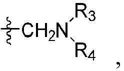 and R₃ and R₄ are each independently selected from C1-C6 alkyl;
In some preferred embodiments, R₂ is 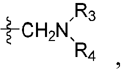 and R₃ and R₄ are each independently selected from methyl, ethyl, n-propyl, isopropyl, n-butyl, isobutyl; more preferably, R₃ and R₄ are both methyl;
In some preferred embodiments, R₂ is 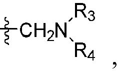 wherein R₃, R₄, and the N atom to which they are bonded form a 3-, 4-, 5-, 6-, 7-, 8-, 9-, or 10-membered ring structure;
In some preferred embodiments, R₂ is 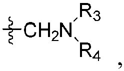 wherein R₃, R₄, and the N atom to which they are bonded form a 5- to 8-membered ring structure;
In some preferred embodiments, R₂ is 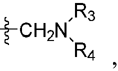 wherein R₃, R₄, and the N atom to which they are bonded form a 5- to 6-membered ring structure;
In some preferred embodiments, R₂ is 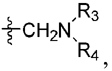 wherein R₃, R₄, and the N atom to which they are bonded form a 4- to 6-membered ring structure;
In the above-described preferred embodiments, R₃, R₄, and the N atom to which they are bonded form a monocyclic, spirocyclic, or fused ring structure;
In some preferred embodiments, R₂ is 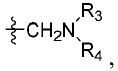 wherein the ring structure formed by R₃, R₄, and the N atom to which they are bonded is selected from azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

In the above preferred embodiments, the ring structure formed by R₃, R₄, and the N atom to which they are bonded is substituted by one or more substituents; preferably, said substituents are selected from C1-C6 alkyl, 5-6-membered nitrogen-containing heterocyclic groups, C1-C6 alkoxycarbonyl (e.g., ethoxycarbonyl); Further preferably, the 5- to 6-membered nitrogen-containing heterocyclic group is selected from tetrahydropyrrole, piperidine, piperazine, or morpholine.
In some preferred embodiments, R₅ is a hydroxyl group;
In some preferred embodiments, R₅ is selected from C1-C6 alkoxy or C1-C6 alkyl ester groups;
In some preferred embodiments, R₅ is selected from fluorine, chlorine, bromine, or iodine.

In some preferred embodiments, R₅ is -OCONR₆R₇, where R₆ is hydrogen and R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, amino-substituted C1-C6 alkyl, 5-6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by one or more C1-C6 alkyl groups.
In some preferred embodiments, R₅ is -OCONR₆R₇, where R₆ is C1-C6 alkyl and R₇ is C1-C6 alkyl;
In some preferred embodiments, R₅ is -OCONR₆R₇, wherein R₆, R₇, and the N atom to which they are bonded form a 3-, 4-, 5-, 6-, 7-, 8-, 9-, or 10-membered ring structure;
In some preferred embodiments, R₅ is -OCONR₆R₇, wherein R₆, R₇, and the N atom to which they are bonded form a 5- or 6-membered ring structure;
In some preferred embodiments, R₅ is -OCONR₆R₇, wherein R₆, R₇, and the N atom to which they are attached form a 5- or 6-membered ring structure selected from tetrahydropyrrole, piperidine, piperazine, or morpholine.

In the above-described preferred embodiments, the ring structure formed by R₆, R₇, and the connecting nitrogen atom is substituted by one or more substituents; Preferably, said substituents are selected from C1-C6 alkyl groups and 5-6-membered nitrogen-containing heterocyclic groups; further preferably, said 5-6-membered nitrogen-containing heterocyclic groups are selected from tetrahydropyrolidyl, piperidyl, piperazinyl, or morpholinyl groups.

In the first and second aspects of the present invention, the guaiacol-type sesquiterpene lactone derivatives are represented by Formula IV: 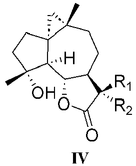
wherein, R₁ and R₂ together form a double bond;
or R₁ is hydrogen or deuterium, and R₂ is 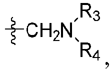 wherein R₃ and R₄ are each an alkyl group, or R₃, R₄, and the N atom to which they are attached form a 3- to 10-membered ring structure.

In some preferred embodiments, R₂ is 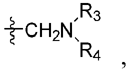 and R₃ and R₄ are each independently selected from C1-, C2-, C3-, C4-, C5-, C6-, C7-, C8-, C9-, or C10-alkyl;
In some preferred embodiments, R₂ is 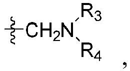 and R₃ and R₄ are each independently selected from C1-C6 alkyl;
In some preferred embodiments, R₂ is 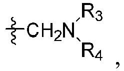 R₃ and R₄ are each independently selected from methyl, ethyl, n-propyl, isopropyl, n-butyl and isobutyl; more preferably, R₃ and R₄ are both methyl.

In some preferred embodiments, R₂ is 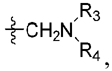 wherein R₃, R₄, and the N atom to which they are bonded form a 3-, 4-, 5-, 6-, 7-, 8-, 9-, or 10-membered ring structure;
In some preferred embodiments, R₂ is 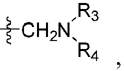 wherein R₃, R₄, and the N atom to which they are bonded form a 5- to 8-membered ring structure;
In some preferred embodiments, R₂ is 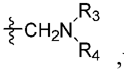 wherein R₃, R₄, and the N atom to which they are bonded form a 5- to 6-membered ring structure;
In some preferred embodiments, R₂ is 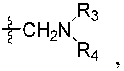 wherein R₃, R₄, and the N atom to which they are bonded form a 4- to 6-membered ring structure;
In the above preferred embodiments, R₃, R₄, and the N atom to which they are bonded form a monocyclic, spirocyclic, or fused ring structure;
In some preferred embodiments, R₂ is 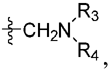 and the ring structure formed by R₃, R₄, and the N atom to which they are bonded is selected from azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

In the above preferred embodiments, the ring structure formed by R₃, R₄, and the N atom to which they are attached is substituted by one or more substituents; preferably, said substituents are selected from C1-C6 alkyl, 5-6-membered nitrogen-containing heterocyclic groups, C1-C6 alkoxycarbonyl (e.g., ethoxycarbonyl); Further preferably, the 5- to 6-membered nitrogen-containing heterocyclic group is selected from tetrahydropyrrole, piperidine, piperazine, or morpholine.

In the first and second aspects of the present invention, the pharmaceutically acceptable salt of the guaiacol sesquiterpene lactone derivative is selected from hydrochloride, sulfate, phosphate, maleate, fumarate, or citrate salts.

The third aspect of the present invention provides a use of the guaiacol sesquiterpene lactone derivative and its pharmaceutically acceptable salt in the preparation of a medicament for treating tumors. Preferably, the tumor is hepatocellular carcinoma, colorectal cancer, or lymphoma.

In the third aspect of the present invention, the guaiacol sesquiterpene lactone derivative is represented by Formula I: 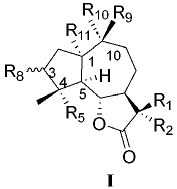
Wherein R₁ and R₂ together form a double bond; or R₁ is hydrogen or deuterium, and R₂ is 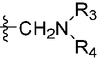 wherein R₃ and R₄ are each C1-C3 alkyl, or R₃, R₄, and N atom to which they are attached form a 5-6 membered ring structure, the 5-6 membered ring structure being selected from pyrrole, proline, piperidine, piperazine, or morpholine;
R₅ is hydroxyl, methoxy, methyl ester, halogen, or -OCONR₆R₇, wherein R₆ is hydrogen or C1-C6 alkyl; R₇ is C1-C6 alkyl, fluorine-substituted C1-C3 alkyl, cyclopropyl, hydroxyl-substituted C1-C6 alkyl, methoxy-substituted C1-C6 alkyl, dimethylaminoethyl, morpholine-substituted C1-C6 alkyl, piperazine-substituted C1-C6 alkyl, or benzene ring -substituted methyl; or R₆, R₇, and the N atom form a substituted 5- to 6-membered ring structure; or the 3rd carbon atom and the 4th carbon atom form a double bond; or the 4th carbon atom and the 5th carbon atom form a double bond;
R₈ is hydrogen or hydroxyl;
R₉ is methyl, R₁₀ and R₁₁ form a cyclopropane ring; or a double bond is formed between the 1st and 10th carbon atoms.

In a third aspect of the present invention, the guaiacol-type sesquiterpene lactone derivative is represented by Formula II: 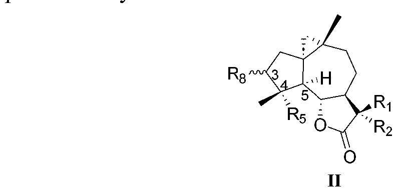
R₁ and R₂ together form a double bond; or R₁ is hydrogen or deuterium, and R₂ is 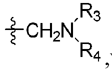 wherein R₃ and R₄ are each C1-C3 alkyl, or R₃, R₄, and N atom to which they are attached form a 5- to 6-membered ring structure selected from pyrrole, proline, piperidine, piperazine, and morpholine;
R₅ is hydroxyl, methoxy, methyl ester, or halogen; or the 3rd carbon atom is bonded to the 4th carbon atom to form a double bond; or the 4th carbon atom is bonded to the 5th carbon atom to form a double bond;
R₈ is hydrogen or hydroxyl.

In the third aspect of the present invention, the guaiacol-type sesquiterpene lactone derivative is represented by Formula III: 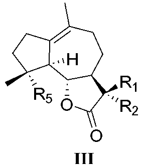
R₁ and R₂ together form a double bond; or R₁ is hydrogen or deuterium, and R₂ is 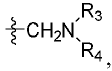 wherein R₃ and R₄ are each C1-C3 alkyl, or R₃, R₄, and N atom form a pyrrole ring;
R₅ is -OCONR₆R₇, wherein R₆ is hydrogen or C1-C6 alkyl; R₇ is C1-C6 alkyl, fluorine-substituted C1-C3 alkyl, cyclopropyl, hydroxy-substituted C1-C6 alkyl, methoxy-substituted C1-C6 alkyl, dimethylaminoethyl, morpholino-substituted C1-C6 alkyl, piperazine-substituted C1-C6 alkyl, or phenyl-substituted methyl; or R₆, R₇, and the N atom form a substituted 5- to 6-membered ring structure.

In a third aspect of the present invention, the pharmaceutically acceptable salt of the guaiacol sesquiterpene lactone derivative is selected from hydrochloride, sulfate, phosphate, maleate, fumarate, or citrate salts.

The fourth aspect of the present invention provides a use of guaiacol sesquiterpene lactone derivatives and their pharmaceutically acceptable salts in the preparation of medicaments for treating inflammatory diseases or soothing the skin. Preferably, the inflammatory diseases are selected from (1) acute lung injury; (2) hepatitis; (3) sepsis caused by bacterial and/or viral infections; (4) acute and chronic kidney diseases; (5) renal fibrosis; (6) lupus nephritis; (7) diabetic nephropathy; and (8) atopic dermatitis.

In the fourth aspect of the present invention, the guaiacol-type sesquiterpene lactone derivative is represented by Formula IV: 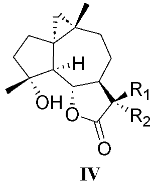 wherein R₁ and R₂ together form a double bond; or R₁ is hydrogen or deuterium, and R₂ is 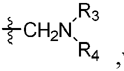 wherein R₃ and R₄ are each C1-C4 alkyl or isopropyl; or R₃, R₄, and N form a 4-6-membered monocyclic structure, with the 4-6-membered monocyclic structure selected from azetidine, pyrrole, morpholine, piperidine, piperazine, with ring substituents selected from ethyl, ethyl ester, morpholine, or piperidine; or R₃, R₄, and the N atom form an 8-membered cyclic structure, wherein the 8-membered cyclic structure is selected from azetinoctane; or R₃, R₄, and the N atom form an 8-membered spiro ring structure, wherein the 8-membered spiro ring structure is selected from 6-azaspiro[2,5]octanes; or R₃, R₄, and the N atom form a 10-membered spiro ring structure, wherein the 10-membered spiro ring structure is selected from 8-azaspiro[4,5]decane; or R₃, R₄, and the N atom form an 8-membered fused ring structure, wherein the 8-membered spiro ring structure is selected from octahydrocyclopenta[c]pyrrole.

In the fourth aspect of the present invention, the pharmaceutically acceptable salt of the guaiacol sesquiterpene lactone derivative is selected from hydrochloride, sulfate, phosphate, maleate, fumarate, or citrate salts.

In all aspects of the present invention, the guaiacol sesquiterpene lactone derivative may optionally be selected from the following compounds:

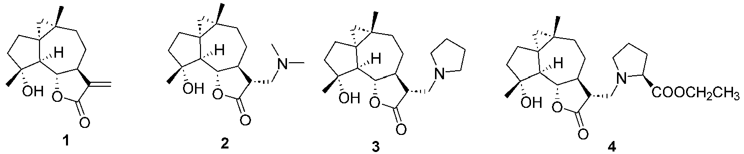

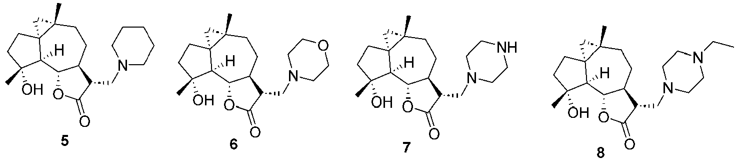

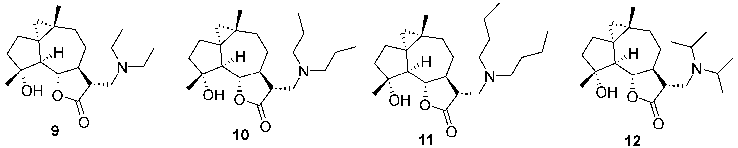

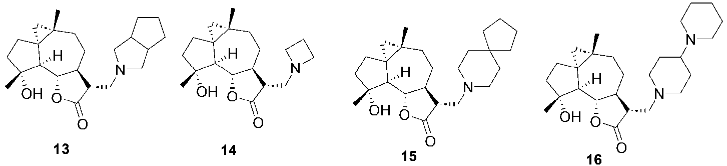

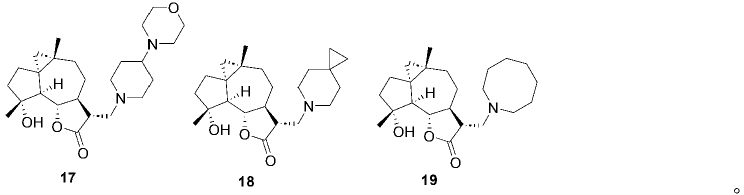

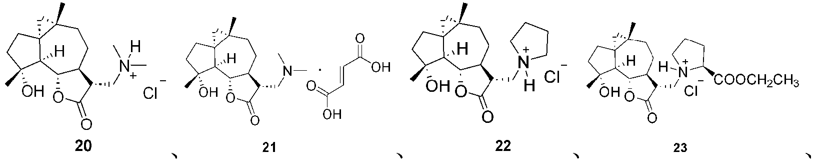

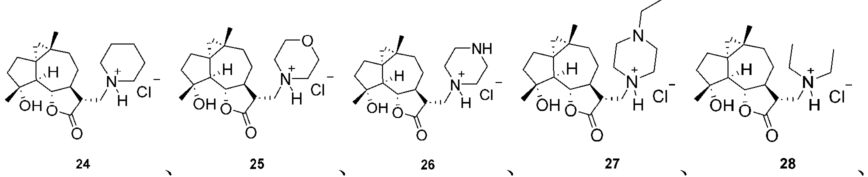

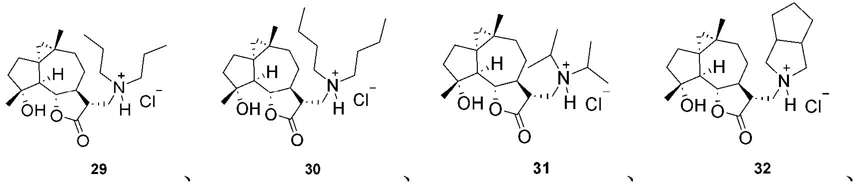

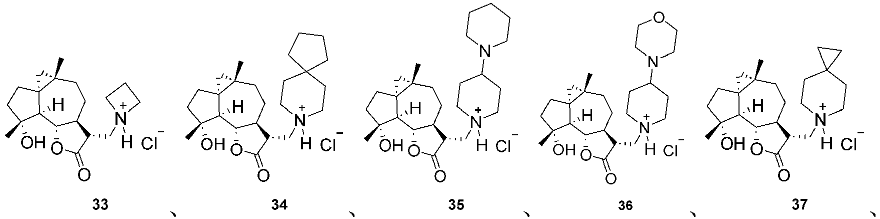

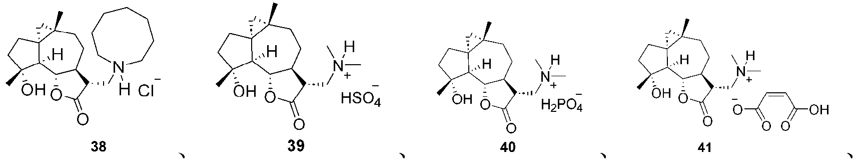

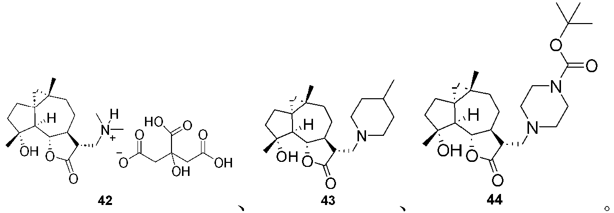

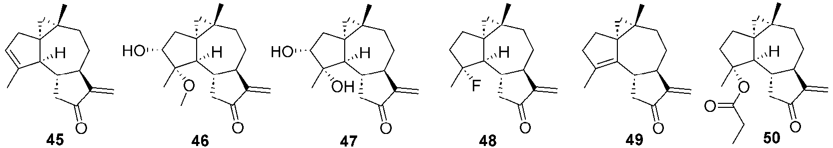

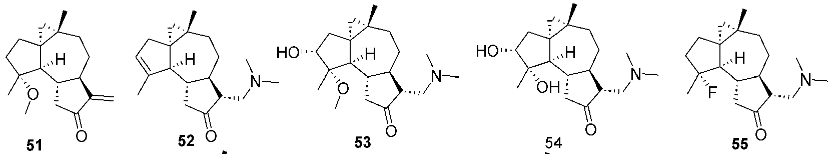

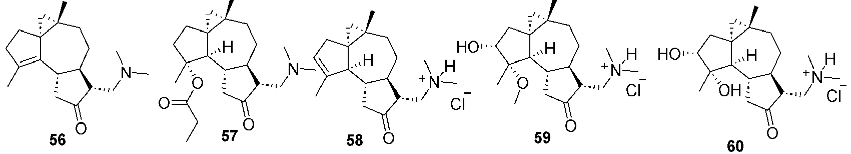

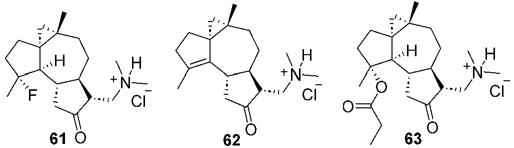

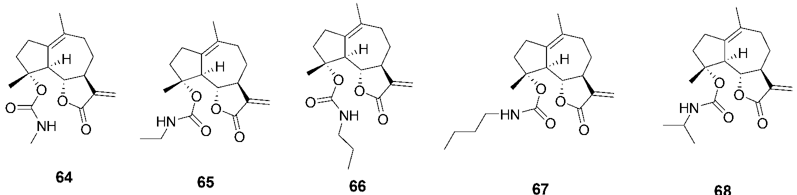

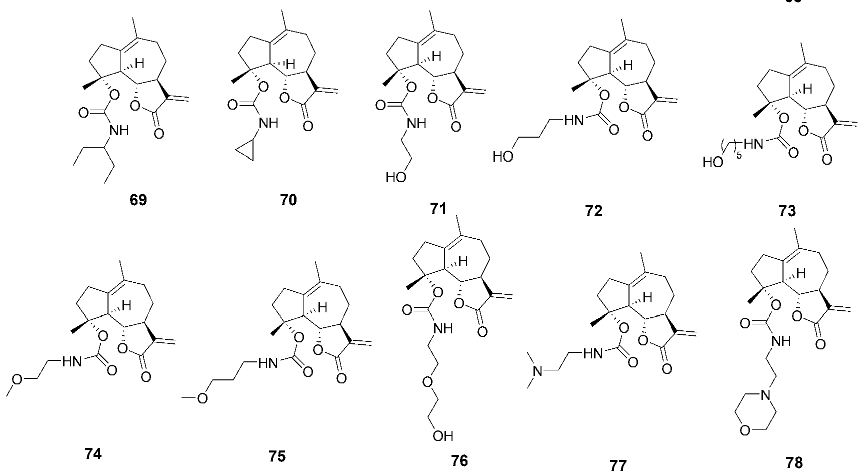

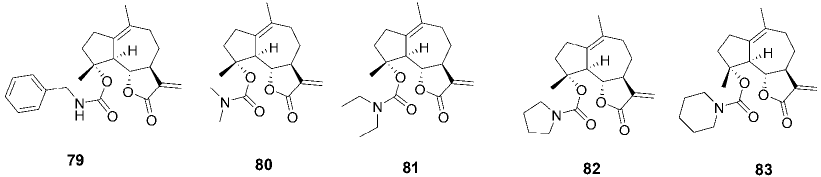

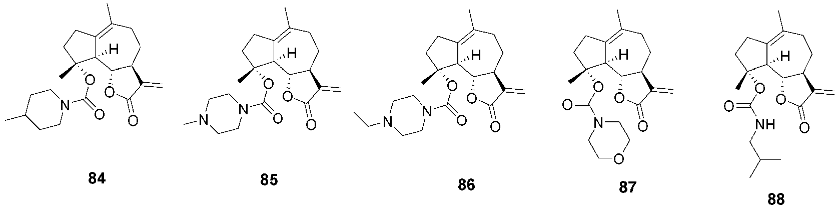

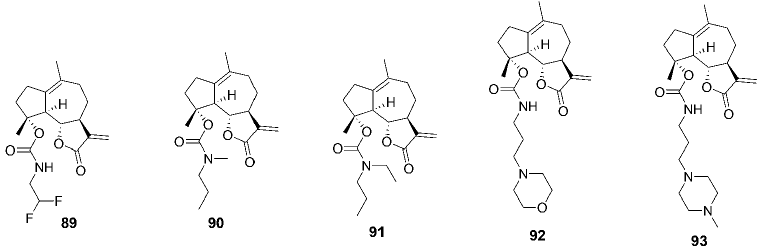

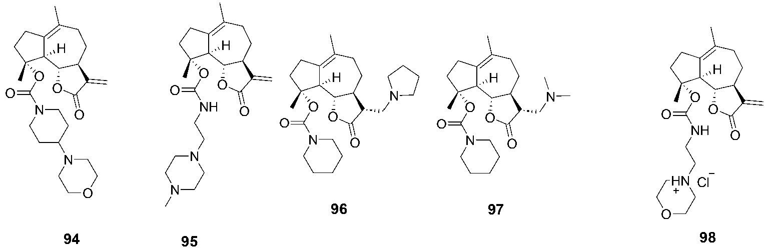

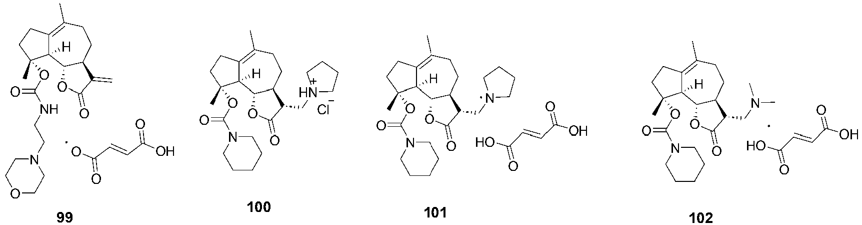

Preferably, the compounds of the present invention are selected from the following compounds:

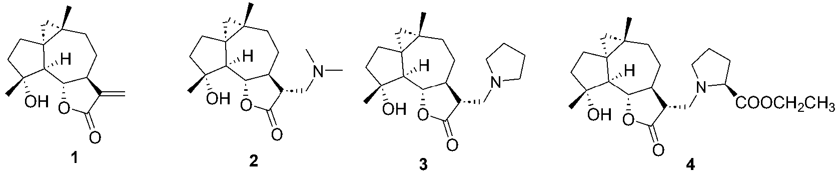

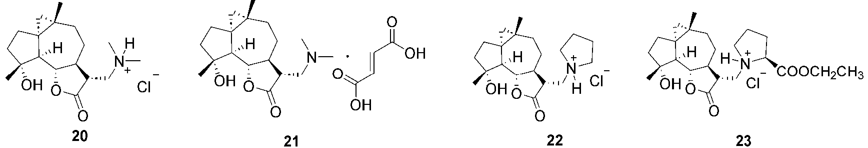

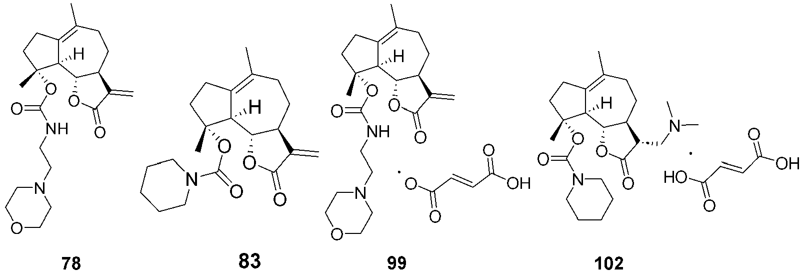

The fifth aspect of the present invention provides the use of guaianone sesquiterpene lactone derivatives or their pharmaceutically acceptable salts, prodrugs, solvates, or stereoisomers in the preparation of antitumor drugs; preferably, the tumor is hepatocellular carcinoma, colorectal cancer, or lymphoma.

It is further provided for the use of guaiacol sesquiterpene lactone derivatives or their pharmaceutically acceptable salts, prodrugs, solvates, or stereoisomers in the preparation of drugs for treating inflammatory diseases or soothing skin conditions; preferably, the inflammatory diseases are selected from: (1) acute lung injury; (2) hepatitis; (3) sepsis caused by bacterial and/or viral infections; (4) acute and chronic kidney diseases; (5) renal fibrosis; (6) lupus nephritis; (7) diabetic nephropathy; (8) atopic dermatitis.

Preferably, the skin soothing is achieved by increasing skin hyaluronic acid levels and/or inhibiting mast cell degranulation.

The guaianone sesquiterpene lactone derivative is compound 1.

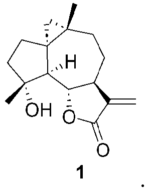

Preferably, the guaiacol sesquiterpene lactone derivative prodrug may be selected from compounds 2 to 44:

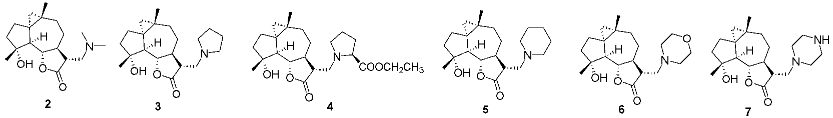

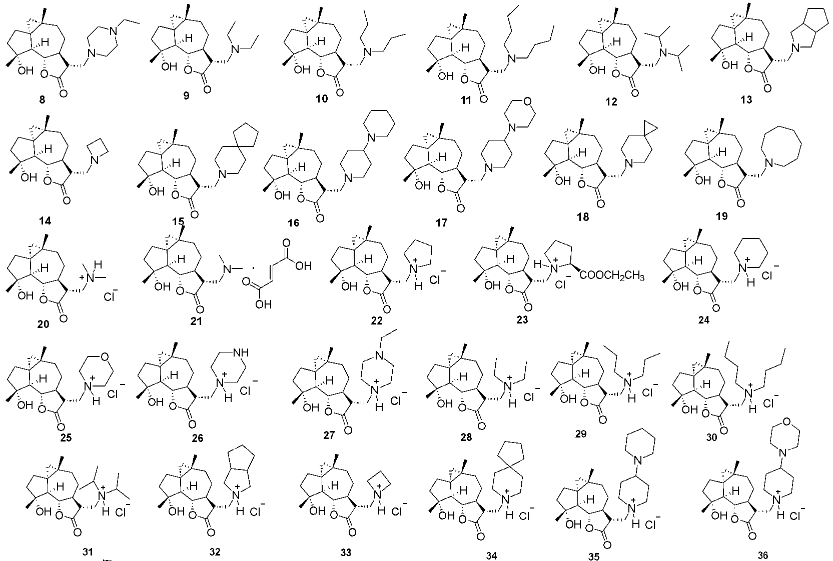

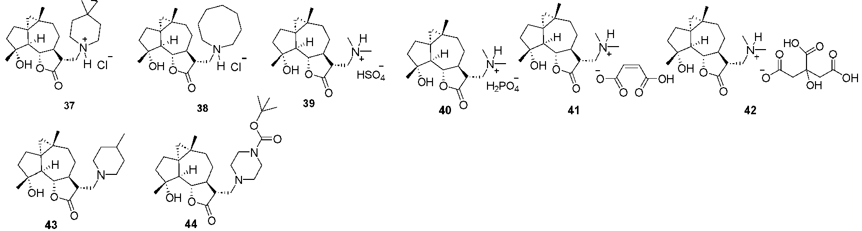

Preferably, the guaiacol sesquiterpene lactone derivative prodrug may be selected from the following compounds:

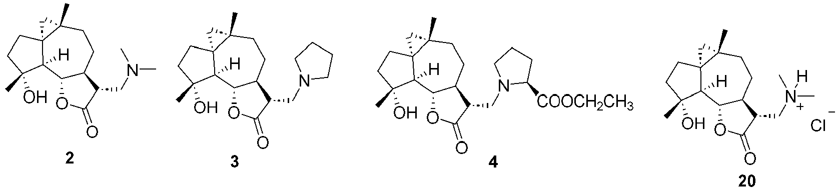

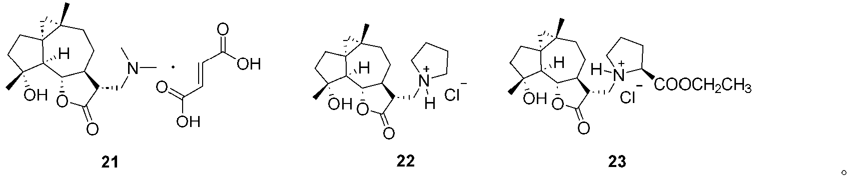

The sixth aspect of the present invention provides a Michelia lactone prodrug and its pharmaceutically acceptable salts, wherein the Michelia lactone prodrug has the structure shown in Formula III. 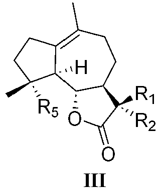
R₁ and R₂ together form a double bond;
or R₁ is hydrogen or deuterium, and R₂ is 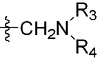 wherein R₃ and R₄ are each an alkyl group, or R₃, R₄, and N form a 3- to 10-membered ring structure;
R₅ is selected from hydroxyl, C1-C6 alkoxy, C1-C6 alkyl ester, halogen, and - OCONR₆R₇, where R₆ is hydrogen or C1-C6 alkyl; R₇ is C1-C6 alkyl, fluorinated C1-C6 alkyl, C1-C6 cycloalkyl, hydroxy-substituted C1-C6 alkyl, C1-C6 alkoxy-substituted C1-C6 alkyl, OH-(CH₂)n-O-substituted C1-C6 alkyl, amino-substituted C1-C6 alkyl, 5-6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by one or more C1-C6 alkyl groups, or R₆, R₇, and the N atom to which they are attached may form a 3- to 10-membered cyclic structure;
n is selected from 1, 2, 3, 4, 5, or 6.
In some preferred embodiments, R₁ and R₂ together form a double bond;
In some preferred embodiments, R₁ is hydrogen;
In some preferred embodiments, R₂ is 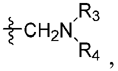 and R₃ and R₄ are each independently selected from C1-, C2-, C3-, C4-, C5-, C6-, C7-, C8-, C9-, or C10-alkyl;
In some preferred embodiments, R₂ is 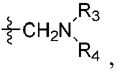 and R₃ and R₄ are each independently selected from C1-C6 alkyl;
In some preferred embodiments, R₂ is 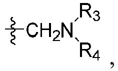 and R₃ and R₄ are each independently selected from methyl, ethyl, n-propyl, isopropyl, n-butyl, isobutyl; more preferably, R₃ and R₄ are both methyl;
In some preferred embodiments, R₂ is 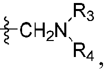 wherein R₃, R₄, and the N atom to which they are attached form a 3-, 4-, 5-, 6-, 7-, 8-, 9-, or 10-membered ring structure;
In some preferred embodiments, R₂ is 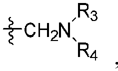 wherein R₃, R₄, and the N atom to which they are attached form a 5- to 8-membered ring structure;
In some preferred embodiments, R₂ is 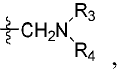 wherein R₃, R₄, and the N atom to which they are bonded form a 5- to 6-membered ring structure;
In some preferred embodiments, R₂ is 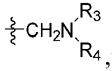 wherein R₃, R₄, and the N atom to which they are bonded form a 4- to 6-membered ring structure;
In the above-described preferred embodiments, R₃, R₄, and the N atom to which they are bonded form a monocyclic, spirocyclic, or fused ring structure;
In some preferred embodiments, R₂ is 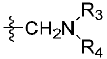 wherein the ring structure formed by R₃, R₄, and the N atom to which they are bonded is selected from azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

In the above preferred embodiments, the ring structure formed by R₃, R₄, and the N atom to which they are attached is substituted by one or more substituents; preferably, said substituents are selected from C1-C6 alkyl, 5-6-membered nitrogen-containing heterocyclic groups, and C1-C6 alkoxycarbonyl; further preferably, the 5- to 6-membered nitrogen-containing heterocyclic group is selected from tetrahydropyrolidyl, piperidyl, piperazinyl, or morpholinyl.

In some preferred embodiments, R₅ is a hydroxyl group;
In some preferred embodiments, R₅ is selected from C1-C6 alkoxy or C1-C6 alkyl ester groups;
In some preferred embodiments, R₅ is selected from fluorine, chlorine, bromine, or iodine.

In some preferred embodiments, R₅ is -OCONR6R7, where R₆ is hydrogen, and R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, amino-substituted C1-C6 alkyl, 5-6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by one or more C1-C6 alkyl groups.
In some preferred embodiments, R₅ is -OCONR₆R₇, where R₆ is C1-C6 alkyl and R₇ is C1-C6 alkyl;
In some preferred embodiments, R₅ is -OCONR₆R₇, wherein R₆, R₇, and the N atom to which they are bonded form a 3-, 4-, 5-, 6-, 7-, 8-, 9-, or 10-membered ring structure;
In some preferred embodiments, R₅ is -OCONR₆R₇, wherein R₆, R₇, and the N atom to which they are bonded form a 5- or 6-membered ring structure;
In some preferred embodiments, R₅ is -OCONR₆R₇, wherein R₆, R₇, and the N atom to which they are bonded form a 5- or 6-membered ring structure selected from tetrahydropyrole, piperidine, piperazine, or morpholine.

In the above preferred embodiments, the cyclic structure formed by R₆, R₇, and the connected N atom is substituted by one or more substituents; Preferably, the substituent is selected from C1-C6 alkyl and 5-6-membered nitrogen-containing heterocyclic groups; further preferably, the 5-6-membered nitrogen-containing heterocyclic group is selected from tetrahydropyrrole, piperidine, piperazine, or morpholine.

The seventh aspect of the present invention further discloses a pharmaceutical composition comprising a guaianone sesquiterpene lactone derivative of the present invention or a pharmaceutically acceptable salt thereof, and a pharmaceutically acceptable carrier.

The present invention further discloses a pharmaceutical composition comprising a guaiacol-type sesquiterpene lactone derivative of the present invention or a pharmaceutically acceptable salt thereof, a pharmaceutically acceptable carrier, and an immune checkpoint inhibitor.

Preferably, the immune checkpoint inhibitor is an anti-PD-L1/PD-1 monoclonal antibody or an anti-CTLA-4 monoclonal antibody.

The present invention further discloses the use of the aforementioned pharmaceutical composition in the preparation of antitumor drugs, treatments for inflammatory diseases, or skin soothing agents.

The tumors are selected from: hepatocellular carcinoma, colorectal cancer, or lymphoma;
The inflammatory diseases are selected from (1) acute lung injury; (2) hepatitis; (3) sepsis caused by bacterial and/or viral infections; (4) acute and chronic kidney diseases; (5) renal fibrosis; (6) lupus nephritis; (7) diabetic nephropathy; (8) atopic dermatitis.

Preferably, the soothing effect on the skin is achieved by increasing skin hyaluronic acid levels and/or inhibiting mast cell degranulation.

In the seventh aspect of the present invention, the pharmaceutically acceptable salt of the guaiacol sesquiterpene lactone derivative is selected from hydrochloride, sulfate, phosphate, maleate, fumarate, or citrate salts.

Advantages: Compared to the prior art, the present invention offers the following significant advantages: (1) Strong anti-inflammatory activity, significantly improving tissue damage in kidneys, liver, lungs, etc., of septic mice; (2) Significantly improving LPS-induced acute lung injury in mice; (3) Alleviates CCl4-induced hepatic injury responses; (4) obtained salt-form compounds with markedly enhanced water solubility, which can be converted back to the parent compound in HEPES buffer solution and mouse plasma to exert anti-inflammatory activity.

Compared to prior art, the present invention offers the following significant advantages: (1) Strong anti-inflammatory activity, significantly reducing renal inflammatory responses induced by folic acid or unilateral ureteral ligation while decreasing folic acid-induced renal fibrosis; (2) Reducing renal inflammatory responses in lupus nephritis mice; (3) Reducing renal inflammatory responses and pathological damage in diabetic nephropathy mice; (4) Alleviates ear and back lesions in atopic dermatitis (AD) mice, along with associated inflammatory factor levels; (5) Yields salt-form compounds with significantly enhanced water solubility, which can convert to the parent compound in both HEPES buffer solution and mouse plasma to exert anti-inflammatory activity. Concurrently, the compound preparation process is straightforward with high yield.

Compared to prior art, the present invention offers the following significant advantages: (1) Demonstrates superior therapeutic efficacy against thermotolerant tumors such as hepatocellular carcinoma, colorectal cancer, or lymphoma; Oral administration of compound 20 (40 µmol/kg/d) achieved a maximum tumor inhibition rate of 96.78% in the Hepa1-6 mouse xenograft model; At a concentration of 10 µM, compound 1 exhibited a maximum inhibition rate of 83.3% against the chemotaxis of myeloid-derived suppressor cells (MDSCs); (2) Synergistic effects were observed when combined with immune checkpoint inhibitors, significantly enhancing antitumor efficacy. Intraperitoneal injection of compound 20 (1 mg/kg/d) combined with anti-PD-L1 monoclonal antibody (5 mg/kg/d) achieved a maximum tumor inhibition rate of 96.99% in the Hepa1-6 mouse xenograft model; (3) Derivatives of the present invention demonstrated extremely low toxicity when applied in antitumor therapy.

The present invention possesses the following significant advantages: (1) It exerts certain skin-soothing effects by downregulating hyaluronidase in human keratinocytes and upregulating hyaluronic acid levels; (2) It exerts certain skin-soothing effects by inhibiting mast cell degranulation.

### Specific Embodiments

The present invention are further illustrated below with reference to the examples. All experimental materials used in the examples can be obtained through conventional channels.

### Example 1

Preparation of Compounds:
**Preparation of parthenolide:** Take 5 kg of dried *Magnolia delavayi Franch* root bark, grind into coarse powder, and soak in 10 times the volume of 95% ethanol for 12 hours. Reflux extract twice, each for 2 hours, filter, combine filtrates, concentrate under reduced pressure, and dry to obtain crude extract of *Magnolia delavayi Franch* root bark. Refine by silica gel column chromatography using a petroleum ether-ethyl acetate gradient elution. Collect fractions rich in parthenolide and woody lactone, combine, concentrate, and recrystallize to obtain desired compound. Yield: 4.0%; Purity: 96.3%. ¹H NMR (500 MHz, CDCl₃): δ 6.31 (d, *J* = 2.9 Hz, 1H), 5.62 (d, *J* = 3.4 Hz, 1H), 5.20 (d, *J* = 11.8 Hz, 2H), 3.85 (t, *J* = 8.6 Hz, 1H), 2.78 (d, *J* = 8.9 Hz, 1H), 2.45-2.32(m, 2H), 2.22-2.10 (m, 4H), 1.70 (s, 3H), 1.69-1.66 (m, 1H), 1.29 (s, 3H), 1.27-1.18 (m, 1H)_{∘}. ESI-MS (m/z): [M+H]⁺ = 249.1 (calcd: 249.1)_{∘}

### Preparation of Compound 1

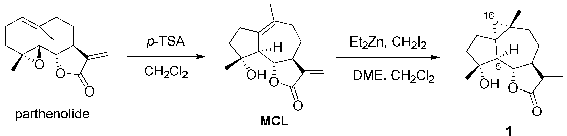

Add dichloromethane (50 mL), p-toluenesulfonic acid (125 mg, 0.73 mmol), and parthenolide (5 g, 20.16 mmol) sequentially to a 150 mL round-bottom flask. Stir at room temperature until TLC analysis indicates reaction completion. The reaction mixture was sequentially washed with water (10 mL × 3) and saturated saline solution (10 mL × 3). After drying over anhydrous sodium sulfate, the solution was concentrated under reduced pressure. Separation by silica gel column chromatography yielded the intermediate MCL in 90% yield. ¹H NMR (500 MHz, CDCl₃): δ 6.21 (d, *J* = 3.5 Hz, 1H), 5.51 *(d, J* = 3.0 Hz, 1H), 3.81 (t*, J* = 10.5 Hz, 1H), 2.73 *(d, J* = 10.5 Hz, 1H), 2.68-2.64 (m, 2H), 2.42-2.37 (m, 1H), 2.26-2.16 (m, 3H), 2.11-2.08 (m, 1H), 1.83-1.75 (m, 2H), 1.69 (s, 3H), 1.31 (s, 3H), 1.27-1.25 (m, 1H). ESI-MS (m/z): [M+Na]⁺ = 271.1 (calcd: 271.1)_{∘}

Under ice bath and nitrogen atmosphere conditions, add ethylene glycol dimethyl ether (1.67 mL, 21.26 mmol) to anhydrous dichloromethane (67 mL). stirred thoroughly, then 13.3 mL of diethylzinc solution (1 M n-hexane solution) was added. Methyl iodide (2.67 mL, 3.11 mmol) was slowly added dropwise, stirred for 10 min, yielding the cyclopropanation reagent. In a separate round-bottom flask, add Micheliolide MCL (300 mg, 1.21 mmol) and anhydrous dichloromethane (5 mL). Stir to dissolve, then protect under nitrogen and place on an ice bath. Add the cyclopropanation reagent dropwise to the substrate solution. After completion of the addition, continue the reaction for 1 h, then transfer to room temperature and react overnight. The reaction mixture was quenched with saturated ammonium chloride solution, filtered, and sequentially washed with water (10 mL × 3) and saturated saline solution (10 mL × 3). After drying over anhydrous sodium sulfate, the solution was concentrated under reduced pressure. Separation by silica gel column chromatography yielded compound 1 in 75% yield. ¹H NMR (500 MHz, CDCl₃): δ 6.14 (d, *J* = 3.5 Hz, 1H), 5.45 (d, *J* = 3.0 Hz, 1H), 3.82 (t, *J* = 10.5 Hz, 1H), 2.52-2.48 (m, 1H), 2.26-2.21 (m, 1H), 2.09-1.99 (m, 2H), 1.93-1.89 (m, 1H), 1.88-1.86 (m, 1H, H₅), 1.85-1.83 (m, 1H), 1.55 (s, 3H), 1.52-1.44 (m, 2H), 1.26-1.14 (m, 2H), 1.11 (s, 3H), 0.81 (d, *J* = 4.0 Hz, 1H, H₁₆ₐ), 0.54 (d, *J* = 4.0 Hz, 1H, H_{16b})_{∘} The ROESY spectrum indicates a signal correlation between H₅ and H₁₆ₐ, and confirms that the cyclopropane is in the α configuration. ESI-MS (m/z): [M+Na]⁺ = 285.2 (calcd: 285.2)_{∘}

### Preparation of Compound 2

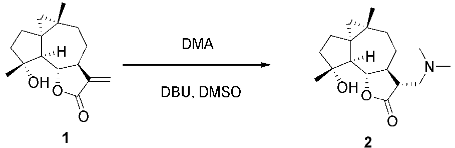

Compound 1 (262 mg, 1.00 mmol), dimethylamine (68 mg, 1.50 mmol), DBU (380 mg, 2.5 mmol), and dimethyl sulfoxide (3 mL) were added to a round-bottom flask and stirred for 8 h. The reaction mixture was diluted with water (50 mL), then extracted with ethyl acetate (20 mL × 3). The combined organic phase was washed successively with water (10 mL × 3) and saturated saline solution (10 mL × 3). After drying over anhydrous sodium sulfate, the solution was concentrated under reduced pressure. The residue was purified by silica gel column chromatography (petroleum ether: ethyl acetate: triethylamine = 1:1:0.02) to obtain compound 2 in 85% yield. ¹H NMR (500 MHz, CDCl₃): δ3.92 (t, *J* = 10.5 Hz, 1H), 3.55-3.47 (m, 1H), 3.33-3.29 (m, 1H), 2.38-2.34 (m, 1H), 2.28 (s, 6H), 2.21-2.16 (m, 2H), 1.98 (d, *J* = 15.0 Hz, 2H), 1.88 (t, *J* = 5.0 Hz, 2H), 1.72 (d, *J* = 10.0 Hz, 1H), 1.60-1.52 (m, 2H), 1.50 (s, 3H), 1.20 (t, *J* = 5.0 Hz, 1H), 1.12 (s, 3H), 1.08-1.04 (m, 1H), 0.76 (d, *J* = 5.0 Hz, 1H), 0.53 (d, *J* = 5.0 Hz, 1H)_{∘} ESI-MS (m/z): [M+Na]⁺ = 330.2 (calcd: 330.2)_{∘}

### Preparation of Compound 3

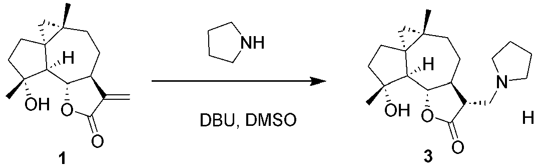

The preparation method is the same as that for Compound 2. The yield of Compound 3 is 86%. ¹H NMR (500 MHz, CDCl₃): δ 3.76 (t, *J* = 10.2 Hz, 1H), 2.86-2.74 (m, 2H), 2.53-2.43 (m, 4H), 2.35-2.28 (m, 1H), 2.14 (dd, *J* = 14.3, 6.6 Hz, 1H), 1.93-1.83 (m, 3H), 1.76 (s, 1H), 1.75-1.68 (m, 5H), 1.51 (s, 3H), 1.48-1.37 (m, 2H), 1.29-1.23 (m, 1H), 1.09 (s, 3H), 1.03 (d, *J* = 13.1 Hz, 1H), 0.75 (d, *J =* 3.4 Hz, 1H), 0.49 (d, *J* = 3.4 Hz, 1H). ESI-MS (m/z): [M+H]⁺ = 334.2 (calcd: 334.2).

### Preparation of Compound 4

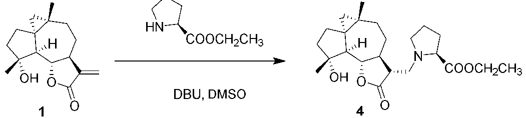

The preparation method is the same as that for compound 2. The yield of compound 4 is 83%. ¹H NMR (500 MHz, CDCl₃): δ 4.14 (m, *J* = 7.1 Hz, 2H), 3.78 (t*, J* = 10.3 Hz, 1H), 3.05 (dd, *J* = 13.4, 5.0 Hz, 1H), 3.00-2.87 (m, 2H), 2.46 (q, *J* = 7.1, 6.7 Hz, 1H), 2.36 (s, 1H), 2.29 (d, *J* = 11.4 Hz, 1H), 2.16 (dd, *J* = 14.6, 6.6 Hz, 1H), 2.11-2.01 (m, 3H), 1.93-1.84 (m, 4H), 1.78 (d, *J* = 10.4 Hz, 2H), 1.52 (s, 3H), 1.49-1.34 (m, 2H), 1.26 (t, *J* = 7.4 Hz, 4H), 1.10 (s, 4H), 0.78 (d, *J* = 3.6 Hz, 1H), 0.50 (d, *J* = 3.6 Hz, 1H). ESI-MS (m/z): [M+H]⁺ = 406.3 (calcd: 406.3).

### Preparation of Compound 5

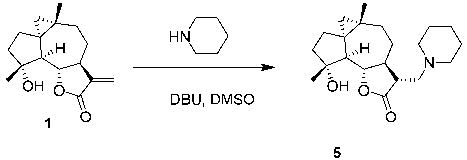

The preparation method is the same as that for compound 2. The yield of compound 5 is 88%. ¹H NMR (500 MHz, CDCl₃): δ 3.79 (t, *J* = 10.5 Hz, 1H), 2.79-2.75 (m, 1H), 2.56-2.53 (m, 1H), 2.46-2.40 (m, 3H), 2.39-2.32(m,3H), 2.19-2.08(m, 4H), 1.94-1.85 (m, 3H), 1.77 (d*, J* = 10.5 Hz, 1H), 1.54 (s, 6H), 1.45-1.39 (m, 4H), 1.12 (s, 3H), 1.09-1.04 (m, 1H), 0.79 (d, *J*=4.0 Hz, 1H), 0.52 (d, *J* = 4.0 Hz, 1H). ESI-MS (m/z): [M+H]⁺ = 348.3 (calcd: 348.3)_{∘}

### Preparation of Compound 6

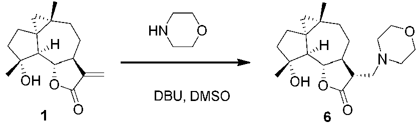

The preparation method is the same as that for compound 2. The yield of compound 6 is 86%. ¹H NMR (500 MHz, CDCl₃): δ 3.81(t, *J* = 10.5 Hz, 1H), 3.74-3.67 (m,3H), 2.82-2.79 (m, 1H), 2.65-2.61(m, 1H), 2.53-2.44(m, 5H), 2.40-2.35(m, 1H), 2.21-2.17(m, 1H), 2.13-2.07(m, 2H), 1.95-1.86(m, 3H), 1.78(d, *J*=10.5 Hz, 1H), 1.55(s, 3H), 1.52-1.39 (m, 2H), 1.31-1.27(m, 1H), 1.12(s, 3H), 1.09-1.04 (m,1H), 0.80 (d, *J* =4.0Hz, 1H), 0.54(d, *J*=4.0 Hz,1H). ESI-MS (m/z): [M+H]⁺ = 350.2 (calcd: 350.2)_{∘}

### Preparation of Compound 7

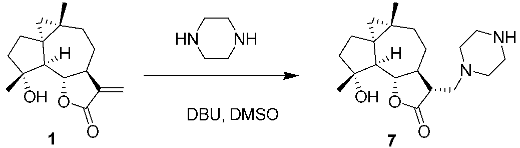

The preparation method is the same as that for compound 2. The yield of compound 7 is 82%.¹H NMR (500 MHz, CDCl₃): δ 3.79 (t, *J* = 10.5 Hz, 1H), 2.82-2.79 (m, 1H), 2.64-2.60 (m, 1H), 2.55-2.42 (m, 5H), 2.38-2.34 (m, 2H), 2.29 (s, 3H), 2.20-2.16 (m, 1H), 2.15-2.05 (m,2H), 1.93-1.83 (m,3H), 1.76 (d, *J* = 10.5 Hz,1H), 1.54(s, 3H), 1.49-1.38(m, 2H), 1.29-1.25 (m, 1H), 1.12 (s, 3H), 1.09-1.03 (m, 1H),0.78(d, *J*= 4.0 Hz, 1H), 0.52(d, *J*= 4.0 Hz,1H). ESI-MS (m/z): [M+H]⁺ = 349.2 (calcd: 349.2).

### Preparation of Compound 8

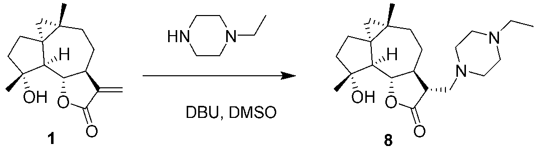

The preparation method is the same as that for compound 2. The yield of compound 8 is 76%.¹H NMR (500 MHz, CDCl₃): δ 3.75 (t, *J* = 10.2 Hz, 1H), 2.77 (dd, *J* = 13.2, 4.2 Hz, 1H), 2.58 (dd, *J* = 13.2, 6.8 Hz, 2H), 2.47 (d, *J* = 30.9 Hz, 4H), 2.41-2.35 (m, 4H), 2.32 (dd, *J* = 12.1, 6.5 Hz, 1H), 2.26 - 2.17 (m, 4H), 1.92-1.77 (m, 3H), 1.72 (d, *J* = 10.6 Hz, 1H), 1.50 (s, 3H), 1.47- 1.20 (m, 3H), 1.08 (s, 3H), 1.06-0.95 (m, 3H), 0.74 (d, *J* = 3.7 Hz, 1H), 0.48 (d, *J* = 3.5 Hz, 1H). ESI-MS (m/z): [M+H]⁺ = 377.3 (calcd: 377.3)_{∘}

### Preparation of Compound 9

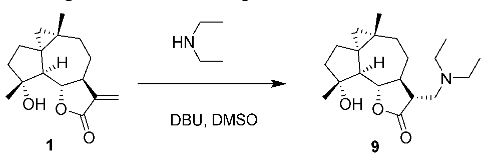

The preparation method is the same as that for compound 2. The yield of compound 9 is 78%. ¹H NMR (500 MHz, CDCl₃): δ 3.77 (t, *J=* 10.3 Hz, 1H), 2.83 (dd, *J* = 13.6, 4.6 Hz, 1H), 2.63 (dd, *J* = 13.7, 6.6 Hz, 1H), 2.58-2.50 (m, 2H), 2.45 m, *J* = 6.8 Hz, 2H), 2.38 (s, 1H), 2.29 (dt, *J* = 11.7, 5.6 Hz, 1H), 2.19-2.11 (m, 2H), 2.11-2.04 (m, 1H), 1.91-1.78 (m, 3H), 1.75 *(d, J* = 10.5 Hz, 1H), 1.52 (s, 3H), 1.50 - 1.36 (m, 2H), 1.09 (s, 3H), 1.05 (d, *J* = 13.1 Hz, 1H), 0.98 (t, *J* = 7.1 Hz, 6H), 0.76 (d, *J* = 4.0 Hz, 1H), 0.49 (d, *J* = 4.0 Hz, 1H). ESI-MS (m/z): [M+H]⁺ = 336.2 (calcd: 336.2).

### Preparation of Compound 10

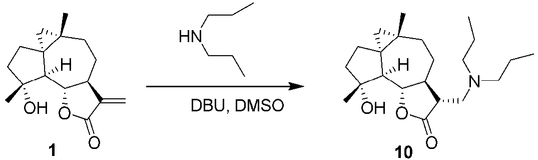

The preparation method is the same as that for compound 2. The yield of compound 10 is 78%. ¹H NMR (500 MHz, CDCl₃): δ 3.78 (t, *J* = 10.2 Hz, 1H), 2.87-2.80 (m, 1H), 2.66 (dd, *J* = 13.5, 6.6 Hz, 1H), 2.38 (d, *J* = 12.2 Hz, 3H), 2.34-2.25 (m, 3H), 2.15 (d, *J* = 11.7 Hz, 2H), 2.11-2.04 (m, 1H), 1.93 -1.82 (m, 3H), 1.75 (d, *J* = 10.6 Hz, 1H), 1.52 (s, 3H), 1.45-1.38 (m, 5H), 1.10 (s, 3H), 1.04 (t, *J* = 12.2 Hz, 1H), 0.85 (t, *J* = 7.3 Hz, 6H), 0.76 (d, *J* = 3.9 Hz, 1H), 0.50 (d, *J* = 3.9 Hz, 1H). ESI-MS (m/z): [M+H]⁺ = 364.3 (calcd: 364.3).

### Preparation of Compound 11

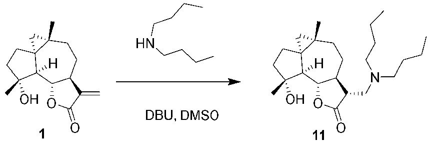

The preparation method is the same as that for compound 2. The yield of compound 11 is 78%. ¹H NMR (500 MHz, CDCl₃): δ 3.78 (t, *J* = 10.2 Hz, 1H), 2.82 (dd, *J* = 13.6, 4.2 Hz, 1H), 2.64 (dd, *J* = 13.5, 6.6 Hz, 1H), 2.42 (m, *J* = 14.3, 7.7 Hz, 2H), 2.38-2.24 (m, 4H), 2.19-2.04 (m, 3H), 1.93-1.82 (m, 3H), 1.75 (d, *J* = 10.6 Hz, 1H), 1.52 (s, 3H), 1.43-1.32 (m, 5H), 1.28 (m, *J* = 7.1 Hz, 5H), 1.10 (s, 3H), 0.90 (t, *J* = 7.3 Hz, 6H), 0.77 (d, *J* = 3.8 Hz, 1H), 0.50 *(d, J* = 3.8 Hz, 1H). ESI-MS (m/z): [M+H]⁺ = 392.3 (calcd: 392.3).

### Preparation of Compound 12

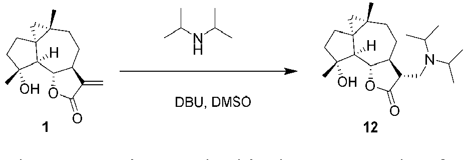

The preparation method is the same as that for compound 2. The yield of compound 12 is 75%. ¹H NMR (500 MHz, CDCl₃): δ 3.78 (t, *J* = 10.3 Hz, 1H), 2.83 (d, *J* = 8.7 Hz, 1H), 2.65 (dd, *J* = 13.2, 6.2 Hz, 1H), 2.38 (m, *J* = 12.7, 9.1 Hz, 3H), 2.14 (d, *J* = 12.2 Hz, 2H), 2.11-2.01 (m, 1H), 1.88 (m, *J* = 10.0, 7.8 Hz, 3H), 1.74 (d, *J* = 10.5 Hz, 1H), 1.51 (s, 3H), 1.49-1.35 (m, 6H), 1.09 (s, 3H), 1.03 (t, *J* = 12.2 Hz, 1H), 0.85 (q, *J* = 9.0, 7.3 Hz, 6H), 0.75 (d, *J* = 3.6 Hz, 1H), 0.49 (d, *J* = 3.5 Hz, 1H), 0.06 (s, 2H).ESI-MS (m/z): [M+H]⁺ =364.3 (calcd: 364.3).

### Preparation of Compound 13

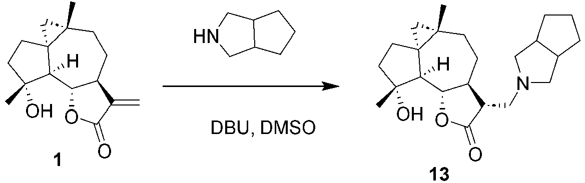

The preparation method is the same as that for compound 2. The yield of compound 13 is 80%. ¹H NMR (500 MHz, CDCl₃): δ 3.76 (t, *J* = 10.3 Hz, 1H), 2.76-2.66 (m, 2H), 2.62-2.47 (m, 4H), 2.38 (s, 1H), 2.31- 2.25 (m, 1H), 2.20-2.03 (m, 5H), 1.94-1.82 (m, 3H), 1.74 (d, *J* = 10.6 Hz, 1H), 1.67-1.55 (m, 3H), 1.51 (s, 3H), 1.49-1.37 (m, 3H), 1.33 (dd, *J* = 11.0, 6.7 Hz, 2H), 1.09 (s, 3H), 0.76 (d, *J* = 3.6 Hz, 1H), 0.49 (d, *J* = 3.6 Hz, 1H).ESI-MS (m/z): [M+H]⁺ = 374.3 (calcd: 374.3).

### Preparation of Compound 14

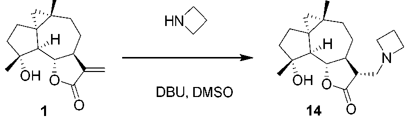

The preparation method is the same as that for compound 2. The yield of compound 14 is 88%. ¹H NMR (500 MHz, CDCl₃): δ 3.72 (t, *J* = 10.3 Hz, 1H), 3.22-3.14 (m, 4H), 2.75-2.68 (m, 2H), 2.37 (s, 1H), 2.17- 2.08 (m, 2H), 2.04-1.95 (m, 3H), 1.91-1.81 (m, 3H), 1.74 (d, *J* = 10.6 Hz, 1H), 1.50 (s, 3H), 1.46-1.24 (m, 3H), 1.07 (s, 3H), 0.74 (d, *J* = 3.7 Hz, 1H), 0.48 (d, *J* = 3.7 Hz, 1H). ESI-MS (m/z): [M+H]⁺ = 320.2 (calcd: 320.2).

### Preparation of Compound 15

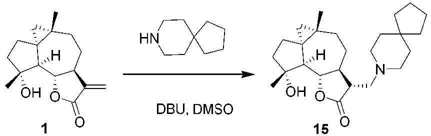

The preparation method is the same as that for compound 2. The yield of compound 15 is 88%. ¹H NMR (500 MHz, CDCl₃): δ 3.76 *(t, J=* 10.3 Hz, 1H), 2.74 (dd, *J* = 13.1, 4.4 Hz, 1H), 2.53 (dd, *J* = 13.1, 6.8 Hz, 1H), 2.40 (s, 3H), 2.34-2.28 (m, 2H), 2.17-2.02 (m, 3H), 1.87 (dd, *J* = 10.0, 3.1 Hz, 2H), 1.85-1.79 (m, 1H), 1.73 (d, *J* = 10.6 Hz, 1H), 1.56 (s, 3H), 1.51 (s, 3H), 1.44 -1.41 (m, 8H), 1.36 (d, *J* = 6.7 Hz, 4H), 1.08 (s, 3H), 1.03 (t, *J* = 12.5 Hz, 1H), 0.75 (d, *J* = 3.8 Hz, 1H), 0.48 (d, *J* = 3.7 Hz, 1H). ESI-MS (m/z): [M+H]⁺ =402.3 (calcd: 402.3).

### Preparation of Compound 16

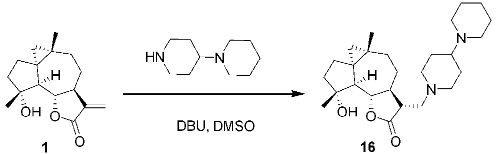

The preparation method is the same as that for compound 2. The yield of compound 16 is 82%. ¹H NMR (500 MHz, CDCl₃): δ 3.72 (t*, J* = 10.2 Hz, 1H), 2.81 (dd, *J* = 13.3, 4.4 Hz, 2H), 2.69 (dd, *J* = 13.2, 4.2 Hz, 1H), 2.50 (dd, *J* = 13.3, 7.0 Hz, 1H), 2.44 (m, 4H), 2.32- 2.25 (m, 1H), 2.18-2.07 (m, 2H), 2.05-1.97 (m, 2H), 1.91-1.80 (m, 3H), 1.79-1.67 (m, 4H), 1.57-1.50 (m, 4H), 1.47 (s, 3H), 1.45-1.37 (m, 4H), 1.36-1.20 (m, 2H), 1.04 (s, 3H), 0.98 (t, *J* = 12.7 Hz, 1H), 0.71 (d, *J* = 3.6 Hz, 1H), 0.45 (d, *J* = 3.5Hz, 1H). ESI-MS (m/z): [M+H]⁺ =431.3 (calcd: 431.3).

### Preparation of Compound 17

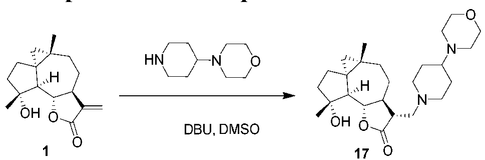

The preparation method is the same as that for compound 2. The yield of compound 17 is 82%. ¹H NMR (500 MHz, CDCl₃): δ 3.75 (t, *J* = 10.2 Hz, 1H), 3.69 (s, 4H), 2.87 (d, *J =* 11.1 Hz, 1H), 2.80 (d, *J* = 11.2 Hz, 1H), 2.72 (dd, *J* = 13.2, 3.7 Hz, 1H), 2.58-2.53 (m, 1H), 2.51 (s, 4H), 2.39 (s, 1H), 2.35 -2.28 (m, 1H), 2.14-2.04 (m, 4H), 1.93 (t, *J* = 11.8 Hz, 1H), 1.85 (d, *J* = 8.5 Hz, 2H), 1.80 (d, *J* = 10.9 Hz, 3H), 1.71 (d, *J* = 10.6 Hz, 1H), 1.49 (s, 3H), 1.41 (dd, 3H), 1.23 (dd, *J* = 13.5, 5.7 Hz, 1H), 1.07 (s, 3H), 1.00 (t, *J* = 13.1 Hz, 1H), 0.73 (d, *J* = 3.7 Hz, 1H), 0.47(d, *J* = 3.6 Hz, 1H). ESI-MS (m/z): [M+H]⁺ =433.3 (calcd: 433.3).

### Preparation of Compound 18

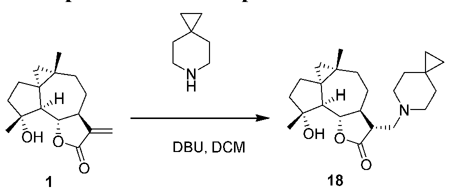

The preparation method is the same as that for compound 2. The yield of compound 18 is 90%.¹H NMR (500 MHz, CDCl₃): δ 3.78 (t, *J =* 10.2 Hz, 1H), 2.81 (d, *J* = 13.0 Hz, 1H), 2.60 (dd, *J* = 12.6, 6.2 Hz, 1H), 2.49 (s, 2H), 2.15 (d, *J* = 12.0 Hz, 2H), 2.07 (t, *J* = 11.6 Hz, 1H), 1.86-1.80 (m, 3H), 1.74 (d, *J* = 10.5 Hz, 1H), 1.52 (s, 3H), 1.50-1.39 (m, 3H), 1.33 (d, *J* = 20.0 Hz, 4H), 1.26 (d, *J* = 13.6 Hz, 1H), 1.09 (s, 3H), 1.04 (t, *J* = 12.6 Hz, 1H), 0.86 (d, *J* = 7.1 Hz, 1H), 0.76 (d, *J* = 3.8 Hz, 1H), 0.49 (d, *J* = 3.7Hz, 1H), 0.24 (s, 4H). ESI-MS (m/z): [M+H]⁺ =374.3 (calcd: 374.3).

### Preparation of Compound 19

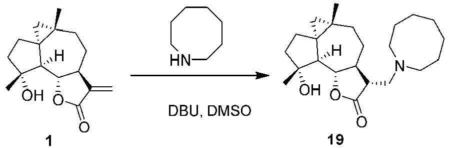

The preparation method is the same as that for compound 2. The yield of compound 19 is 83%. ¹H NMR (500 MHz, CDCl₃) δ 3.78 (t, *J* = 10.2 Hz, 1H), 2.89 (dd, *J* = 13.6, 4.4 Hz, 1H), 2.80 (dd,*J* = 13.6, 4.8 Hz, 1H), 2.61-2.58 (m, 1H), 2.58-2.51 (m, 4H), 2.38 (d, *J* = 9.0 Hz, 1H), 2.25-2.15 (m, 3H), 2.12-2.05 (m, 1H), 2.05-1.97 (m, 3H), 1.92-1.85 (m, 3H), 1.75 (d, *J* = 10.7 Hz, 1H), 1.68 (d, *J* = 10.9 Hz, 4H), 1.56 (s, 3H), 1.48-1.39 (m, 3H), 1.11 (s, 3H), 1.09-1.02 (m, 1H), 0.78 (d, *J* = 3.9 Hz, 1H), 0.50 (d, *J* = 3.7 Hz, 1H). ESI-MS (m/z): [M+H]⁺ =376.3 (calcd: 376.3).

### Preparation of Compound 20

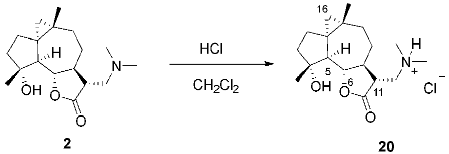

Compound 2 (307 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 2 (compound 20), yielding 90%. ¹H NMR (500 MHz, CD₃OD):δ4.15-4.10 (m, 1H, H₆), 3.42-3.37 (m, 1H),3.31-3.26 (m, 1H), 3.04-2.98 (m, 1H, H₁₁),2.91 (s, 6H), 2.21-2.12 (m, 2H), 2.02 (d, *J* = 15.0 Hz, 2H), 1.88 (t, *J* = 5.0 Hz, 2H), 1.78 (d, *J* = 10.0 Hz, 1H, H₅), 1.64-1.54 (m, 2H), 1.52 (s, 3H), 1.26 (t, *J* = 5.0 Hz, 1H), 1.14 (s, 3H), 1.10-1.05 (m, 1H), 0.76 (d, *J* = 5.0 Hz, 1H, H₁₆ₐ), 0.53 (d, *J* = 5.0 Hz, 1H, H_{16b})_{∘} The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]+ = 308.2 (calcd: 308.2).

### Preparation of Compound 21

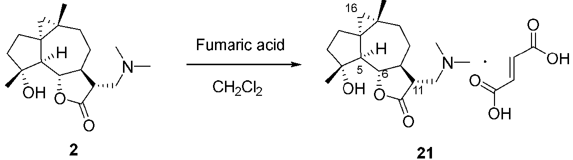

Fumaric acid was substituted for hydrochloric acid, and compound 21 was prepared by following the method used for synthesizing compound 2 hydrochloride. The yield was 80%. ¹H NMR (500 MHz, CD₃OD):δ6.72 (s, 2H), 4.13 (t, *J* = 10.0 Hz, 1H, H₆), 3.41-3.39 (m, 1H), 3.30-3.27 (m, 1H), 3.03-2.98 (m, 1H, H₁₁), 2.91 (s, 6H), 2.21-2.12 (m, 2H), 1.94-1.91 (m, 1H), 1.88 (t, *J* = 5.0 Hz, 2H), 1.84-1.80 (m, 1H), 1.78 (d, *J* = 15.0 Hz, 1H, H₅), 1.64-1.54 (m, 2H), 1.52 (s, 3H), 1.14 (s, 3H), 1.10-1.04 (m, 1H), 0.75 (d, *J* = 5.0 Hz, 1H, H₁₆ₐ), 0.52 (d, *J* = 5.0 Hz, 1H, H_{16b})_{∘} The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 308.2 (calcd: 308.2).

### Preparation of Compound 22

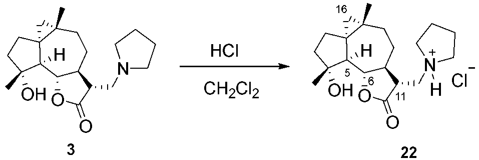

Compound 3 (333 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 3 (compound 22), yield 86%. ¹H NMR (500 MHz, CD₃OD): δ 3.76 (t, *J* = 10.2 Hz, 1H, H₆), 2.86-2.74 (m, 2H), 2.53-2.45 (m, 4H), 2.37-2.28 (m, 1H, H₁₁), 2.14 (dd, *J* = 14.3, 6.6 Hz, 1H), 1.93-1.83 (m, 3H), 1.76 (s, 1H), 1.75-1.68 (m, 5H), 1.51 (s, 3H), 1.48-1.37 (m, 2H), 1.29-1.24 (m, 1H), 1.09 (s, 3H), 1.03 (d, *J* = 13.1 Hz, 1H,H₅), 0.75 (d, *J* = 3.4 Hz, 1H,H₁₆ₐ), 0.49 (d, *J* = 3.4 Hz, 1H,H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]+ = 334.2 (calcd: 334.2).

### Preparation of Compound 23

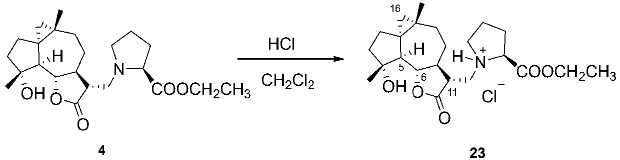

Compound 4 (404 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 4 (compound 23), yielding 83%. ¹H NMR (500 MHz, CD₃OD): δ 4.14 (d, *J* = 7.0 Hz, 2H), 3.78 (t, *J*= 10.3 Hz, 1H,H₆), 3.15 (dd, *J* = 13.4, 5.0 Hz, 1H), 3.00-2.91 (m, 2H), 2.46-2.42 (m, 1H), 2.36 (s, 1H), 2.29 (d, *J* = 11.4 Hz, 1H,H₁₁), 2.16 (dd, *J* = 14.6, 6.6 Hz, 1H), 2.11-2.04 (m, 3H), 1.93-1.84 (m, 4H), 1.78 (d, *J* = 10.4 Hz, 1H,H₅), 1.52 (s, 3H), 1.49-1.37(m, 3H), 1.26 (t, *J* = 7.4 Hz, 4H), 1.10 (s, 4H), 0.78 (d, *J* = 3.6 Hz, 1H, H₁₆ₐ), 0.50 (d, *J* = 3.6 Hz, 1H, H_{16b}). The ROESY diagram shows hydrogen signals for H₅ and H₁₆ₐ, as well as H₆ and H₁₁, are correlated, confirming the cyclopropane configuration is α configuration and the 11-H configuration is β configuration. ESI-MS (m/z): [M+H]⁺ = 406.2 (calcd: 406.2).

### Preparation of Compound 24

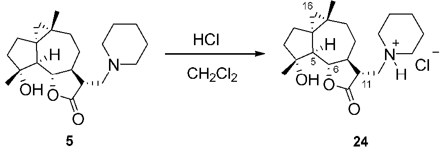

Compound 5 (346 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 5 (compound 24), yielding 87%. ¹H NMR (500 MHz, CD₃OD): δ 3.79 (t, *J=* 10.5 Hz, 1H, H₆), 2.79-2.75(m, 1H), 2.56-2.53(m, 1H, H₁₁), 2.46-2.40(m, 3H),2.39-2.32(m, 3H), 2.19-2.08(m, 4H),1.94-1.85(m, 3H),1.77(d, *J*=10.4 Hz, 1H, H₅), 1.54 (s,6H), 1.45-1.39 (m, 4H), 1.12(s, 3H), 1.09-1.04 (m, 1H), 0.79(d, *J*=4.0 Hz, 1H, H₁₆ₐ), 0.52(d, *J*=4.0Hz, 1H, H_{16b}). The ROESY diagram shows hydrogen signals for H₅ and H₁₆ₐ, as well as H₆ and H₁₁, are correlated, confirming the cyclopropane configuration is α configuration and the 11-H configuration is β configuration. ESI-MS (m/z): [M+H]⁺ = 348.2 (calcd: 348.2).

### Preparation of Compound 25

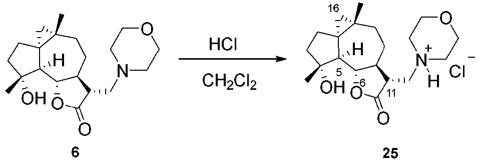

Compound 6 (348 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 6 (compound 25), yield 88%. ¹H NMR (500 MHz, CD₃OD): δ3.81 (t, *J*= 10.5 Hz, 1H, H₆), 3.74-3.67 (m, 3H), 2.82-2.79 (m, 1H, H₁₁), 2.65-2.61(m, 1H), 2.53-2.44 (m,5H), 2.40-2.35 (m,1H), 2.21-2.17 (m, 1H), 2.13-2.07 (m, 2H), 1.95-1.86 (m, 3H), 1.78 (d, *J*= 10.4 Hz, 1H, H₅), 1.55(s, 3H), 1.52-1.39 (m, 2H), 1.31-1.27 (m, 1H), 1.12 (s, 3H), 1.09-1.04 (m, 1H), 0.80 (d, *J*= 4.0 Hz, 1H, H₁₆ₐ), 0.54 (d, *J*= 4.0 Hz, 1H, H_{16b}). The ROESY diagram shows hydrogen signals for H₅ and H₁₆ₐ, as well as H₆ and H₁₁, are correlated, confirming the cyclopropane configuration is α configuration and the 11-H configuration is β configuration. ESI-MS (m/z): [M+H]⁺ = 350.2 (calcd: 350.2).

### Preparation of Compound 26

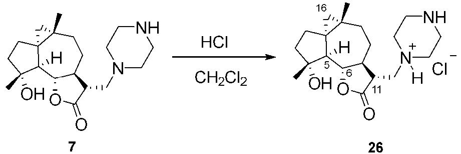

Compound 7 (347 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 7 (compound 26), yielding 86%. ¹H NMR (500 MHz, CD₃OD): δ3.79 (t, *J*= 10.5 Hz, 1H, H₆), 2.82-2.79(m, 1H), 2.64-2.55 (m, 1H, H₁₁), 2.55-2.42(m, 5H), 2.38-2.33 (m, 2H), 2.29(s, 3H), 2.20-2.16(m, 1H), 2.15-2.05(m, 2H), 1.93-1.83(m, 3H),1.76 (d,*J*= 10.4 Hz, 1H, H₅), 1.54 (s, 3H), 1.49-1.38(m, 2H), 1.29-1.25 (m, 1H), 1.12 (s, 3H), 1.09-1.03(m, 1H), 0.78(d, *J* = 4.0 Hz, 1H, H₁₆ₐ), 0.52 (d, *J*= 4.0Hz, 1H, H_{16b}). The ROESY diagram shows hydrogen signals for H₅ and H₁₆ₐ, as well as H₆ and H₁₁, are correlated, confirming the cyclopropane configuration is α configuration and the 11-H configuration is β configuration. ESI-MS (m/z): [M+H]⁺= 349.2 (calcd: 349.2).

### Preparation of Compound 27

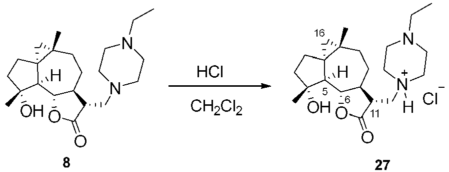

Compound 8 (375 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 8 (compound 27), yield 88%. ¹H NMR (500 MHz, CD₃OD): δ 3.75 (t, *J* = 10.2 Hz, 1H,H₆), 2.77 (dd, *J* = 13.2, 4.2 Hz, 1H), 2.58 (dd, *J* = 13.2, 6.8 Hz, 2H), 2.47 (d, *J* = 13.9 Hz, 4H), 2.41-2.35 (m, 4H), 2.32 (dd, *J* = 12.1, 6.5 Hz, 1H,H₁₁), 2.26-2.17 (m, 4H), 1.92-1.87 (m, 3H), 1.72 (d, *J* = 10.6 Hz, 1H,H₅), 1.50 (s, 3H), 1.47-1.20 (m, 3H), 1.08 (s, 3H), 1.06-0.95 (m, 3H), 0.74 (d, *J* = 3.7 Hz, 1H, H₁₆ₐ), 0.48 (d, *J* = 3.5 Hz, 1H, H_{16b}). The ROESY diagram shows signal correlation between H₅ and H₁₆ₐ, as well as between H₆ and H₁₁, confirming the cyclopropane configuration is α configuration and the 11-H configuration is β configuration. ESI-MS (m/z): [M+H]⁺ = 378.3 (calcd: 378.3).

### Preparation of Compound 28

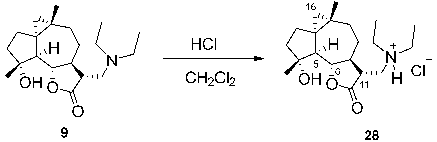

Compound 9 (334 mg, 1 mmol) was dissolved in dichloromethane (2 mL) and stirred at room temperature for 2 h. A hydrochloric acid solution was then added dropwise until the pH reached 4-5. The mixture was filtered, and the solid was washed with dichloromethane. The resulting oily liquid was the hydrochloride salt of compound 9 (compound 28), yielding 86%. ¹H NMR (500 MHz, CD₃OD): δ 3.77 (t, *J* = 10.3 Hz, 1H,H₆), 2.89 (dd, *J* = 13.6, 4.6 Hz, 1H), 2.63 (dd, *J* = 13.7, 6.6 Hz, 1H,H₁₁), 2.58-2.50 (m, 2H), 2.45 (m, *J* = 6.8 Hz, 2H), 2.38 (s, 1H), 2.29 (m, *J* = 11.7, 5.6 Hz, 1H), 2.19-2.13 (m, 2H), 2.11-2.04 (m, 1H), 1.91-1.78 (m, 3H), 1.75 (d, *J* = 10.5 Hz, 1H,H₅), 1.52 (s, 3H), 1.50-1.36 (m, 2H), 1.12 (s, 3H), 1.05 (d, *J* = 13.1 Hz, 1H), 0.98 (t, *J* = 7.1 Hz, 6H), 0.76 (d, *J* = 4.0 Hz, 1H,H₁₆ₐ), 0.49 (d, *J* = 4.0 Hz, 1H,H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 336.2 (calcd: 336.2).

### Preparation of Compound 29

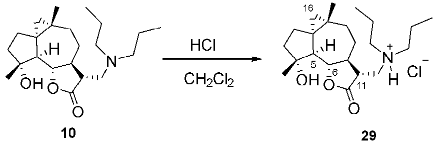

Compound 10 (362 mg, 1 mmol) was dissolved in dichloromethane (2 mL) and stirred at room temperature for 2 h. A hydrochloric acid solution was then added dropwise until the pH reached 4-5. The mixture was filtered, and the solid was washed with dichloromethane. The resulting oily liquid was the hydrochloride salt of compound 10 (compound 29), yielding 85%. ¹H NMR (500 MHz, CD₃OD): δ 3.78 (t, *J* = 10.2 Hz, 1H,H₆), 2.87-2.80 (m, 1H), 2.66 (dd, *J* = 13.5, 6.6 Hz, 1H,H₁₁), 2.38 (d, *J* = 12.2 Hz, 3H), 2.34-2.25 (m, 3H), 2.15 (d, *J* = 11.7 Hz, 2H), 2.11-2.04 (m, 1H), 1.93-1.82 (m, 3H), 1.75 (d, *J* = 10.6 Hz, 1H,H₅), 1.52 (s, 3H), 1.45-1.38 (m, 5H), 1.10 (s, 3H), 1.04 (t, *J* = 12.2 Hz, 1H), 0.85 (t, *J* = 7.3 Hz, 6H), 0.76 (d, *J* = 3.9 Hz, 1H,H₁₆ₐ), 0.50 (d, *J* = 3.9 Hz, 1H,H_{16b}). The ROESY diagram shows hydrogen signals for H₅ and H₁₆ₐ, as well as H₆ and H₁₁, are correlated, confirming the cyclopropane configuration is α configuration and the 11-H configuration is β configuration. ESI-MS (m/z): [M+H]⁺ = 364.3 (calcd: 364.3).

### Preparation of Compound 30

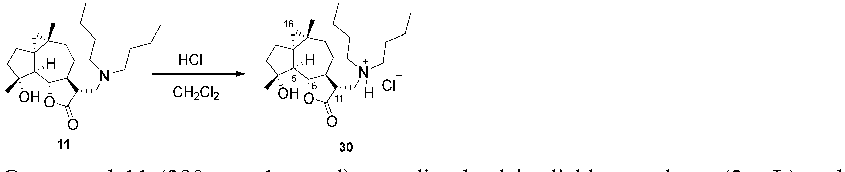

Compound 11 (390 mg, 1 mmol) was dissolved in dichloromethane (2 mL) and stirred at room temperature for 2 h. A hydrochloric acid solution was then added dropwise until the pH reached 4-5. The mixture was filtered, and the solid was washed with dichloromethane. The resulting oily liquid was the hydrochloride salt of compound 11 (compound 30), yielding 82%. ¹H NMR (500 MHz, CD₃OD): δ 3.78 (t*, J* = 10.2 Hz, 1H,H₆), 2.82 (dd, *J* = 13.6, 4.2 Hz, 1H), 2.64 (dd, *J* = 13.5, 6.6 Hz, 1H,H₁₁), 2.46-2.42 (m, 2H), 2.38-2.24 (m, 4H), 2.19-2.04 (m, 3H), 1.93-1.82 (m, 3H), 1.75 (d, *J* = 10.6 Hz, 1H,H₅), 1.52 (s, 3H), 1.43-1.32 (m, 5H), 1.28 (p, *J* = 7.1 Hz, 5H), 1.10 (s, 3H), 0.90 (t, *J* = 7.3 Hz, 6H), 0.77 (d, *J* = 3.8 Hz, 1H,H₁₆ₐ), 0.50 (d, *J* = 3.8 Hz, 1H,H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 392.3 (calcd: 392.3).

### Preparation of Compound 31

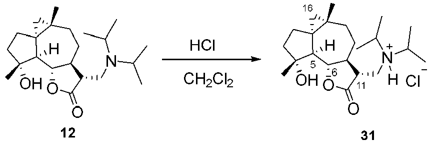

Compound 12 (362 mg, 1 mmol) was dissolved in dichloromethane (2 mL) and stirred at room temperature for 2 h. A hydrochloric acid solution was then added dropwise until the pH reached 4-5. The mixture was filtered, and the solid was washed with dichloromethane. The resulting oily liquid was the hydrochloride salt of compound 12 (compound 31), yielding 80%. ¹H NMR (500 MHz, CD₃OD): δ 3.78 (t, *J* = 10.3 Hz, 1H,H₆), 2.83 (d, *J* = 8.7 Hz, 1H), 2.65 (dd, *J* = 13.2, 6.2 Hz, 1H,H₁₁), 2.38 (m, *J* = 12.8, 8.8 Hz, 3H), 2.14 (d, *J* = 12.2 Hz, 2H), 2.11-2.01 (m, 1H), 1.88 (q, *J* = 10.0, 7.8 Hz, 3H), 1.74 (d, *J* = 10.5 Hz, 1H,H₅), 1.51 (s, 3H), 1.49-1.35 (m, 6H), 1.09 (s, 3H), 1.05 (t, *J* = 12.2 Hz, 1H), 0.85 (q, *J* = 9.0, 7.2 Hz, 6H), 0.75 (d, *J* = 3.6 Hz, 1H,H₁₆ₐ), 0.49 (d, *J* = 3.5 Hz, 1H,H_{16b}), 0.06 (s, 2H). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 364.3 (calcd: 364.3).

### Preparation of Compound 32

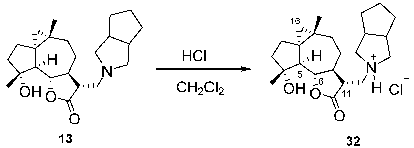

Compound 13 (375 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 13 (compound 32), yielding 81%. ¹H NMR (500 MHz,CD₃OD): δ 3.76 (t, *J* = 10.3 Hz, 1H,H₆), 2.76-2.66 (m, 2H), 2.62-2.47 (m, 4H), 2.38 (s, 1H), 2.31-2.25 (m, 1H,H₁₁), 2.20-2.03 (m, 5H), 1.94-1.83 (m, 3H), 1.74 (d, *J* = 10.6 Hz, 1H,H₅), 1.67-1.55 (m, 3H), 1.51 (s, 3H), 1.49-1.37 (m, 3H), 1.33 (dd, *J* = 11.0, 6.7 Hz, 2H), 1.09 (s, 3H), 0.76 (d, *J* = 3.6 Hz, 1H,H₁₆ₐ), 0.49 (d, *J* = 3.6 Hz, 1H,H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 374.3 (calcd: 374.3).

### Preparation of Compound 33

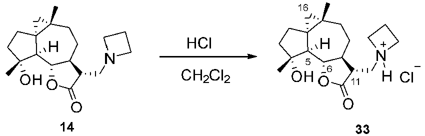

Compound 14 (318 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 14 (compound 33), yielding 82%. ¹H NMR (500 MHz, CD₃OD): δ 3.72 (t, *J* = 10.3 Hz, 1H, H₆), 3.22-3.14 (m, 4H), 2.75-2.68 (m, 1H, H₁₁), 2.37 (s, 1H), 2.17-2.08 (m, 3H), 2.04-1.95 (m, 3H), 1.91-1.81 (m, 3H), 1.74 (d, *J* = 10.6 Hz, 1H, H₅), 1.50 (s, 3H), 1.46-1.24 (m, 3H), 1.07 (s, 3H), 0.74 (d, *J* = 3.7 Hz, 1H, H₁₆ₐ), 0.48 (d, *J* = 3.7 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 320.2 (calcd: 320.2).

### Preparation of Compound 34

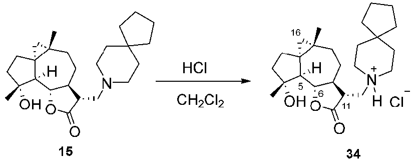

Compound 15 (400 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 15 (compound 34), yielding 81%. ¹H NMR (500 MHz, CD₃OD): δ 3.76 (t, *J* = 10.3 Hz, 1H,H₆), 2.74 (dd, *J* = 13.1, 4.4 Hz, 1H), 2.53 (dd, *J* = 13.1, 6.8 Hz, 1H,H₁₁), 2.40 (s, 3H), 2.34-2.28 (m, 2H), 2.17-2.02 (m, 3H), 1.87 (dd, *J* = 10.0, 3.1 Hz, 2H), 1.85-1.79 (m, 1H), 1.73 *(d, J* = 10.6 Hz, 1H,H₅), 1.56 (s, 3H), 1.51 (s, 3H), 1.44 - 1.41 (m, 8H), 1.36 (d, *J* = 6.7 Hz, 4H), 1.08 (s, 3H), 1.03 (t, *J* = 12.5 Hz, 1H), 0.75 (d, *J* = 3.8 Hz, 1H, H₁₆ₐ), 0.48 (d, *J* = 3.7 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 402.3 (calcd: 402.3).

### Preparation of Compound 35

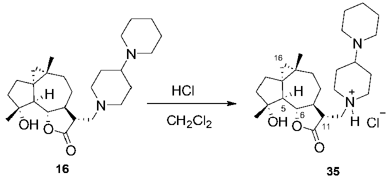

Compound 16 (429 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 16 (compound 35), yielding 83%.¹H NMR (500 MHz, CD₃OD): δ 3.72 (t, *J* = 10.2 Hz, 1H,H₆), 2.81 (dd, *J* = 13.3, 4.4 Hz, 2H), 2.69 (dd, *J* = 13.2, 4.2 Hz, 1H,H₁₁), 2.50 (dd, *J* = 13.3, 7.0 Hz, 1H), 2.44 (m, 4H), 2.32- 2.25 (m, 1H), 2.18-2.07 (m, 2H), 2.05-1.97 (m, 2H), 1.91-1.80 (m, 3H), 1.73 (d, *J* = 10.6 Hz, 1H,H₅), 1.73-1.67 (m, 3H), 1.57-1.50 (m, 4H), 1.47 (s, 3H), 1.45-1.37 (m, 4H), 1.36-1.20 (m, 2H), 1.04 (s, 3H), 0.98 (t, *J* = 12.7 Hz, 1H), 0.71 (d, *J* = 3.6 Hz, 1H, H₁₆ₐ), 0.45 (d, *J* = 3.5 Hz, 1H, H_{16b}). ESI-MS (m/z): [M+H]⁺ = 431.3 (calcd: 431.3). The ROESY diagram shows hydrogen signals from H₅ and H₁₆ₐ, as well as H₆ and H₁₁, are correlated, confirming the cyclopropane configuration is α configuration and the 11-H configuration is β configuration.

### Preparation of Compound 36

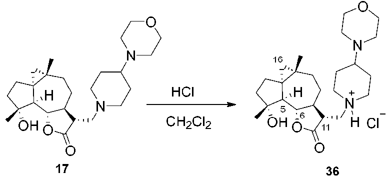

Compound 17 (431 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 17 (compound 36), yielding 84%. ¹H NMR (500 MHz, CD₃OD): δ 3.75 (t, *J* = 10.2 Hz, 1H,H₆), 3.69 (s, 4H), 2.87 (d, *J* = 11.1 Hz, 1H), 2.80 (d, *J* = 11.2 Hz, 1H), 2.72 (dd, *J* = 13.2, 3.8 Hz, 1H), 2.58-2.53 (m, 1H,H₁₁), 2.51 (s, 4H), 2.39 (s, 1H), 2.35-2.28 (m, 1H), 2.14-2.04 (m, 4H), 1.93 (t, *J* = 11.8 Hz, 1H), 1.85 (d, *J* = 8.4 Hz, 2H), 1.80 (*d, J* = 10.8 Hz, 3H), 1.71 (*d, J* = 10.6 Hz, 1H,H₅), 1.49 (s, 3H), 1.42-1.38 (m, 3H), 1.23 (dd, *J* = 13.4, 5.8 Hz, 1H), 1.07 (s, 3H), 1.00 (t, *J* = 13.1 Hz, 1H), 0.73 (d, *J* = 3.7 Hz, 1H, H₁₆ₐ), 0.47(d, *J* = 3.6 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 433.3 (calcd: 433.3).

### Preparation of Compound 37

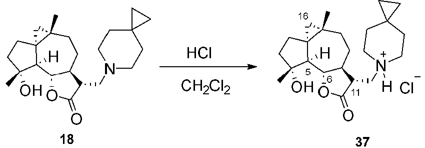

Compound 18 (372 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 18 (compound 37), yielding 92%. ¹H NMR (500 MHz, CD₃OD): δ 3.78 (t*, J* = 10.2 Hz, 1H,H₆), 2.81 (d, *J* = 13.0 Hz, 1H), 2.60 (dd, *J* = 12.6, 6.2 Hz, 1H), 2.49 (s, 2H), 2.15 (d, *J* = 12.0 Hz, 2H), 2.07 (t, *J* = 11.6 Hz, 1H,H₁₁), 1.86 (m, 3H), 1.74 (d, *J* = 10.5 Hz, 1H,H₅), 1.52 (s, 3H), 1.50-1.39 (m, 3H), 1.33 (d, *J* = 20.0 Hz, 4H), 1.26 (d, *J* = 13.6 Hz, 1H), 1.09 (s, 3H), 1.04 (t, *J* = 12.6 Hz, 1H), 0.86 (d, *J* = 7.1 Hz, 1H), 0.76 (d, *J* = 3.8 Hz, 1H,H₁₆ₐ), 0.49 (d, *J* = 3.7Hz, 1H,H_{16b}), 0.24 (s, 4H). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 374.3 (calcd: 374.3).

### Preparation of Compound 38

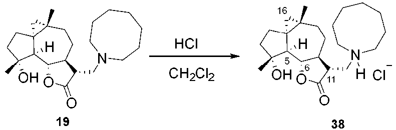

Compound 19 (374 mg, 1 mmol) was dissolved in dichloromethane (2 mL) and stirred at room temperature for 2 h. A hydrochloric acid solution was then added dropwise until the pH reached 4-5. The mixture was filtered, and the solid was washed with dichloromethane. The resulting oily liquid was the hydrochloride salt of compound 19 (compound 38), yield 80%.¹H NMR (500 MHz, CD₃OD): δ 3.78 (t, *J* = 10.2 Hz, 1H,H₆), 2.97 (dd, *J* = 13.6, 4.4 Hz, 1H), 2.80 (dd, *J* = 13.6, 4.8 Hz, 1H,H₁₁), 2.61-2.58 (m, 1H), 2.58-2.55 (m, 4H), 2.38 (d, *J* = 9.0 Hz, 1H), 2.25-2.15 (m, 3H), 2.12-2.05 (m, 1H), 2.05-1.97 (m, 3H), 1.92-1.85 (m, 3H), 1.75 (d, *J =* 10.7 Hz, 1H,H₅), 1.68 (d, *J =* 10.9 Hz, 4H), 1.56 (s, 3H), 1.48-1.39 (m, 3H), 1.11 (s, 3H), 1.09-1.02 (m, 1H), 0.78 (d, *J* = 3.9 Hz, 1H, H₁₆ₐ), 0.50 (d, *J =* 3.7 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 376.3 (calcd: 376.3).

### Preparation of Compound 39

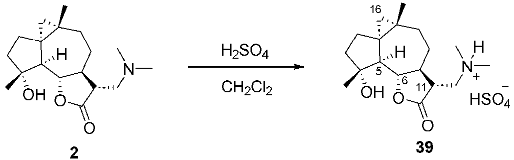

Replace hydrochloric acid with sulfuric acid to prepare the sulfate salt (Compound 39) following the method used for synthesizing the hydrochloride salt of Compound 2. Yield: 75%.¹H NMR (500 MHz, CD₃OD):δ4.13-4.11 (m, 1H, H₆), 3.41-3.35 (m, 1H), 3.31-3.27 (m, 1H), 3.03-2.99 (m, 1H, H₁₁),2.92 (s, 6H), 2.21-2.10 (m, 2H), 2.00 (d, *J =* 15.0 Hz, 2H), 1.85 (t, *J =* 5.0 Hz, 2H), 1.77 (d, *J =* 10.0 Hz, 1H, H₅), 1.62-1.53 (m, 2H), 1.51 (s, 3H), 1.25 (t, *J =* 5.0 Hz, 1H), 1.13 (s, 3H), 1.10-1.03 (m, 1H), 0.75 (d, *J =* 5.0 Hz, 1H, H₁₆ₐ), 0.52 (d, *J =* 5.0 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 308.2 (calcd: 308.2).

### Preparation of Compound 40.

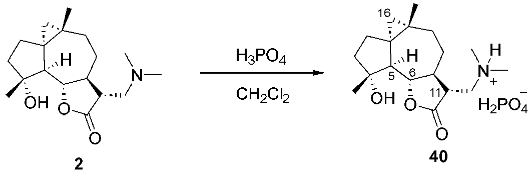

Replace hydrochloric acid with phosphoric acid and prepare the phosphate (Compound 40) by following the method used for synthesizing the hydrochloride salt of Compound 2. Yield: 88%. ¹H NMR (500 MHz, CD₃OD): δ4.14-4.11 (m, 1H, H₆), 3.43-3.36 (m, 1H), 3.31-3.28 (m, 1H), 3.04-2.99 (m, 1H, H₁₁),2.93 (s, 6H), 2.21-2.11 (m, 2H), 2.01 (d, *J* = 15.0 Hz, 2H), 1.86 (t, *J* = 5.0 Hz, 2H), 1.78 (d, *J* = 10.0 Hz, 1H, H₅), 1.63-1.54 (m, 2H), 1.52 (s, 3H), 1.26 (t, *J* = 5.0 Hz, 1H), 1.14 (s, 3H), 1.10-1.04 (m, 1H), 0.75 (d, *J* = 5.0 Hz, 1H, H₁₆ₐ), 0.52 (d, *J* = 5.0 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 308.2 (calcd: 308.2).

### Preparation of Compound 41

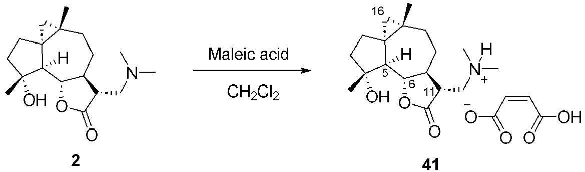

Replace hydrochloric acid with maleic acid and prepare the maleate salt (Compound 41) by following the method used for synthesizing the hydrochloride salt of Compound 2. Yield: 83%. ¹H NMR (500 MHz, CD₃OD): δ6.27 (s, 2H), 4.14-4.10 (m, 1H, H₆), 3.43-3.35 (m, 1H),3.30-3.27 (m, 1H), 3.03-2.97 (m, 1H, H₁₁),2.91 (s, 6H), 2.20-2.11 (m, 2H), 2.00 (d, *J =* 15.0 Hz, 2H), 1.85 (t, *J =* 5.0 Hz, 2H), 1.76 (d, *J* = 10.0 Hz, 1H, H₅), 1.62-1.52 (m, 2H), 1.52 (s, 3H), 1.25 (t, *J* = 5.0 Hz, 1H), 1.13 (s, 3H), 1.10-1.03 (m, 1H), 0.74 (d, *J* = 5.0 Hz, 1H, H₁₆ₐ), 0.51 (d, *J* = 5.0 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 308.2 (calcd: 308.2).

### Preparation of Compound 42

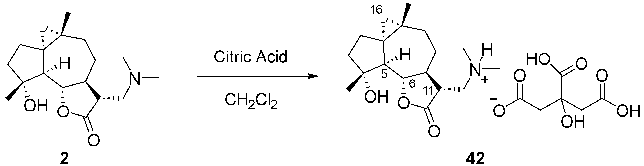

Replace hydrochloric acid with citric acid and prepare the citrate salt (Compound 42) by following the method used for synthesizing the hydrochloride salt of Compound 2. Yield: 79%. ¹H NMR (500 MHz, CD₃OD): δ4.12 (t, *J* = 10.0 Hz, 1H, H₆), 3.40-3.39 (m, 1H), 3.29-3.26 (m, 1H), 3.02-2.97 (m, 1H, H₁₁), 2.90 (s, 6H), 2.73 (d, *J =* 5.2 Hz, 2H), 2.55 (d, *J* = 5.2 Hz, 2H), 2.20-2.12 (m, 2H), 1.93-1.91 (m, 1H), 1.87 (t, *J =* 5.0 Hz, 2H), 1.83-1.79 (m, 1H), 1.78 (d, *J* = 15.0 Hz, 1H, H₅), 1.62-1.53 (m, 2H), 1.51 (s, 3H), 1.13 (s, 3H), 1.09-1.02 (m, 1H), 0.76 (d, *J =* 5.0 Hz, 1H, H₁₆ₐ), 0.53 (d, *J =* 5.0 Hz, 1H, H_{16b}). The ROESY diagram shows hydrogen signals from H₅ and H₁₆ₐ, as well as H₆ and H₁₁, are correlated, confirming the cyclopropane configuration is α configuration and the 11-H configuration is β configuration. ESI-MS (m/z): [M+H]⁺ = 308.2 (calcd: 308.2).

### Compounds 43-63 were prepared according to the method described in CN 115403546 A.

### Preparation of Compound 64:

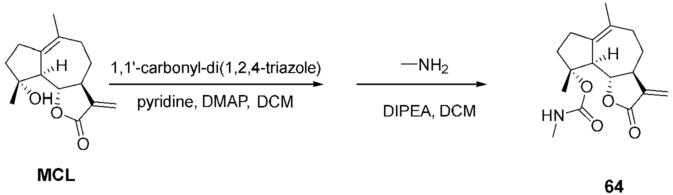

Add sequentially to a 5 mL reaction flask: MCL magnolol (100 mg, 0.40 mmol), dichloromethane (1.0 mL), N, N-carbonyldi(1,2,4-triazole) (131 mg, 0.80 mmol), pyridine (64 mg, 0.80 mmol), and 4-dimethylaminopyridine (4 mg, 0.04 mmol). Stir at room temperature until TLC analysis confirmed reaction completion, yielding a crude intermediate solution. To the above solution, sequentially add N, N-diisopropylethylamine (52 mg, 0.4 mmol), methylamine aqueous solution (47 µL, 0.60 mmol, 40% aqueous solution), and stir at room temperature until reaction completion as determined by TLC. The reaction mixture was diluted with ethyl acetate (10 mL) and sequentially extracted with water (10 mL × 3), saturated citric acid solution (10 mL × 3; use saturated copper sulfate solution if the subsequent product contains basic groups), saturated sodium bicarbonate solution (10 mL × 3), and saturated saline solution (10 mL × 3). After drying over anhydrous sodium sulfate, the mixture was concentrated under reduced pressure. Separation by silica gel column chromatography yielded compound 64 in 72% yield. ¹H NMR (500 MHz, CDCl₃): *δ* 6.19 (d, *J* = 3.5 Hz, 1H), 5.47 (d, *J =* 3.0 Hz, 1H), 4.73 (s, 1H), 3.80 (t, *J =* 10.5 Hz, 1H), 3.11 (d, *J* = 9.5 Hz, 1H), 2.74 (d, *J =* 4.5 Hz, 3H), 2.70-2.65 (m, 1H), 2.48-2.43 (m, 2H), 2.28-2.20 (m, 3H), 2.11-2.02 (m, 2H), 1.72 (s, 3H), 1.53 (s, 3H), 1.40-1.32 (m, 1H). ESI-MS (m/z): [M+H]⁺ = 306.16 (calcd: 306.16).

### Preparation of Compound 65:

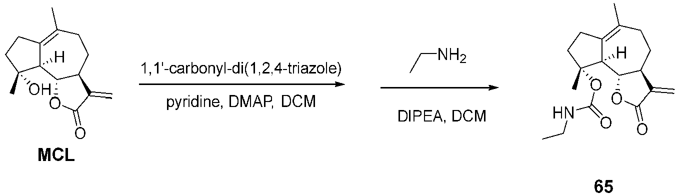

The preparation method for compound 65 is similar to that of compound 64, yielding a 72% yield.¹H NMR (500 MHz, CDCl₃): *δ* 6.20 (d, *J =* 3.5 Hz, 1H), 5.48 (d, *J* = 3.0 Hz, 1H), 4.76 (s, 1H), 3.80 (t, *J =* 10.5 Hz, 1H), 3.21-3.11 (m, 3H), 2.76-2.66 (m, 1H), 2.48-2.43 (m, 2H), 2.28-2.20 (m, 3H), 2.12-2.06 (m, 2H), 1.72 (s, 3H), 1.53 (s, 3H), 1.40-1.32 (m, 1H), 1.14 (t, *J =* 7.0 Hz, 3H).ESI-MS (m/z): [M+H]⁺ = 320.18 (calcd: 320.18)_{∘}

### Preparation of Compound 66:

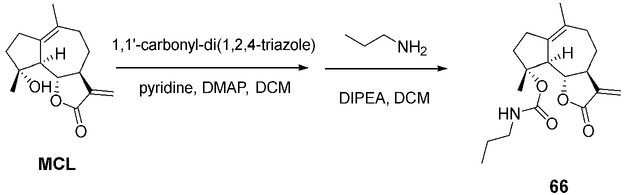

The preparation method for compound 66 is similar to that of compound 64, yielding a 76% yield. ¹H NMR (500 MHz, CDCl₃):5 6.20 (d, *J =* 3.5 Hz, 1H), 5.47 (d, *J =* 3.0 Hz, 1H), 4.80 (s, 1H), 3.80 (t, *J =* 10.5 Hz, 1H), 3.14-3.05 (m, 3H), 2.70-2.66 (m, 1H), 2.48-2.41 (m, 2H), 2.27-2.21 (m, 3H), 2.11-2.05 (m, 2H), 1.72 (s, 3H), 1.53 (s, 3H), 1.55-1.48 (m, 2H), 1.40-1.32 (m, 1H), 0.92 *(t, J =* 7.5 Hz, 3H)_{∘} ESI-MS (m/z): [M+H]⁺ = 334.19 (calcd: 334.19)_{∘}

### Preparation of Compound 67:

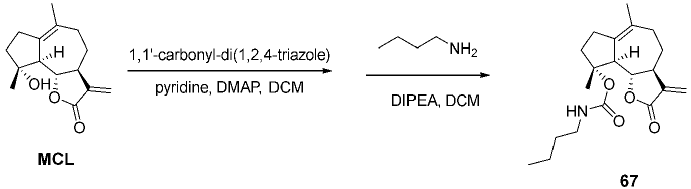

The preparation method for compound 67 is similar to that of compound 64, yielding a 74% yield. ¹H NMR (500 MHz, CDCl₃):*δ* 6.17 (d, *J* = 3.5 Hz, 1H), 5.46 (d, *J* = 3.0 Hz, 1H), 4.78 (s, 1H), 3.79 (t, *J =* 10.5 Hz, 1H), 3.17-3.10 (m, 3H), 2.69-2.64 (m, 1H), 2.46-2.41 (m, 2H), 2.26-2.18 (m, 3H), 2.10-2.04 (m, 2H), 1.71 (s, 3H), 1.51 (s, 3H), 1.48-1.44 (m, 2H), 1.37-1.31 (m, 3H), 0.91 (t, *J =* 7.5 Hz, 3H)_{∘} ESI-MS (m/z): [M+H]⁺ = 348.21 (calcd: 348.21)_{∘}

### Preparation of Compound 68:

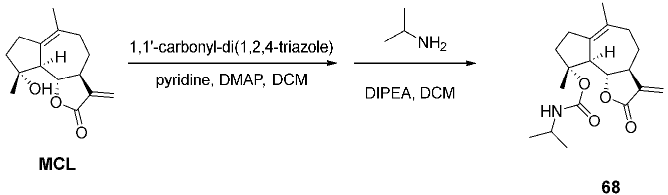

The preparation method for compound 68 is similar to that of compound 64, yielding a 41% yield. ¹H NMR (500 MHz, CDCl₃):6 6.20 (d, *J* = 3.5 Hz, 1H), 5.48 (d, *J* = 3.0 Hz, 1H), 4.66 (s, 1H), 3.83-3.74 (m, 2H), 3.13 (d, *J =* 10.0 Hz, 1H), 2.71-2.66 (m, 1H), 2.48-2.44 (m, 2H), 2.29-2.20 (m, 3H), 2.13-2.05 (m, 2H), 1.73 (s, 3H), 1.53 (s, 3H), 1.41-1.33 (m, 1H), 1.16 (t, *J =* 7.0 Hz, 6H)_{∘} ESI-MS (m/z): [M+H]⁺ = 334.19 (calcd: 334.19)_{∘}

### Preparation of Compound 69:

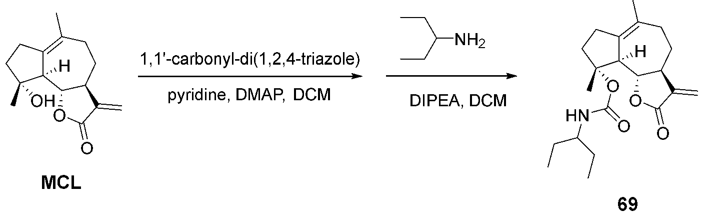

The preparation method for compound 69 is similar to that of compound 64, yielding a 33% yield. ¹H NMR (500 MHz, CDCl₃): *δ* 6.16 (d, *J* = 3.5 Hz, 1H), 5.44 (d, *J* = 3.0 Hz, 1H), 4.51 (d, *J* = 9.0 Hz, 1H), 3.77 (t, *J =* 10.5 Hz, 1H), 3.39-3.38 (m, 1H), 3.19-3.17 (m, 1H), 2.68-2.63 (m, 1H), 2.45-2.34 (m, 2H), 2.24-2.21 (m, 3H), 2.17-2.06 (m, 2H), 1.70 (s, 3H), 1.54-1.47 (m, 2H), 1.49 (s, 3H), 1.41-1.30 (m, 3H), 0.92-0.86 (m, 6H)_{∘} ESI-MS (m/z): [M+H]⁺ = 362.23 (calcd: 362.23)_{∘}

### Preparation of Compound 70:

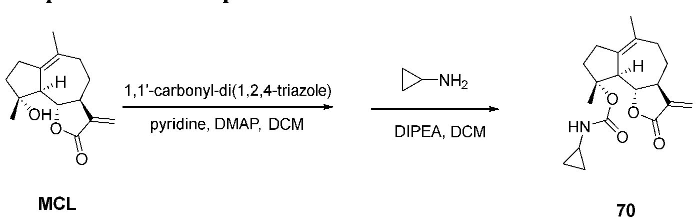

The preparation method for compound 70 is similar to that of compound 64, yielding a 69% yield. ¹H NMR (500 MHz, CDCl₃) *δ* 6.19 (d, *J* = 3.3 Hz, 1H), 5.47 (d, *J* = 3.0 Hz, 1H), 5.01 (s, 1H), 3.80 (t, *J* = 10.1 Hz, 1H), 3.09 (s, 1H), 2.71-2.62 (m, 1H), 2.56 (s, 1H), 2.50-2.41 (m, 2H), 2.32-2.16 (m, 3H), 2.13-1.99 (m, 2H), 1.72 (s, 3H), 1.53 (s, 3H), 1.39-1.32 (m, 1H), 0.69 (d, *J =* 6.8 Hz, 2H), 0.53 (s, 2H)_{∘} ESI-MS (m/z): [M+H]⁺ = 332.18 (calcd: 332.18)_{∘}

### Preparation of Compound 71:

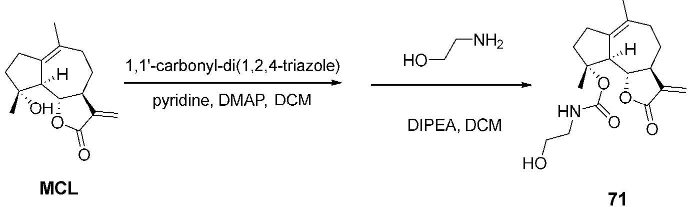

The preparation method for compound 71 is similar to that of compound 64, yielding a 71% yield. ¹H NMR (500 MHz, CDCl₃): *δ* 6.13 (d, *J* = 3.5 Hz, 1H), 5.42 (d, *J* = 3.0 Hz, 1H), 5.30 (s, 1H), 4.66 (s, 1H), 3.75 (t, *J =* 10.5 Hz, 1H), 3.63-3.61 (m, 1H), 3.24-3.20 (m, 1H), 3.13-3.11 (m, 1H), 2.63-2.59 (m, 1H), 2.54-2.47 (m, 2H), 2.40-2.28 (m, 2H), 2.20-2.15 (m, 3H), 2.06-1.98 (m, 2H), 1.64 (s, 3H), 1.42 (s, 3H), 1.33-1.26 (m, 1H)_{∘} ESI-MS (m/z): [M+H]⁺ = 336.17 (calcd: 336.17)_{∘}

### Preparation of Compound72:

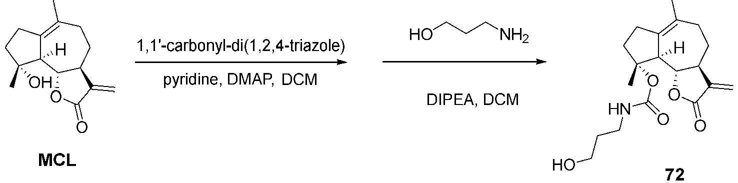

The preparation method for compound 72 is similar to that of compound 64, yielding a 62% yield. ¹H NMR (500 MHz, CDCl₃): *δ* 6.21 (d, *J* = 3.5 Hz, 1H), 5.49 (d, *J* = 3.0 Hz, 1H), 3.83 (t, *J =* 10.5 Hz, 1H), 3.68 (t, *J* = 5.5 Hz, 2H), 3.37-3.26 (m, 2H), 3.19 (d, *J* = 10.5 Hz, 1H), 2.71-2.67 (m, 1H), 2.48-2.44 (m, 1H), 2.40-2.37 (m, 1H), 2.29-2.20 (m, 3H), 2.16-2.10 (m, 2H), 1.72 (s, 3H), 1.71-1.68 (m, 2H), 1.51 (s, 3H), 1.40-1.35 (m, 1H)_{∘} ESI-MS (m/z): [M+H]⁺ = 350.19 (calcd: 350.19)_{∘}

### Preparation of Compound 73:

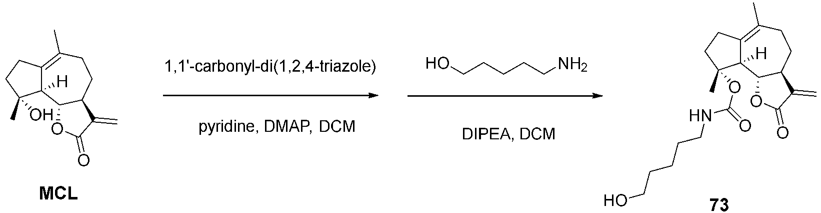

The preparation method for compound 73 is similar to that of compound 64, yielding a 59% yield. ¹H NMR (500 MHz, CDCl₃): *δ* 6.18 (d, *J* = 3.5 Hz, 1H), 5.47 (d, *J* = 3.0 Hz, 1H), 5.06 (s, 1H), 4.49 (s, 1H), 3.79 (t, *J =* 10.5 Hz, 1H), 3.62 (t, *J =* 6.5 Hz, 2H), 3.15-3.10 (m, 3H), 2.68-2.64 (m, 1H), 2.45-2.38 (m, 2H), 2.25-2.22 (m, 3H), 2.10-2.04 (m, 2H), 1.70 (s, 3H), 1.60-1.55 (m, 2H), 1.53-1.50 (m, 2H), 1.49 (s, 3H), 1.43-1.33 (m, 3H)_{∘} ESI-MS (m/z): [M+H]⁺ = 377.24 (calcd: 377.24)_{∘}

### Preparation of Compound 74:

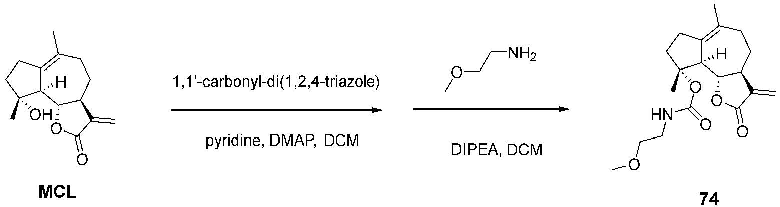

The preparation method for compound 74 is similar to that of compound 64, yielding a 75% yield. ¹H NMR (500 MHz, CDCl₃): *δ* 6.20 (d, *J* = 3.5 Hz, 1H), 5.47 (d, *J* = 3.0 Hz, 1H), 5.51 (s, 1H), 3.80 (t, *J =* 10.5 Hz, 1H), 3.48-3.46 (m, 2H), 3.37 (s, 3H), 3.36-3.29 (m, 2H), 3.14 (d, *J =* 10.0 Hz, 1H), 2.71-2.66 (m, 1H), 2.48-2.42 (m, 2H), 2.27-2.20 (m, 3H), 2.12-2.03 (m, 2H), 1.73 (s, 3H), 1.55 (s, 3H), 1.40-1.32 (m, 1H)_{∘} ESI-MS (m/z): [M+H]⁺ = 350.19 (calcd: 350.19)_{∘}

### Preparation of Compound 75:

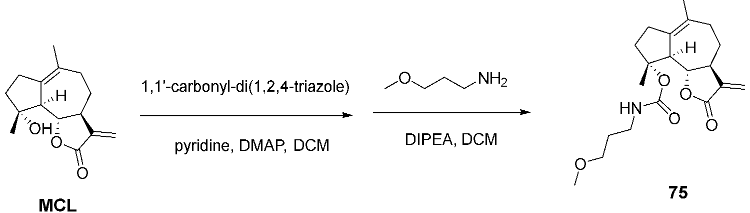

The preparation method for compound 75 is similar to that of compound 64, yielding a 74% yield.¹H NMR (500 MHz, CDCl₃) *δ* 6.20 (d, *J =* 3.3 Hz, 1H), 5.48 (d, *J =* 3.0 Hz, 1H), 5.06 (s, 1H), 3.80 (t, *J =* 10.1 Hz, 1H), 3.46 (t, *J =* 5.8 Hz, 2H), 3.35 (s, 3H), 3.29-3.21 (m, 2H), 3.14 (d, *J* = 10.1 Hz, 1H), 2.71-2.66 (m, 1H), 2.48-2.41 (m, 2H), 2.27-2.20 (m, 3H), 2.12-2.04 (m, 2H), 1.80-1.76 (m, 2H), 1.73 (s, 3H), 1.53 (s, 3H), 1.40-1.33 (m, 1H)_{∘} ESI-MS (m/z): [M+H]⁺ = 364.20 (calcd: 364.20)_{∘}

### Preparation of Compound 76:

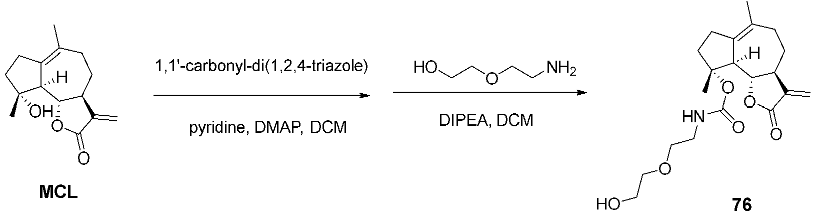

The preparation method for compound 76 is similar to that of compound 64, yielding a 62% yield. ¹H NMR (500 MHz, CDCl₃): *δ* 6.20 (d, *J =* 3.5 Hz, 1H), 5.56 (s, 1H), 5.49 (d, *J* = 3.0 Hz, 1H), 3.82 (t, *J =* 10.5 Hz, 1H), 3.62-3.58 (m, 4H), 3.36-3.34 (m, 2H), 3.12 (d, *J* = 10.5 Hz, 1H), 2.98-2.87 (m, 1H), 2.71-2.66 (m, 1H), 2.48-2.43 (m, 2H), 2.27-2.20 (m, 3H), 2.13-1.97 (m, 3H), 1.72 (s, 3H), 1.52 (s, 3H), 1.40-1.32 (m, 1H)_{∘} ESI-MS (m/z): [M+H]⁺ = 380.20 (calcd: 380.20)_{∘}

### Preparation of Compound 77:

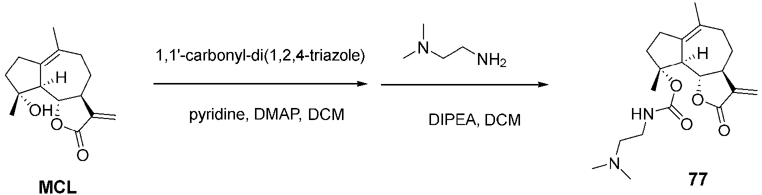

The preparation method for compound 77 is similar to that of compound 64, yielding a 61% yield. ¹H NMR (500 MHz, CDCl₃): *δ* 6.20 (d, *J* = 3.5 Hz, 1H), 5.48 (d, *J* = 3.0 Hz, 1H), 5.22 (s, 1H), 3.80 (t, *J =* 10.5 Hz, 1H), 3.31-3.22 (m, 2H), 3.16 (d, *J* = 10.0 Hz, 1H), 2.70-2.67 (m, 1H), 2.48-2.40 (m, 4H), 2.34-2.20 (m, 3H), 2.26 (s, 6H), 2.11-2.04 (m, 2H), 1.72 (s, 3H), 1.55 (s, 3H), 1.39-1.32 (m, 1H)_{∘} ESI-MS (m/z): [M+H]⁺ = 363.22 (calcd: 363.22)_{∘}

### Preparation of Compound 78:

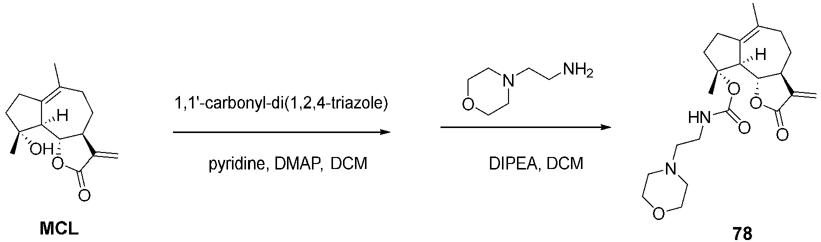

The preparation method for compound 78 is similar to that of compound 64, yielding a 71% yield. ¹H NMR (500 MHz, CDCl₃): *δ* 6.18 (d, *J* = 3.5 Hz, 1H), 5.46 (d, *J* = 3.0 Hz, 1H), 5.18 (s, 1H), 3.79 (t, *J =* 10.5 Hz, 1H), 3.72-3.70 (m, 4H), 3.25-3.23 (m, 2H), 3.17 (d, *J* = 9.5 Hz, 1H), 2.70-2.66 (m, 1H), 2.48-2.37 (m, 8H), 2.26-2.19 (m, 3H), 2.12-2.07 (m, 2H), 1.71 (s, 3H), 1.53 (s, 3H), 1.39-1.31 (m, 1H)_{∘} ESI-MS (m/z): [M+H]⁺ = 405.23 (calcd: 405.23)_{∘}

### Preparation of Compound 79:

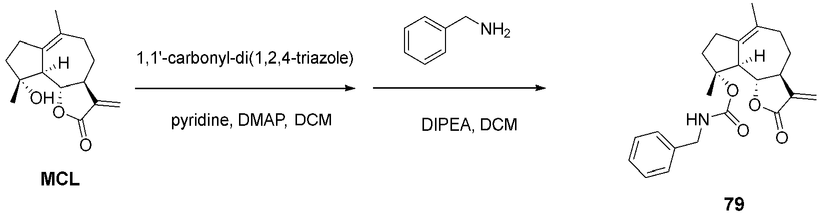

The preparation method for compound 79 is similar to that of compound 64, yielding a 69% yield. ¹H NMR (500 MHz, CDCl₃): *δ* 7.35-7.30 (m, 4H), 7.28-7.25 (m, 1H), 6.20 (d, *J =* 3.5 Hz, 1H), 5.48 (d, *J =* 3.0 Hz, 1H), 5.13 (s, 1H), 4.43-4.39 (m, 1H), 4.30-4.26 (m, 1H), 3.81 (t, *J* = 10.5 Hz, 1H), 3.18 (d, *J =* 9.5 Hz, 1H), 2.68-2.65 (m, 1H), 2.49-2.42 (m, 2H), 2.28-2.21 (m, 3H), 2.17-2.06 (m, 2H), 1.73 (s, 3H), 1.56 (s, 3H), 1.40-1.32 (m, 1H)_{∘} ESI-MS (m/z): [M+H]⁺ = 382.19 (calcd: 382.19)_{∘}

### Preparation of Compound 80:

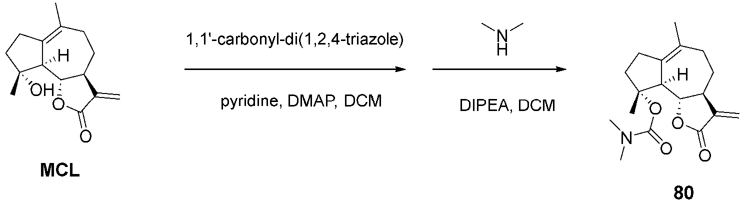

The preparation method for compound 80 is similar to that of compound 64, yielding a 70% yield. ¹H NMR (500 MHz, CDCl₃): *δ* 6.20 (d, *J* = 3.5 Hz, 1H), 5.48 (d, *J* = 3.0 Hz, 1H), 3.83 (t, *J =* 10.5 Hz, 1H), 3.07 (d, *J =* 10.0 Hz, 1H), 2.96 (s, 3H), 2.89 (s, 3H), 2.72-2.66 (m, 1H), 2.54-2.44 (m, 2H), 2.30-2.21 (m, 3H), 2.14-2.09 (m, 1H), 2.03-1.97 (m, 1H), 1.73 (s, 3H), 1.54 (s, 3H), 1.42-1.34 (m, 1H)_{∘} ESI-MS (m/z): [M+H]⁺ = 320.18 (calcd: 320.18)_{∘}

### Preparation of Compound 81:

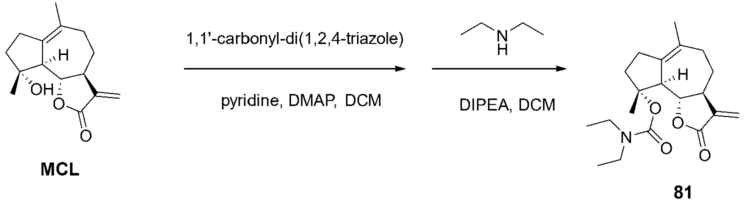

The preparation method for compound 81 is similar to that of compound 64, yielding a 69% yield. ¹H NMR (500 MHz, CDCl₃): *δ* 6.20 (d, *J* = 3.5 Hz, 1H), 5.48 (d, *J* = 3.0 Hz, 1H), 3.83 (t, *J* = 10.5 Hz, 1H), 3.41-3.35 (m, 1H), 3.30-3.22 (m, 3H), 3.06 (d, *J* = 10.0 Hz, 1H), 2.72-2.67 (m, 1H), 2.57-2.53 (m, 1H), 2.49-2.44 (m, 1H), 2.30-2.22 (m, 3H), 2.14-2.09 (m, 1H), 2.04-1.97 (m, 1H), 1.73 (s, 3H), 1.55 (s, 3H), 1.42-1.34 (m, 1H), 1.14 (s, 6H)_{∘} ESI-MS (m/z): [M+H]⁺ = 348.21 (calcd: 348.21)_{∘}

### Preparation of Compound 82:

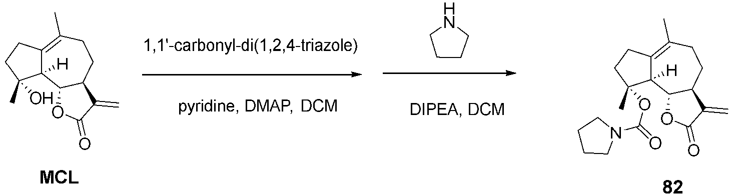

The preparation method for compound 82 is similar to that of compound 64, yielding a 71% yield. ¹H NMR (500 MHz, CDCl₃): *δ* 6.20 (d, *J* = 3.5 Hz, 1H), 5.48 (d, *J* = 3.0 Hz, 1H), 3.82 (t, *J* = 10.5 Hz, 1H), 3.53-3.49 (m, 1H), 3.37-3.30 (m, 3H), 3.10 (d, *J* = 10.0 Hz, 1H), 2.72-2.66 (m, 1H), 2.53-2.44 (m, 2H), 2.29-2.21 (m, 3H), 2.13-2.08 (m, 1H), 2.07-2.00 (m, 1H), 1.88-1.83 (m, 4H), 1.73 (s, 3H), 1.56 (s, 3H), 1.42-1.34 (m, 1H)_{∘} ESI-MS (m/z): [M+H]⁺ = 346.19 (calcd: 346.19)_{∘}

### Preparation of Compound 83:

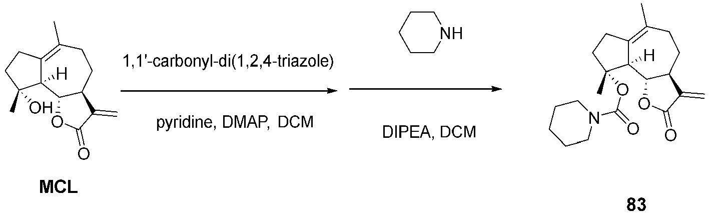

The preparation method for compound 83 is similar to that of compound 64, yielding a 68% yield. ¹H NMR (500 MHz, CDCl₃): *δ* 6.20 (d, *J* = 3.5 Hz, 1H), 5.47 (d, *J* = 3.0 Hz, 1H), 3.82 (t, *J =* 10.5 Hz, 1H), 3.51-3.42 (m, 4H), 3.10 (d, *J =* 10.0 Hz, 1H), 2.72-2.68 (m, 1H), 2.52-2.44 (m, 2H), 2.30-2.22 (m, 3H), 2.14-2.01 (m, 2H), 1.73 (s, 3H), 1.60-1.56 (m, 6H), 1.53 (s, 3H), 1.41-1.35 (m, 1H)_{∘} ESI-MS (m/z): [M+H]⁺ = 360.21 (calcd: 360.21)_{∘}

### Preparation of Compound 84:

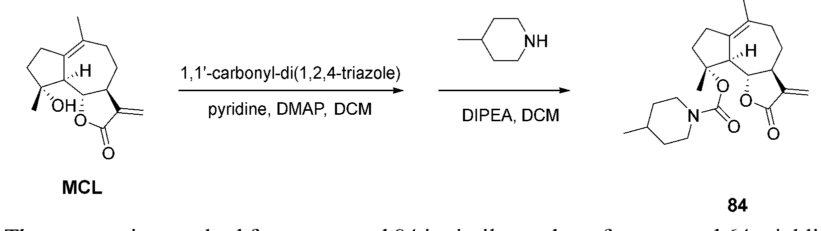

The preparation method for compound 34 is similar to that of compound 64, yielding a 67% yield. ¹H NMR (500 MHz, CDCl₃): *δ* 6.19 (d, *J* = 3.5 Hz, 1H), 5.47 (d, *J* = 3.0 Hz, 1H), 4.24-4.12 (m, 2H), 3.81 (t, *J =* 10.5 Hz, 1H), 3.08 (s, 1H), 2.83-2.67 (m, 3H), 2.51-2.43 (m, 2H), 2.29-2.20 (m, 3H), 2.12-2.09 (m, 1H), 2.05-1.99 (m, 1H), 1.72 (s, 3H), 1.65-1.60 (m, 2H), 1.55-1.48 (m, 1H), 1.52 (s, 3H), 1.41-1.33 (m, 1H), 1.19-1.04 (m, 2H), 0.94 (d, *J =* 6.5 Hz, 3H)_{∘} ESI-MS (m/z): [M+H]⁺ = 374.23 (calcd: 374.23)_{∘}

### Preparation of Compound 85:

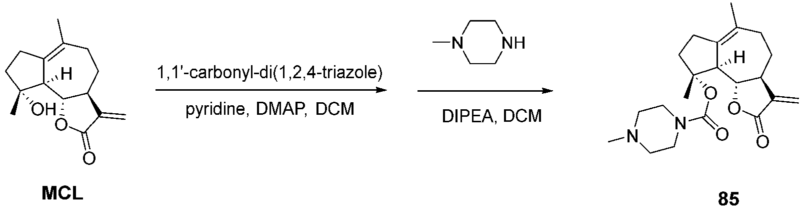

The preparation method for compound 85 is similar to that of compound 64, yielding a 52% yield. ¹H NMR (500 MHz, CDCl₃): *δ* 6.20 (d, *J* = 3.5 Hz, 1H), 5.48 (d, *J* = 3.0 Hz, 1H), 3.83 (t, *J =* 10.5 Hz, 1H), 3.57-3.40 (m, 4H), 3.08 (d, *J =* 10.0 Hz, 1H), 2.71-2.67 (m, 1H), 2.53-2.46 (m, 2H), 2.43-2.38 (m, 3H), 2.32 (s, 3H), 2.30-2.22 (m, 4H), 2.13-2.10 (m, 1H), 2.04-2.02 (m, 1H), 1.73 (s, 3H), 1.53 (s, 3H), 1.42-1.37 (m, 1H)_{∘} ESI-MS (m/z): [M+H]⁺ = 375.22 (calcd: 375.22)_{∘}

### Preparation of Compound 86:

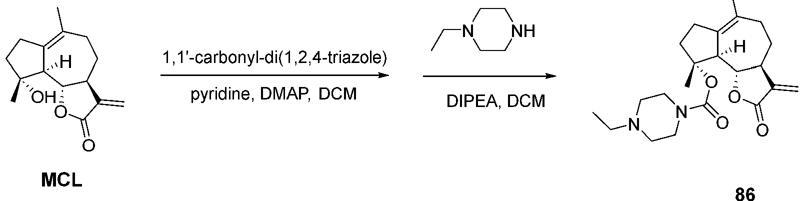

The preparation method for compound 86 is similar to that of compound 64, yielding a 55% yield. ¹H NMR (500 MHz, CDCl₃): *δ* 6.20 (d, *J* = 3.5 Hz, 1H), 5.48 (d, *J* = 3.0 Hz, 1H), 3.82 (t, *J =* 10.5 Hz, 1H), 3.71-3.41 (m, 4H), 3.08 (d, *J =* 10.0 Hz, 1H), 2.71-2.66 (m, 1H), 2.52-2.43 (m, 8H), 2.29-2.22 (m, 3H), 2.13-2.10 (m, 1H), 2.07-2.00 (m, 1H), 1.72 (s, 3H), 1.52 (s, 3H), 1.42-1.34 (m, 1H), 1.11 (t, *J =* 7.0 Hz, 3H)_{∘} ESI-MS (m/z): [M+H]⁺ = 389.24 (calcd: 389.24)_{∘}

### Preparation of Compound 87:

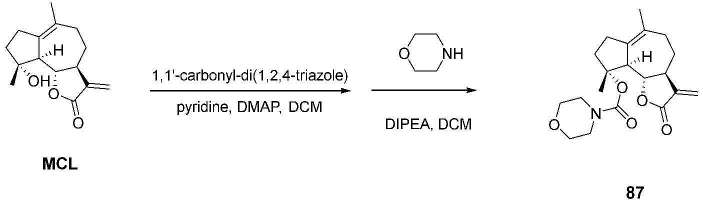

The preparation method for compound 87 is similar to that of compound 64, yielding a 71% yield. ¹H NMR (500 MHz, CDCl₃): *δ* 6.18 (d, *J* = 3.5 Hz, 1H), 5.47 (d, *J =* 3.0 Hz, 1H), 3.81 (t, *J* = 10.5 Hz, 1H), 3.68-3.62 (m, 5H), 3.49-3.41 (m, 3H), 3.06 (d, *J* = 10.0 Hz, 1H), 2.70-2.64 (m, 1H), 2.51-2.44 (m, 2H), 2.28-2.21 (m, 3H), 2.13-2.00 (m, 2H), 1.72 (s, 3H), 1.52 (s, 3H), 1.40-1.33 (m, 1H)_{∘} ESI-MS (m/z): [M+H]⁺ = 362.19 (calcd: 362.19)_{∘}

### Preparation of Compound 88:

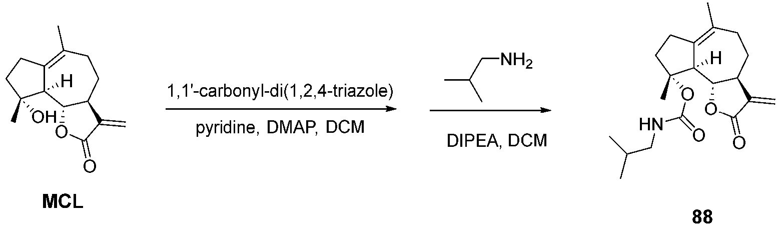

The preparation method for compound 88 is similar to that of compound 64, yielding a 75% yield. ¹H NMR (500 MHz, CDCl₃) δ 6.17 (d, *J =* 3.3 Hz, 1H), 5.46-5.45 (m, 1H), 4.82 (s, 1H), 3.78 (t, *J =* 10.1 Hz, 1H), 3.13 (d, *J* = 9.9 Hz, 1H), 3.02-2.97 (m, 1H), 2.90-2.85 (m, 1H), 2.71-2.62 (m, 1H), 2.48-2.35 (m, 2H), 2.26-2.17 (m, 3H), 2.12-2.02 (m, 2H), 1.77-1.71 (m, 1H), 1.70 (s, 3H), 1.51 (s, 3H), 1.38-1.30 (m, 1H), 0.89 (d, *J =* 6.7 Hz, 6H)_{∘} ESI-MS (m/z): [M+H]⁺ = 348.21 (calcd: 348.21)_{∘}

### Preparation of Compound 89:

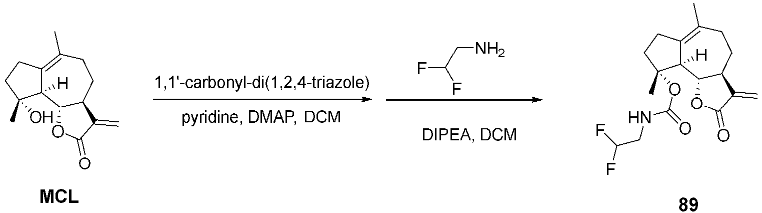

The preparation method for compound 89 is similar to that of compound 64, yielding a 78% yield. ¹H NMR (500 MHz, CDCl₃) δ 6.20 (d, *J =* 3.3 Hz, 1H), 5.83 (t, *J =* 56.1 Hz, 1H), 5.48 (d, *J =* 2.9 Hz, 1H), 5.16 (s, 1H), 3.80 (t, *J =* 10.1 Hz, 1H), 3.62-3.38 (m, 2H), 3.11 (d, *J =* 10.5 Hz, 1H), 2.73-2.62 (m, 1H), 2.48-2.41 (m, 2H), 2.27-2.20 (m, 3H), 2.12-1.99 (m, 2H), 1.72 (s, 3H), 1.53 (s, 3H), 1.38-1.31 (m, 1H)_{∘} ESI-MS (m/z): [M+H]⁺ = 356.16 (calcd: 356.16)_{∘}

### Preparation of Compound 90:

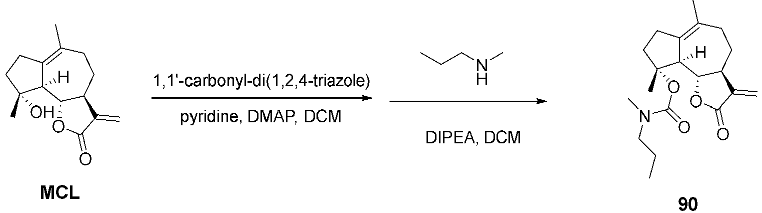

The preparation method for compound 90 is similar to that of compound 64, yielding a 67% yield. ¹H NMR (500 MHz, CDCl₃) δ 6.17 (d, *J* = 3.2 Hz, 1H), 5.46 (d, *J* = 3.0 Hz, 1H), 3.80 (t, *J =* 10.0 Hz, 1H), 3.37-3.12 (m, 2H), 3.10-3.00 (m, 1H), 2.88 (d, *J =* 28.6 Hz, 3H), 2.72-2.62 (m, 1H), 2.54-2.41 (m, 2H), 2.29-2.18 (m, 3H), 2.11-2.07 (m, 1H), 2.02-1.93 (m, 1H), 1.71 (s, 3H), 1.63-1.53 (m, 2H), 1.51 (s, 3H), 1.40-1.32 (m, 1H), 0.88 (t, *J =* 7.4 Hz, 3H). ESI-MS (m/z): [M+H]⁺ = 348.21 (calcd: 348.21).

### Preparation of Compound 91:

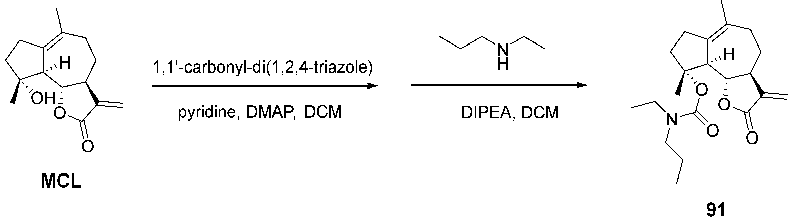

The preparation method for compound 91 is similar to that of compound 64, yielding a 68% yield. ¹H NMR (500 MHz, CDCl₃) δ 6.19 (d, *J* = 3.3 Hz, 1H), 5.46 (d, *J* = 3.0 Hz, 1H), 3.81 (t, *J =* 10.1 Hz, 1H), 3.44-3.00 (m, 5H), 2.69-2.66 (m, 1H), 2.59-2.42 (m, 2H), 2.29-2.20 (m, 3H), 2.12-2.08 (m, 1H), 2.03-1.97 (m, 1H), 1.72 (s, 3H), 1.61-1.54 (m, 2H), 1.53 (s, 3H), 1.42-1.33 (m, 1H), 1.19-1.07 (m, 3H), 0.89-0.87 (m, 3H)_{∘} ESI-MS (m/z): [M+H]⁺ = 362.23 (calcd: 362.23)_{∘}

### Preparation of Compound 92:

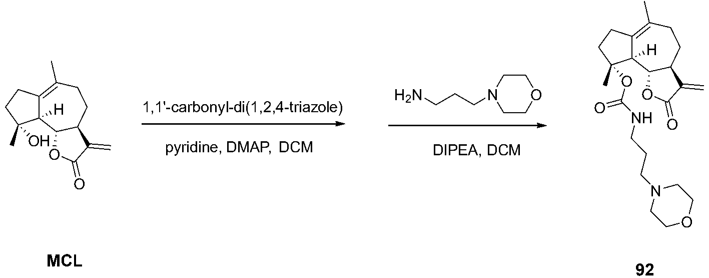

The preparation method for compound 92 is similar to that of compound 64, yielding a 55% yield. ¹H NMR (500 MHz, CDCl₃) δ 6.20 (d, *J =* 3.3 Hz, 1H), 5.72 (s, 1H), 5.48 (d, *J* = 3.1 Hz, 1H), 3.81 (t, *J =* 10.1 Hz, 1H), 3.73 (t, *J* = 4.7 Hz, 4H), 3.30-3.12 (m, 3H), 2.72-2.65 (m, 1H), 2.47-2.39 (m, 8H), 2.29-2.20 (m, 3H), 2.14-2.05 (m, 2H), 1.72 (s, 3H), 1.69-1.66 (m, 2H), 1.53 (s, 3H), 1.40-1.34 (m, 1H)_{∘} ESI-MS (m/z): [M+H]⁺ = 419.25 (calcd: 419.25)_{∘}

### Preparation of Compound 93:

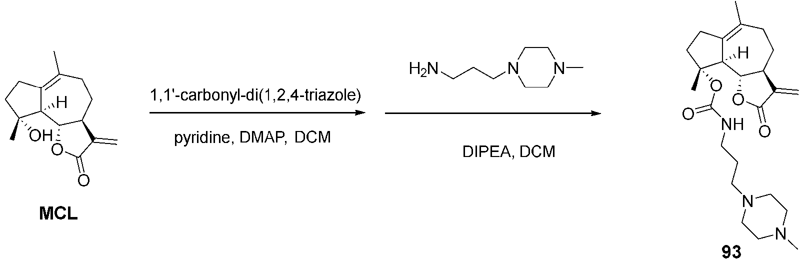

The preparation method for compound 93 is similar to that of compound 64, yielding a 47% yield. ¹H NMR (500 MHz, CDCl₃) δ 6.17 (d, *J* = 3.3 Hz, 1H), 5.83 (s, 1H), 5.46 (d, *J* = 3.0 Hz, 1H), 3.78 (t, *J =* 10.1 Hz, 1H), 3.26-3.11 (m, 3H), 2.65 (t, *J* = 9.3 Hz, 1H), 2.56-2.36 (m, 11H), 2.26 (s, 7H), 2.12-2.04 (m, 2H), 1.71 (s, 3H), 1.69-1.63 (m, 2H), 1.51 (s, 3H), 1.39-1.32 (m, 1H)_{∘} ESI-MS (m/z): [M+H]⁺ = 432.28 (calcd: 432.28)_{∘}

### Preparation of Compound 94:

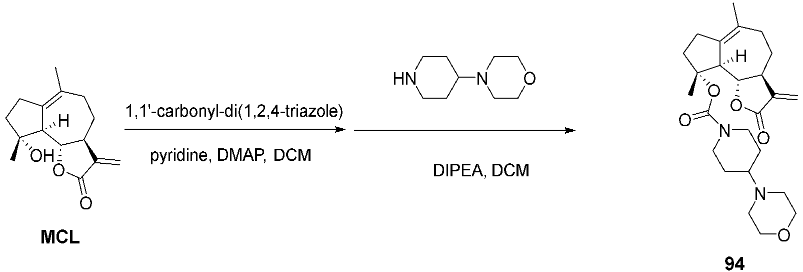

The preparation method for compound 94 is similar to that of compound 64, yielding a 62% yield.¹H NMR (500 MHz, CDCl₃) δ 6.18 (d, *J* = 3.2 Hz, 1H), 5.46 (d, *J* = 3.0 Hz, 1H), 4.28-4.14 (m, 2H), 3.80 (t, *J =* 10.1 Hz, 1H), 3.75-3.68 (m, 4H), 3.06 (s, 1H), 2.92-2.63 (m, 4H), 2.49-2.42 (m, 2H), 2.37-2.18 (m, 5H), 2.14-2.07 (m, 2H), 2.01 (d, *J* = 10.2 Hz, 1H), 1.84-1.82 (m, 2H), 1.71 (s, 3H), 1.49 (s, 3H), 1.46-1.32 (m, 3H)_{∘} ESI-MS (m/z): [M+H]⁺ = 445.26 (calcd: 445.26)_{∘}

### Preparation of Compound 95:

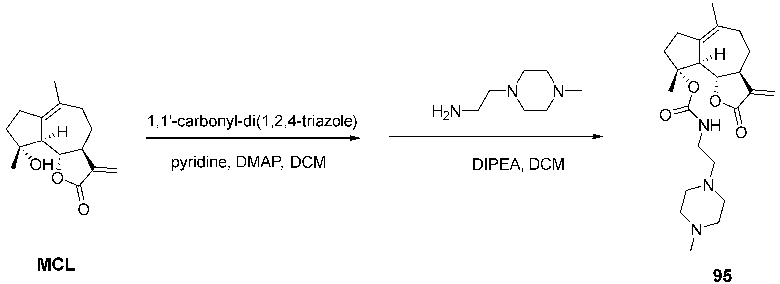

The preparation method for compound 95 is similar to that of compound 64, yielding a 44% yield. ¹H NMR (500 MHz, CDCl₃) δ 6.17 (d, *J =* 3.3 Hz, 1H), 5.45 (d, *J* = 3.0 Hz, 1H), 5.22 (s, 1H), 3.78 (t, *J =* 10.1 Hz, 1H), 3.31-3.19 (m, 2H), 3.15 (d, *J* = 9.5 Hz, 1H), 2.83 (s, 1H), 2.67 (t, *J =* 9.4 Hz, 1H), 2.48-2.37 (m, 11H), 2.29 (s, 3H), 2.25-2.18 (m, 3H), 2.11-2.04 (m, 2H), 1.70 (s, 3H), 1.52 (s, 3H), 1.38-1.31 (m, 1H)_{∘} ESI-MS (m/z): [M+H]⁺ = 418.26 (calcd: 418.26)_{∘}

### Preparation of Compound 96:

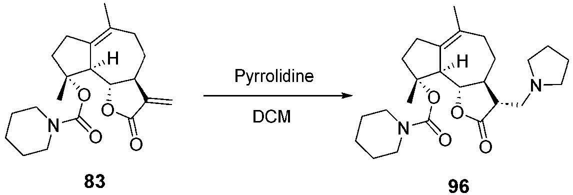

In a 5 mL reaction flask, dissolve compound 83 (100 mg, 0.28 mmol) in 1 mL of dichloromethane. Add tetrahydropyrole (197 mg, 2.80 mmol) and stir for 3 hours. Diluted with ethyl acetate (10 mL), then washed successively with water (10 mL × 3), saturated sodium bicarbonate solution (10 mL × 3), and saturated saline solution (10 mL × 3). After drying over anhydrous sodium sulfate, the mixture was concentrated under reduced pressure. Separation by silica gel column chromatography yielded compound 96 in 66% yield. ¹H NMR (500 MHz, CDCl₃) δ 3.80 (t, *J* = 10.2 Hz, 1H), 3.57-3.30 (m, 4H), 3.03-2.97 (m, 1H), 2.91-2.81 (m, 2H), 2.59-2.53 (m, 2H), 2.52-2.37 (m, 5H), 2.28-2.14 (m, 4H), 2.14-1.97 (m, 2H), 1.79-1.69 (m, 8H), 1.61-1.53 (m, 5H), 1.52 (s, 4H)_{∘} ESI-MS (m/z): [M+H]⁺ = 431.28 (calcd: 431.28)_{∘}

### Preparation of Compound 97:

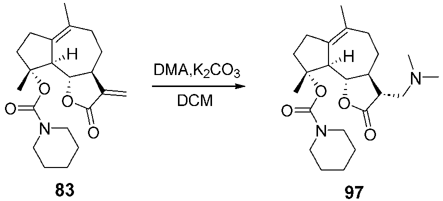

Using compound 83 and dimethylamine hydrochloride as starting materials, the preparation method is the same as that for compound 96. The yield of compound 97 is 85%.¹H NMR (500 MHz, CDCl₃) δ 3.82 (t, *J =* 10.1 Hz, 1H), 3.58-3.30 (m, 4H), 3.05-2.96 (m, 1H), 2.74 (dd, *J =* 12.9, 4.9 Hz, 1H), 2.60 (dd, *J* = 12.9, 6.6 Hz, 1H), 2.53-2.35 (m, 3H), 2.26 (s, 6H), 2.28-2.14 (m, 4H), 2.09-1.97 (m, 2H), 1.74-1.65 (m, 6H), 1.59-1.54 (m, 6H), 1.39-1.27 (m, 1H)_{∘} ESI-MS (m/z): [M+H]⁺ = 405.27 (calcd: 405.27)_{∘}

### Preparation of Compound 98:

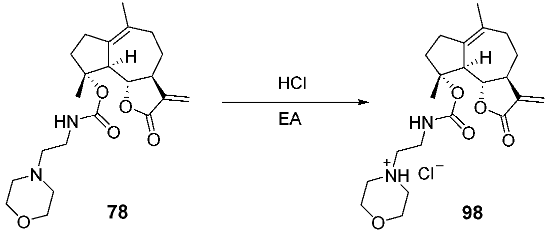

Compound 78 (404 mg, 1 mmol) was dissolved in dichloromethane (2 mL) and stirred at room temperature for 2 h. A hydrochloric acid solution was then added dropwise until the pH reached 4-5. The mixture was filtered, and the resulting solid was washed with dichloromethane. The white solid obtained was the hydrochloride salt of compound 78 (compound 98), yielding 86%.¹H NMR (500 MHz, Methanol-*d*₄) δ 6.15 (d, *J* = 6.9 Hz, 1H), 5.62 (d, *J =* 14.3 Hz, 1H), 4.14-4.08 (m, 2H), 4.05-3.89 (m, 1H), 3.88-3.76 (m, 2H), 3.65-3.42 (m, 4H), 3.28-3.16 (m, 3H), 2.89-2.71 (m, 1H), 2.57-2.46 (m, 1H), 2.42-2.28 (m, 4H), 2.25-2.15 (m, 1H), 2.09-2.00 (m, 3H), 1.76 (s, 3H), 1.57 (s, 3H), 1.44-1.37 (m, 1H)_{∘} ESI-MS (m/z): [M+H]⁺ = 405.23 (calcd: 405.23)_{∘}

### Preparation of Compound 99:

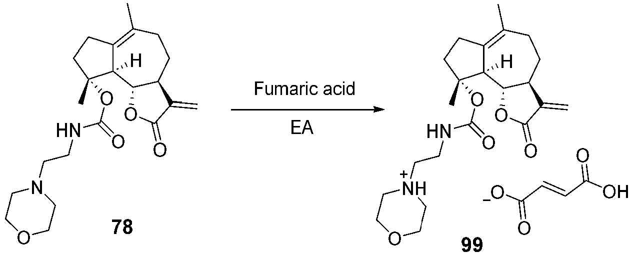

Fumaric acid was substituted for hydrochloric acid to prepare compound 99, following the method used for synthesizing the hydrochloride salt of compound 78. The yield was 71%.¹H NMR (500 MHz, Methanol-*d*₄) δ 6.74 (s, 2H), 6.14 (d, *J =* 10.2 Hz, 1H), 5.61 (d, *J =* 10.5 Hz, 1H), 4.01-3.78 (m, 5H), 3.39-3.36 (m, 2H), 3.19 (d, *J* = 11.5 Hz, 1H), 3.11-3.05 (m, 2H), 2.99-2.85 (m, 4H), 2.82-2.72 (m, 1H), 2.52-2.47 (m, 1H), 2.43-2.24 (m, 4H), 2.22-2.13 (m, 1H), 2.06-2.00 (m, 1H), 1.75 (s, 3H), 1.56 (s, 3H), 1.43-1.35 (m, 1H)_{∘} ESI-MS (m/z): [M+H]⁺ = 405.23 (calcd: 405.23)_{∘}

### Preparation of Compound 100:

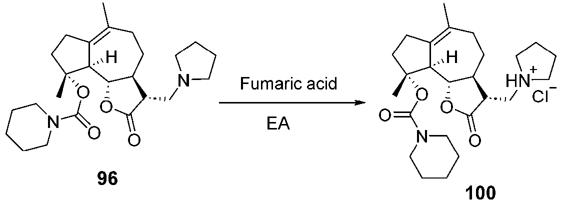

Compound 100 can be prepared from compound 96 as the starting material, following the method used for the preparation of compound 78 hydrochloride, yielding a 91% yield. ¹H NMR (500 MHz, Methanol-*d*₄) δ 4.13 (t, *J* = 10.0 Hz, 1H), 3.81-3.72 (m, 2H), 3.68 (s, 1H), 3.64-3.56 (m, 1H), 3.51-3.39 (m, 3H), 3.26-3.19 (m, 2H), 3.09 (d, *J* = 8.7 Hz, 2H), 2.54-2.40 (m, 2H), 2.34-2.06 (m, 8H), 2.04-1.94 (m, 2H), 1.75 (s, 3H), 1.66-1.60 (m, 2H), 1.55-1.51 (m, 7H), 1.49-1.43 (m, 1H), 1.42-1.26 (m, 1H)_{∘} ESI-MS (m/z): [M+H]⁺ = 431.28 (calcd: 431.28)_{∘}

### Preparation of Compound 101:

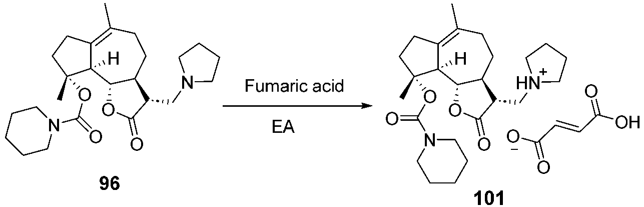

Fumaric acid was substituted for hydrochloric acid. Following the preparation method for compound 78 hydrochloride, compound 101 was synthesized with a yield of 75%. ¹H NMR (500 MHz, Methanol-*d*₄) δ 6.72 (s, 2H), 4.17-4.07 (m, 2H), 3.60-3.40 (m, 9H), 3.11-3.01 (m, 2H), 2.52-2.41 (m, 2H), 2.35-2.21 (m, 3H), 2.18-2.12 (m, 5H), 2.02-1.93 (m, 2H), 1.74 (s, 3H), 1.66-1.61 (m, 2H), 1.55-1.52 (m, 7H), 1.49-1.39 (m, 1H)_{∘} ESI-MS (m/z): [M+H]⁺ = 431.28 (calcd: 431.28)_{∘}

### Preparation of Compound 102:

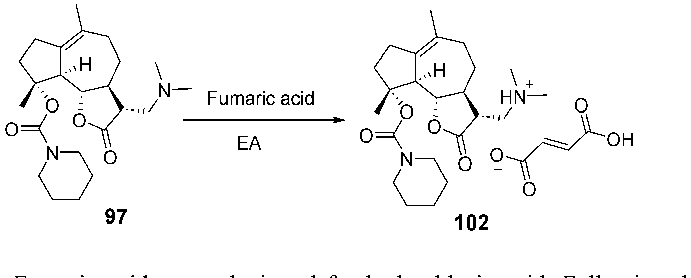

Fumaric acid was substituted for hydrochloric acid. Following the preparation method for compound 78 hydrochloride, compound 102 was synthesized with a yield of 71%.¹H NMR (500 MHz, Methanol-*d*₄) δ 6.71 (s, 2H), 4.14-4.09 (m, 1H), 3.46-3.40 (m, 3H), 3.31-3.27 (m, 1H), 3.12-3.01 (m, 2H), 2.90 (s, 6H), 2.52-2.42 (m, 2H), 2.34-2.22 (m, 3H), 2.14 (q, *J =* 10.9, 10.1 Hz, 1H), 2.01-1.93 (m, 2H), 1.75 (s, 3H), 1.66-1.61 (m, 2H), 1.55-1.51 (m, 7H), 1.48-1.40 (m, 1H), 1.40-1.29 (m, 2H)_{∘} ESI-MS (m/z): [M+H]⁺ = 405.27 (calcd: 405.27).

### Example 2

Water Solubility Testing of Compounds:
Precisely weigh 20 µg each of compounds 1-19, 78, 83, Arg, and MCL, and 10 mg each of compounds 20-42 and 98-102. Add them to 1 mL of deionized water, and sonicate to achieve complete dissolution. After preparing the saturated solution and filtering it, perform HPLC injection analysis. The injection volumes are 1 µL, 3 µL, 5 µL, 10 µL, 15 µL, and 20 µL in sequence, and plot standard curves for the corresponding compounds.

Prepare an unsaturated solution of the aforementioned compound, dissolve it ultrasonically for 4 hours, then place it in a 37 °C water bath for 1 hour. Centrifuge the resulting unsaturated solution, transfer 30 µL of the supernatant, dilute it with 200 µL of deionized water, filter it, and analyze the sample via HPLC. Substitute the relevant data into the previously determined standard curve to obtain the solubility of the tested compound.

**Table 1. Solubility of Compounds 1-42, 78, 83, 98-102 in Water**

| compound | Water-solubility mg/mL | Salt-form compounds | Water-solubility mg/mL |
|---|---|---|---|
| compound 1 | 1.140 | compound 20 | 320.7 |
| compound 2 | 0.862 | compound 21 | 310.4 |
| compound 3 | 1.305 | compound 22 | 400.8 |
| compound 4 | 0.822 | compound 23 | 362.3 |
| compound 5 | 0.730 | compound 24 | 300.5 |
| compound 6 | 0.668 | compound 25 | 342.3 |
| compound 7 | 1.488 | compound 26 | 288.0 |
| compound 8 | 1.520 | compound 27 | 211.8 |
| compound 9 | 0.840 | compound 28 | 366.2 |
| compound 10 | 0.715 | compound 29 | 289.5 |
| compound 11 | 0.682 | compound 30 | 228.4 |
| compound 12 | 0.802 | compound 31 | 254.6 |
| compound 13 | 0.419 | compound 32 | 100.8 |
| compound 14 | 0.460 | compound 33 | 145.5 |
| compound 15 | 0.310 | compound 34 | 128.6 |
| compound 16 | 0.285 | compound 35 | 212.6 |
| compound 17 | 0.942 | compound 36 | 234.6 |
| compound 18 | 0.584 | compound 37 | 357.5 |
| compound 19 | 0.623 | compound 38 | 250.9 |
| compound 78 | 0.200 | compound 39 | 228.2 |
| compound 83 | 0.152 | compound 40 | 198.8 |
| Arglabin (**Arg**) | 0.008 | compound 41 | 252.5 |
| Micheliolide(**MCL**) | 0.062 | compound 42 | 226.6 |
| | | compound 98 | 202.5 |
| | | compound 99 | 220.8 |
| | | compound 100 | 144.8 |
| | | compound 101 | 108.0 |
| | | compound 102 | 132.5 |

As shown in Table 1, compared with Arglabin and Micheliolide, the water solubility of compounds 1-19, 78, 83, as well as the salt-form compounds 20-42 and 98-102 is increased, and the water solubility of the salt-form compounds is enhanced by at least 100 times or more.

### Example 3

Pharmaceutically acceptable salts of guaiane-type sesquiterpene lactone derivatives were selected and evaluated for their conversion rates to Compound 1 and Compound 83 in plasma and HEPES buffer, with results shown in Tables 2-1, 2-2, 3-1, and 3-2.

Experimental Method: Preparation of HEPES 7.4 Solution: Dissolve 1.6 g NaCl, 0.074 g KCl, 0.027 g Na₂HPO₄, 0.2 g glucose, and 1 g 4- (2-Hydroxyethyl)piperazine-1-ethanesulfonic acid (HEPES) solution in 90 mL distilled water. Adjust the pH to 7.4 using 0.5 M NaOH, then dilute to a final volume 100 mL with distilled water.

Plasma Preparation: Collect mouse plasma into an EP tube pre-loaded with sodium heparin, centrifuge at 8000 rpm for 10 minutes at 4°C, and then collect the supernatant.

Sample Analysis: Dissolve 0.6 mg of the compound in 250 µl of deionized water separately. Add 250 µl of mouse serum or HEPES 7.4 solution to the sample, incubate at a constant temperature of 37 °C, and collect samples at different time points. Pipette 20 µl of the sample to an EP tube, add 60 µl of methanol, vortex to mix thoroughly, and centrifuge at 12,000 rpm at 4 °C for 10 min. At 1 h, 2 h, 4 h, 8 h, and 12 h respectively, collect the supernatant for HPLC analysis. Inject 10 µL of sample and record the corresponding peak areas. Chromatographic conditions: Hanbang C18 column (4.6 × 250 mm, 5 µm); mobile phase: acetonitrile: 10 mmol/mL ammonium formate solution = 60:40; flow rate 1.0 mL/min; detection wavelength 210 nm; column temperature 30 °C.

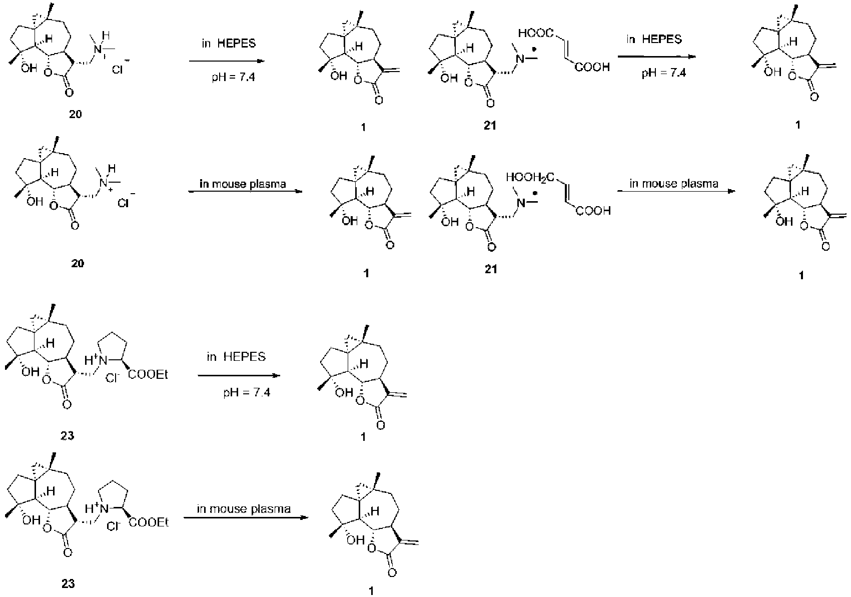

**Table 2-1. Conversion Rate of Compounds 20-42 to Compound 1 at Different Time Points in HEPES Buffer Solution**

| compound | Release rate | | | | |
|---|---|---|---|---|---|
| | 1 h | 2h | 4h | 8 h | 12 h |
| compound 20 | 6.2% | 13.8% | 21.7% | 44.3% | 59.1% |
| compound 21 | 3.3% | 10.2% | 18.1% | 38.9% | 51.9% |
| compound 22 | 6.4% | 14.8% | 26.6% | 41.3% | 53.7% |
| compound 23 | 6.2% | 14.3% | 26.8% | 41.1% | 52.8% |
| compound 24 | 6.8% | 15.1% | 26.7% | 42.8% | 54.0% |
| compound 25 | 6.8% | 14.1% | 26.1% | 42.8% | 51.8% |
| compound 26 | 6.8% | 16.1% | 27.3% | 43.5% | 55.6% |
| compound 27 | 6.9% | 15.4% | 27.4% | 42.2% | 54.8% |
| compound 28 | 6.8% | 14.5% | 24.4% | 40.4% | 50.0% |
| compound 29 | 6.8% | 14.2% | 24.6% | 40.2% | 49.8% |
| compound 30 | 6.6% | 14.6% | 23.8% | 40.8% | 49.8% |
| compound 31 | 6.7% | 14.0% | 23.0% | 41.2% | 49.5% |
| compound 32 | 6.9% | 15.4% | 26.2% | 42.2% | 51.2% |
| compound 33 | 6.9% | 15.8% | 26.0% | 41.8% | 51.1% |
| compound 34 | 7.0% | 16.2% | 27.6% | 43.6% | 54.4% |
| compound 35 | 6.8% | 14.6% | 26.4% | 43.8% | 52.5% |
| compound 36 | 6.9% | 14.8% | 26.0% | 43.6% | 52.4% |
| compound 37 | 7.2% | 16.8% | 28.3% | 44.1% | 65.8% |
| compound 38 | 6.9% | 14.8% | 25.4% | 41.4% | 50.7% |
| compound 39 | 6.6% | 15.0% | 26.8% | 42.7% | 54.3% |
| compound 40 | 6.8% | 14.6% | 26.2% | 44.0% | 50.2% |
| compound 41 | 7.0% | 16.2% | 27.3% | 43.4% | 53.2% |
| compound 42 | 7.0% | 15.0% | 25.2% | 42.8% | 52.3% |

**Table 2-2. Conversion Rate of Compounds 100-102 to Compound 83 at Different Time Points in HEPES Buffer Solution**

| compound | Release rate | | | | |
|---|---|---|---|---|---|
| | 1 h | 2 h | 4 h | 8 h | 12 h |
| compound 100 | 6.5% | 14.0% | 26.4% | 45.2% | 51.4% |
| compound 101 | 7.0% | 16.0% | 28.2% | 46.4% | 53.6% |
| compound 102 | 6.8% | 14.6% | 24.8% | 43.0% | 51.8% |

**Table 3-1. Conversion Rate of Compounds 20-42 to Compound 1 at Different Time Points in Mouse Plasma**

| compound | Release rate | | | | |
|---|---|---|---|---|---|
| | 1 h | 2 h | 4 h | 8 h | 12 h |
| compound 20 | 7.3% | 12.9% | 28.6% | 46.8% | 62.9% |
| compound 21 | 5.4% | 10.7% | 17.9% | 39.3% | 55.4% |
| compound 22 | 7.4% | 16.8% | 27.6% | 46.3% | 57.7% |
| compound 23 | 7.2% | 14.3% | 26.8% | 45.1% | 52.8% |
| compound 24 | 7.7% | 17.1% | 27.7% | 47.8% | 57.9% |
| compound 25 | 6.8% | 14.1% | 25.7% | 43.8% | 51.8% |
| compound 26 | 7.8% | 17.1% | 29.3% | 48.5% | 65.6% |
| compound 27 | 7.9% | 17.4% | 28.4% | 48.2% | 59.1% |
| compound 28 | 6.8% | 14.5% | 24.4% | 40.4% | 49.1% |
| compound 29 | 6.8% | 14.2% | 24.6% | 40.2% | 49.0% |
| compound 30 | 6.6% | 13.6% | 23.8% | 40.8% | 48.8% |
| compound 31 | 6.5% | 13.9% | 24.4% | 40.2% | 48.5% |
| compound 32 | 6.3% | 14.4% | 25.2% | 43.2% | 51.4% |
| compound 33 | 6.9% | 14.8% | 26.0% | 42.8% | 50.7% |
| compound 34 | 7.8% | 17.2% | 28.6% | 47.6% | 58.8% |
| compound 35 | 6.8% | 13.6% | 26.4% | 44.8% | 52.5% |
| compound 36 | 6.6% | 14.8% | 26.0% | 43.6% | 52.1% |
| compound 37 | 8.2% | 17.8% | 29.3% | 48.1% | 67.8% |
| compound 38 | 6.1% | 14.5% | 25.4% | 41.4% | 49.7% |
| compound 39 | 6.6% | 14.0% | 26.2% | 43.6% | 52.8% |
| compound 40 | 6.7% | 14.2% | 26.8% | 44.0% | 52.6% |
| compound 41 | 7.8% | 17.2% | 28.0% | 46.1% | 54.8% |
| compound 42 | 6.3% | 14.2% | 26.2% | 41.0% | 47.2% |

**Table 3-2. Conversion Rates of Compounds 100-102 to Compound 83 at Different Time Points in Mouse Plasma**

| compound | Release rate | | | | |
|---|---|---|---|---|---|
| | 1 h | 2 h | 4 h | 8 h | 12 h |
| compound 100 | 6.6% | 13.8% | 25.6% | 43.2% | 53.0% |
| compound 101 | 7.6% | 16.6% | 27.4% | 46.0% | 54.2% |
| compound 102 | 6.6% | 14.0% | 26.0% | 42.4% | 47.8% |

Experimental Results: As shown in Table 2-1, in HEPES buffer solution, salt-form compounds 20-42 can be converted into the parent compound 1. Moreover, as the incubation time of the compounds increases, the proportion of salt-form compounds 20-42 converted into the parent compound 1 gradually rises. Simultaneously, as shown in Table 3-1, salt-form compound 20-42 can also convert to the parent compound 1 in mouse plasma. Furthermore, the proportion of compound 20-42 converting to the parent compound 1 gradually increased with extended incubation time. The above experiments collectively demonstrate that compound 20-42 functions as a prodrug, converting to the parent compound 1 in both HEPES buffer solution and mouse plasma.

Similarly, as shown in Tables 2-2 and 3-2, prodrug compounds 100, 101, and 102 can also be converted into the corresponding parent drug compound 83 in plasma and HEPES.

### Example 4

Activity testing of Compound 1 and its prodrugs 20 (dimethyl hydrochloride salt) and 21 (fumarate salt) against mouse sepsis:
Experimental Reagents: The drug of the present invention, prepared according to Example 1; Lipopolysaccharide (LPS), Sigma; Sodium carboxymethylcellulose (CMC-Na), Shantou Xilong Chemical Factory, Guangdong; RNA Isolater Total RNA Extraction Reagent, Hiscript Q RT Supermix for qPCR, Nanjing Vazyme Biotech Co., Ltd.; AceQ qPCR SYBR Green Master Mix, Shanghai Yeasen Biotechnology Co., Ltd.
Experimental Animals: Male C57BL/6 mice, 6-8 weeks old, weighing 18-20 g, supplied by Shanghai Slac Animal Breeding Center, Production License No.: SCXK(Su)2019-002. Animals had free access to food and water, were fed standard pellet diet, maintained at room temperature 22 ± 2 °C and humidity 45 ± 10%. They were acclimated for 7 days before experimentation.
Experimental Methods: Establishment of Mouse Sepsis Model and Group-Specific Drug Administration
Mice were randomly assigned to groups, with one group arbitrarily designated as the normal group and the remaining groups serving as model group, Compound 1 (0.1, 1 mg/kg), Hydrochloride 20 (1 mg/kg), and Fumarate 21 (0.1, 1 mg/kg), each containing 6 mice. Except for the normal group receiving intraperitoneal (i.p.) injections of PBS, all other mice were administered LPS (10 mg/kg) via i.p. injection to establish a mouse sepsis model. One hour prior to model induction, Compound 1 (0.1, 1 mg/kg), Hydrochloride 20 (1 mg/kg), and Fumarate 21 (0.1, 1 mg/kg) were administered via oral gavage for 5 consecutive days. The normal and model groups received equal volumes of solvent (0.5% CMC-Na) via oral gavage.
Specimen Collection: One hour after the final drug administration, mice were euthanized by cervical dislocation. The abdominal cavity was opened, and the lungs, liver, and kidneys were removed. These organs were rinsed twice with pre-chilled PBS. Tissue samples were excised from the mouse lungs, left hepatic lobe, and kidneys. Specimens were fixed in 4% paraformaldehyde for 24-48 hours. Remaining tissues were stored at -70 °C for future use.

### Q-PCR Analysis

### (1) Total RNA Extraction

Accurately weigh 20 mg of kidney, lung, and liver tissue. Rinse with pre-chilled PBS solution, cut into small pieces using ophthalmic scissors, and place into a glass homogenizer. Add 1 mL of Trizol reagent and grind to prepare tissue homogenate. Transfer the homogenate to a RNAse-free 1.5 mL EP tube and lyse on ice for 10 min. Add 200 µL chloroform, vortex vigorously, and let stand on ice for 3-5 min. Centrifuge at 12,000 rpm and 4 °C for 15 min. Carefully transfer the supernatant to a new RNAse-free 1.5 mL EP tube. Add an equal volume of pre-chilled isopropanol, incubate on ice for 30 min, then centrifuge at 12,000 rpm and 4 °C for 10 min. Discard the supernatant. Wash the pellet with 75% ethanol-DEPC solution, then dry it upside down on filter paper to obtain total RNA. Subsequently, dissolve the pellet in 10 µL DEPC-treated water. Dilute 1 µL of the total RNA solution 100-fold and measure the OD values at 260 nm and 280 nm using a full-wavelength microplate reader. An OD260/OD280 ratio between 1.8 and 2.0 indicates high RNA purity, suitable for subsequent experiments.

### (2) cDNA synthesis

Following the method described in the kit manual, subsequent experiments were conducted using a 20 µL reverse transcription reaction system:

| Components | Volume |
|---|---|
| 5 × qRTSuperMix II | 4 µL |
| RNase-free Water | 8 µL |
| Total RNA | 8 µL |
| Total volume | 20 µL |

Gently pipette to mix the above reagents. The reaction conditions are as follows:

| Temperature | Time |
|---|---|
| 25 °C | 10 min |
| 42 °C | 30 min |
| 85 °C | 5 min |
| 4 °C | - |

The synthesized cDNA is stored at -80 °C or used immediately for subsequent Q-PCR reactions.

### (3) Primer Design

Primers were designed based on the nucleotide sequence of the mouse in GenBank and synthesized. Detailed information is as follows:

| Primers | | Sequence (5'-3') |
|---|---|---|
| *Gapdh* | Forward | GACATTTGAGAAGGGCCACAT |
| | Reverse | CAAAGAGGTCCAAAACAATCG |
| *Tnf* | Forward | CGTGCCTATGTCTCAGCCTCTT |
| | Reverse | GCCATAGAACTGATGAGAGGGAG |
| *Il1* | Forward | TGGACCTTCCAGGATGAGGACA |
| | Reverse | GTTCATCTCGGAGCCTGTAGTG |
| *Il6* | Forward | TACCACTTCACAAGTCGGAGGC |
| | Reverse | CTGCAAGTGCATCATCGTTGTTC |

### (4) PCR Amplification

Perform subsequent experiments using a 20 µL reaction system according to the kit instructions:

| Components | Volume |
|---|---|
| AceQ^{®} qPCR SYBR^{®} Green Master Mix | 10 µL |
| Forward primer (10 µM) | 0.4 µL |
| Reverse primer (10 µM) | 0.4 µL |
| cDNA | 2 µL |
| RNase-free Water | 7.2 µL |
| Total volume | 20 µL |

After adding the above reagents to micro reaction tubes, seal them with the cap and place them in the Q-PCR instrument. Set the parameters according to the following reaction conditions to perform the amplification experiment. The reaction conditions are as follows:

| Stages | Temperature (°C) | Time | Raps |
|---|---|---|---|
| Stage 1 Predegeneration | 95 | 5 min | 1 |
| Stage 2 Circular reaction | 95 | 10 sec | 40 |
| | Tm | 30 sec | |
| Stage 3 Melting curve | 95 | 15 sec | 1 |
| | 60 | 60 sec | |
| | 95 | 15 sec | |

Record the threshold cycle (Ct) values for each group and perform calculation and analysis using the 2^{-ΔΔCt} method (setting the expression level of the reference gene Gapdh as 1 to correct the expression levels of the target genes).

Data Statistics: All data are presented as Means ± S.E.M. Analysis of variance (ANOVA) was performed to assess the significance of differences. Groups showing significant differences in ANOVA were further compared using one-way ANOVA and Dunnett's test. A P-value < 0.05 was considered statistically significant.

### Experimental Results

### Effects on the Pulmonary Inflammatory Response in Sepsis-Induced Mice

As shown in Table 4, compared with the control group, the mRNA expression levels of inflammatory cytokines (TNF, IL-6, IL-1) in the lung tissue of sepsis-induced mice were significantly elevated, indicating a pronounced inflammatory response in the lungs of these mice. Following intervention with Compound 1 (1 mg/kg), Hydrochloride 20 (1 mg/kg), and Fumarate 21 (1 mg/kg), mRNA expression of TNF, IL-6, and IL-1 in mouse lung tissue was markedly downregulated. Notably, at equivalent doses, Hydrochloride 20 exhibited greater potency than Compound 1 and Fumarate 21. These findings indicate that Compound 1 and its prodrug salts suppress inflammatory cytokine expression and mitigate lung injury in septic mice.

**Table 4. Expression of Lung Inflammatory Factor mRNAs**

| Group | *Tnf* | *Il1* | *Il6* |
|---|---|---|---|
| Normal | 1.00+0.07 | 1.00±0.04 | 1.00±0.01 |
| LPS | 10.92±1.57^{##} | 19.01±2.40^{##} | 20.34±1.96^{##} |
| **1** (0.1 mg/kg) | 8.58+0.85 | 15.29±0.88 | 19.17±0.51 |
| **1** (1 mg/kg) | 7.99±1.36 | 7.89±1.44^{*} | 11.67±1.84^{*} |
| **20** (1 mg/kg) | 6.52+0.29 | 5.22±0.90^{**} | 7.16±0.71^{**} |
| **21**(0.1 mg/kg) | 8.79±1.18 | 13.30+0.88 | 18.40±3.16 |
| **21**(1 mg/kg) | 8.67±1.51 | 6.79+0.72^{**} | 9.10+1.20^{**} |
| **Dex**(5mg/kg) | 8.99+0.76 | 7.20+0.36^{*} | 9.88+0.80^{**} |

| | | | |
|---|---|---|---|
| Note: Compared with the Normal group, ^{##}P < 0.01; compared with the LPS group, *P < 0.05, **P < 0.01. | | | |

### Effects on Hepatic Inflammatory Responses in Sepsis-Induced Mice

As shown in Table 5, compared with the control group, the mRNA expression levels of inflammatory factors (TNF, IL-6, IL-1) in the liver tissues of sepsis-induced mice were significantly elevated, indicating a pronounced inflammatory response in the livers of these mice. Following intervention with Compound 1 (1 mg/kg), Hydrochloride 20 (1 mg/kg), and Fumarate 21 (1 mg/kg), mRNA expression of TNF, IL-6, and IL-1 in mouse liver tissue was markedly downregulated. Notably, at equivalent doses, Hydrochloride Salt 20 exhibited significantly stronger activity than Compound 1 and Fumarate Salt 21. These results indicate that Compound 1 and its prodrug salts suppress inflammatory factor expression and improve liver injury in septic mice.

**Table 5. Expression of Hepatic Inflammatory Factor mRNAs**

| Group | *Tnf* | *Il1* | *Il6* |
|---|---|---|---|
| Normal | 1.00+0.01 | 1.00+0.11 | 1.00+0.08 |
| LPS | 8.92±1.93^{##} | 11.52±1.19^{##} | 11.34±0.75^{##} |
| **1** (0.1 mg/kg) | 8.68+2.12 | 11.32±2.66 | 8.50±3.02 |
| **1** (1 mg/kg) | 7.01±0.92 | 4.84±0.60^{**} | 5.10±0.82^{**} |
| **20**(1 mg/kg) | 5.71±0.61 | 3.07±0.29^{**} | 3.93±0.90^{**} |
| **21**(0.1 mg/kg) | 7.78±1.68 | 10.32±1.45 | 8.96±0.70 |
| **21**(1 mg/kg) | 7.12±1.12 | 4.22±0.90^{**} | 4.82±0.69^{**} |
| **Dex**(5 mg/kg) | 7.04±0.77 | 7.54±0.70^{*} | 4.27±1.33^{*} |

| | | | |
|---|---|---|---|
| Note: Compared with the Normal group, ^{##}P < 0.01; Compared with the LPS, group **P < 0.01. | | | |

### Effects on Renal Inflammatory Responses in Sepsis-Induced Mice

As shown in Table 6, compared with the control group, the mRNA expression levels of inflammatory factors (TNF, IL-6, IL-1) in renal tissues of sepsis-induced mice were significantly elevated, indicating a pronounced inflammatory response in the kidneys of these mice. Following intervention with Compound 1 (1 mg/kg), Hydrochloride 20 (1 mg/kg), and Fumarate 21 (1 mg/kg), mRNA expression of TNF, IL-6, and IL-1 in renal tissue was markedly downregulated. Notably, at equivalent doses, Hydrochloride 20 exhibited significantly stronger activity than Compound 1 and Fumarate 21. These findings indicate that Compound 1 and its prodrug salts suppress inflammatory cytokine expression and mitigate renal injury in septic mice.

**Table 6. mRNA Expression of Renal Inflammatory Factors**

| Group | *Tnf* | *Il1* | *Il6* |
|---|---|---|---|
| Normal | 1.00±0.04 | 1.00±0.04 | 1.00±0.03 |
| LPS | 7.30±0.51^{##} | 7.99±0.85^{##} | 8.80±1.67^{##} |
| **1** (0.1 mg/kg) | 7.17±1.22 | 7.01±0.33 | 7.55±2.08 |
| **1** (1 mg/kg) | 6.59±0.18 | 3.05±0.58^{**} | 3.11±0.29^{*} |
| **20** (1mg/kg) | 6.20±0.60 | 2.76±0.59^{**} | 2.43±0.64^{*} |
| **21** (0.1mg/kg) | 6.70±0.80 | 6.56±0.88 | 6.05±0.19 |
| **21** (1 mg/kg) | 6.04±1.62 | 2.82±0.33^{**} | 2.70±0.52^{*} |
| **Dex** (5 mg/kg) | 6.62±0.68 | 6.05±1.03 | 5.76±0.72^{*} |

| | | | |
|---|---|---|---|
| Note: Compared with the Normal group, ^{##}P < 0.01; compared with the LPS group, *P < 0.05, **P < 0.01. | | | |

### Example 5

Activity Testing of Compound 1 and Its Dimethyl Hydrochloride Prodrug 20 and Fumarate Prodrug 21 Against Acute Lung Injury:
Experimental Reagents: Drugs were prepared as in Example 2; LPS was purchased from Sigma-Aldrich (USA); Dexamethasone (Dex) was purchased from Sigma-Aldrich (USA); ELISA kits were purchased from Dakewe Biotech Co., Ltd.
Experimental Animals: Male C57BL/6 mice, 6-8 weeks old, weighing 18-20 g, purchased from GemPharmatech LLC. [Production License No.: SCXK(Su)2018-0008]. Animals had free access to food and water and were housed at room temperature of 22 ± 2 °C and humidity of 45 ± 10%. After a 7-day acclimatization period, mice were used for subsequent experiments.
Experimental Methods: Establishment of an Acute Lung Injury Model in Mice and Group-Specific Drug Administration
Gastrointestinal administration of 1, 20, and 21 for treating acute lung injury: Male C57BL/6J mice aged 6-8 weeks weighing 18-20 g were randomly assigned by body weight to the control group, model group, 1, 20, and 21 groups (0.1, 1 mg/kg), and positive control drug Dex (5 mg/kg) group, with 8 mice per group. Except for the normal group receiving PBS via gastric lavage, all other mice were administered LPS (7.5 mg/kg) via intraperitoneal injection to establish an acute lung injury model. Compounds 1 (0.1, 1 mg/kg), hydrochloride 20 (0.1, 1 mg/kg), fumarate 21 (0.1, 1 mg/kg), and positive control Dex (5 mg/kg) were administered via oral gavage for three days prior to modeling. LPS (7.5 mg/kg) was intraperitoneally injected 30 minutes after the third day of compound administration. Lung tissue was collected 12 hours post-modeling. ELISA was used to detect the effects of Compounds 1, 20, and 21 on inflammatory cytokine expression in lung tissue from acutely injured mice.
Specimen Collection: Twelve hours after the final drug administration, mice were euthanized by cervical dislocation. The abdomen was opened, and the lungs were removed. They were rinsed twice with pre-chilled PBS. A section of lung tissue was excised and fixed in 4% paraformaldehyde for 24-48 hours. The remaining tissue was stored at -70°C for future use.
ELISA Analysis: Total protein extraction
Accurately weigh 20 mg of lung tissue, rinse with pre-chilled PBS solution, cut into small pieces using ophthalmic scissors, place into a glass homogenizer, add 180 µL PBS (containing 1 mM PMSF), homogenize at 60 Hz for 180 seconds, centrifuge at 3000 rpm/min at 4 °C for 20 minutes, take 1 µL of supernatant, measure protein concentration using Nanodrop, and adjust to a uniform concentration for subsequent experiments.
Data Statistics: All data are expressed as means ± S.E.M. Statistical differences between groups were analyzed using one-way ANOVA and t-tests in SPSS software. P values less than 0.05 were considered statistically significant.

### Experimental Results

### Effects of Intragastric Administration of Compounds 1, 20, and 21 on Acute Lung Injury in Mice

As shown in Table 7, compared with the control group, the expression levels of inflammatory cytokines (IL-1β, IL-6) in mouse lung tissue were significantly elevated, indicating a pronounced inflammatory response in the lungs. Following intervention with Compound 1 (0.1, 1 mg/kg), Hydrochloride 20 (0.1, 1 mg/kg), and Fumarate 21 (0.1, 1 mg/kg), IL-1β and IL-6 protein expression in mouse lung tissue was markedly downregulated. Notably, at equivalent doses, Hydrochloride 20 exhibited greater activity than Compound 1 and Fumarate 21. These results indicate that Compound 1 and its prodrug salt suppress inflammatory cytokine expression and improve acute lung injury in mice.

**Table 7. Expression of Inflammatory Cytokines (IL-1β and IL-6) in Serum of Mice Treated with Intraperitoneal Injection at 1, 20, and 21 Hours**

| Group | Number | IL-1β (pg/mL) | IL-6 (pg/mL) |
|---|---|---|---|
| Normal | 8 | 88.94 ± 0.91 | 71.14 ± 9.55 |
| LPS | 8 | 2411.32 ± 48.50 | 3520.94 ± 222.10 |
| **1** (0.1 mg/kg) | 8 | 1824.88 ± 32.73 | 2760.57 ± 288.02 |
| **1** (1 mg/kg) | 8 | 945.66 ± 36.71 | 1314.91 ± 231.08 |
| **20** (0.1 mg/kg) | 8 | 1570.02 ± 62.95^{*} | 2348.32 ± 202.75 |
| **20** (1 mg/kg) | 8 | 708.74 ± 38.40^{*} | 1172.00 ± 240.50 |
| **21** (0.1 mg/kg) | 8 | 1623.55 ± 32.64^{*} | 2585.26 ± 273.50 |
| **21** (1 mg/kg) | 8 | 888.28 ± 35.20^{*} | 1288.32 ± 123.80^{**} |
| **Dex** (5 mg/kg) | 8 | 1873.00 ± 86.52 | 1423.00 ± 168.40^{***} |

| | | | |
|---|---|---|---|
| Note: *p < 0.05, **p < 0.01, *** p < 0.001 (compared with the LPS group) | | | |

### Example 6

Anti-hepatotoxicity activity testing of compound 1 and its prodrugs: dimethyl hydrochloride prodrug 20 and fumarate prodrug 21:
Experimental Reagents: The drug of the present invention, prepared according to Example 2; Silybin, Shanghai Yuanye Biotechnology Co., Ltd.; Olive oil, Beijing Kelipeite Olive Oil Development Center; Analytical grade carbon tetrachloride (CCl₄), Shantou Xilong Chemical Factory; Sodium carboxymethyl cellulose (CMC-Na), Guangdong Shantou Xilong Chemical Factory; RNA Isolater Total RNA Extraction Reagent, Hiscript Q RT Supermix for qPCR, Nanjing Vazyme Biotech Co., Ltd.; AceQ qPCR SYBR Green Master Mix, Shanghai Yeasen Biotechnology Co., Ltd.; Aspartate Transaminase (AST/GOT) Colorimetric Test Kit, Alanine Transaminase (ALT/GPT) Colorimetric Test Kit, Wuhan Elabscience Biotechnology Co., Ltd.
Experimental animals: C57BL/6 mice, male, 6-8 weeks old, weighing 18-20 g, supplied by Shanghai Slac Animal Breeding Center, Production License No.: SCXK(Su)2019-002. Animals had free access to food and water, were fed standard pellet diet, maintained at room temperature 22 ± 2 °C, and humidity 45 ± 10%. They were acclimated for 7 days before use in experiments.
Experimental Methods: Establishment of Acute Liver Injury Model in Mice and Group-Specific Drug Administration
Mice were randomly assigned to groups. One group was arbitrarily designated as the normal group, while the remaining groups served as model group, Compound 1 group, Hydrochloride 20 group, Fumarate 21 group, and Silibinin group, with 6 mice per group. Except for the normal group receiving 10 mL olive oil/kg via injection, all other mice received 0.5 mL CCl₄ + 9.5 mL olive oil/kg body weight via intraperitoneal injection, maintaining a CCl₄ to olive oil ratio of 1:19. Starting from day 1 of modeling, Compound 1, Hydrochloride 20, and Fumarate 21 were administered orally for 3 consecutive days. The normal and model groups received equal volumes of solvent (0.5% CMC-Na) via gavage.
Specimen Collection: One hour after the final drug administration, blood was collected from the retinal venous plexus. The blood was allowed to stand at room temperature for 2 hours, then centrifuged at 3000 rpm for 20 minutes. Serum was aspirated, aliquoted, and stored at -70 °C for future use. After blood collection, euthanize mice by cervical dislocation. Perform laparotomy to remove the liver. Rinse twice with pre-chilled PBS. Excise the left hepatic lobe tissue. Fix in 4% paraformaldehyde for 24-48 hours. Store remaining tissue at -70 °C for future use.
Serum AST and ALT Assay: Use 10 µL of each sample for testing. Determine mouse serum AST and ALT levels according to the operating manual. Measure absorbance at 510 nm using an enzyme-linked immunosorbent assay (ELISA) reader. Calculate sample concentration (U/L) based on the standard curve.

Q-PCR Analysis: Tissue total RNA extraction, cDNA synthesis, and PCR amplification methods are identical to Example 4. Additional primer information is as follows:

| Primers | | Sequence (5'-3') |
|---|---|---|
| | Reverse | CTGCAAGTGCATCATCGTTGTTC |
| *Acta2* | Forward | GGCTTCGCTGGTGATGATGCTC |
| | Reverse | TTGGTGATGATGCCGTGTTCTATCG |
| *Col1a1* | Forward | GACAGGCGAACAAGGTGACAGAG |
| | Reverse | CAGGAGAACCAGGAGAACCAGGAG |
| *Tgfb1* | Forward | ACCGCAACAACGCCATCTATGAG |
| | Reverse | GGCACTGCTTCCCGAATGTCTG |

Data Statistics: All data are expressed as means ± standard error of the mean (S.E.M.). Differences were analyzed for significance using analysis of variance (ANOVA). For groups showing significant differences in ANOVA, one-way ANOVA followed by Dunnett's test was performed to compare intergroup differences. A p-value < 0.05 was considered statistically significant.

### Experimental Results

### Effects on Serum ALT and AST Levels

Changes in serum ALT and AST levels serve as crucial indicators for assessing liver injury. To confirm whether CCl₄ indeed induced acute liver injury in mice, this study first measured serum ALT and AST levels in the model group. As shown in Table 8, serum ALT and AST levels in CCl₄-treated mice significantly increased compared to the normal olive oil group. This indicates the successful establishment of the acute liver injury model in mice. Furthermore, oral administration of Compound 1 (1 mg/kg), Hydrochloride 20 (1 mg/kg), and Fumarate 21 (1 mg/kg) significantly reduced serum ALT and AST levels in mice. Notably, at equivalent doses, Hydrochloride 20 exhibited greater activity than the parent drug 1 and Fumarate 21.

**Table 8. ALT and AST Levels**

| Group | ALT | AST |
|---|---|---|
| Normal | 9.47±3.21 | 41.42±4.62 |
| LPS | 171.46±3.42^{##} | 430.64±54.91^{##} |
| **1** (0.1 mg/kg) | 173.58+22.75 | 424.52+175.34 |
| **1** (1 mg/kg) | 104.22+5.21^{**} | 237.16±36.70^{*} |
| **20** (1 mg/kg) | 85.02±15.84^{**} | 153.34±27.59^{**} |
| **21** (0.1 mg/kg) | 132.32±31.96 | 385.64±28.70 |
| **21** (1 mg/kg) | 95.82±2.72^{**} | 215.82±63.48^{*} |
| Silybin (200 mg/kg) | 121.58±15.45^{*} | 301.45±38.44^{*} |

| | | |
|---|---|---|
| Note: Compared with the Normal group, ^{##}P < 0.01; compared with the CCl₄ group, ^{*}P < 0.05, **P < 0.01. | | |

### Effects on mRNA Expression of Hepatic Fibrosis-Related Factors

The effects of Compound 1, Hydrochloride 20, and Fumarate 21 on proinflammatory factors in mouse liver were evaluated by measuring changes in mRNA expression levels of fibrosis-related factors (Acta2, Col1a1, Tgfb1) in liver tissue across all groups. As shown in Table 9, compared with the Normal group, the mRNA levels of Acta2, Col1a1, and Tgfb1 in liver tissue were significantly elevated 72 h after intraperitoneal CCl₄ administration. Treatment with Compound 1 (1 mg/kg), Hydrochloride 20 (1 mg/kg), and Fumarate 21 (1 mg/kg) significantly reduced their expression. Notably, at equivalent doses, hydrochloride 20 exhibited stronger activity than compound 1 and fumarate 21. These results indicate that compound 1 and its prodrug salts can partially inhibit CCl₄-induced liver injury.

**Table 9. mRNA Expression of Factors Associated with Liver Fibrosis**

| Group | *Acta2* | *Col1a1* | *Tgfb1* |
|---|---|---|---|
| Normal | 1.00+0.00 | 1.00±0.00 | 1.00+0.00 |
| LPS | 4.35+0.71^{##} | 2.75±0.25^{##} | 1.66±0.12^{##} |
| **1** (0.1 mg/kg) | 3.82±0.88 | 2.28±0.80 | 1.39±0.58 |
| **1** (1 mg/kg) | 2.34±0.16^{*} | 1.40±0.31^{**} | 1.08±0.21^{**} |
| **20** (1 mg/kg) | 1.85±0.77^{**} | 1.16±0.26^{**} | 0.92±0.21^{**} |
| **21** (0.1 mg/kg) | 2.86±0.26 | 1.83±0.53 | 1.08±0.29^{**} |
| **21** (1 mg/kg) | 2.18±0.42^{*} | 1.26±0.27^{**} | 0.88±0.16^{**} |
| **Silybin** (200 mg/kg) | 2.91±0.48^{*} | 1.84±0.17^{*} | 1.11±0.08^{*} |

| | | | |
|---|---|---|---|
| Note: Compared with the Normal group, ^{##}P < 0.01; Compared with the CCl₄ group, ^{**}P < 0.01. | | | |

### Example 7

Effects of Compound 1 and Its Dimethyl Hydrochloride Prodrug 20 and Fumarate Prodrug 21 on Folic Acid and Unilateral Ureteral Occlusion (UUO)-Induced Renal Injury in Mice:
Experimental Reagents: The drug of the present invention, prepared according to Example 2; Folic acid (FA), MP Biomedicals; Sodium carboxymethylcellulose (CMC-Na), Shantou Xilong Chemical Factory, Guangdong; RNA Isolater Total RNA Extraction Reagent, Hiscript Q RT Supermix for qPCR, Nanjing Vazyme Biotech Co., Ltd.; AceQ qPCR SYBR Green Master Mix, Shanghai Yeasen Biotechnology Co., Ltd.; Urea Colorimetric Test Kit, Creatinine Colorimetric Test Kit, Wuhan Elabscience Biotechnology Co., Ltd.
Experimental Animals: Male C57BL/6 mice, 6-8 weeks old, weighing 18-20 g, supplied by Shanghai Slac Animal Breeding Center, Production License No.: SCXK(Su)2019-002. Animals had free access to food and water, were fed standard pellet diet, maintained at room temperature 22 ± 2 °C and humidity 45 ± 10%. They were acclimated for 7 days before experimentation.
Experimental Methods: Establishment of Mouse Kidney Injury Model and Group-Specific Drug Administration
Acute Kidney Injury: Mice were randomly assigned to groups, with one group arbitrarily designated as the normal group and the remaining groups as model group, Compound 1 (0.1, 1 mg/kg), Hydrochloride 20 (1 mg/kg), and Fumarate 21 (0.1, 1 mg/kg), each containing 6 mice. Except for the normal group, all other mice were administered folic acid (250 mg/kg) dissolved in 0.9% NaHCO₃ solution via intraperitoneal injection to establish an acute kidney injury model. One hour prior to model establishment, Compound 1 (0.1, 1 mg/kg), Hydrochloride 20 (1 mg/kg), and Fumarate 21 (0.1, 1 mg/kg) were administered via oral gavage for 3 consecutive days. Mice in the normal and model groups received equal volumes of solvent (0.5% CMC-Na) via oral gavage.
Chronic Kidney Injury: Mice were randomly divided into five groups. One group was arbitrarily designated as the normal group, while the remaining four groups served as model group, Compound 1 (0.1, 1 mg/kg), Hydrochloride 20 (1 mg/kg), and Fumarate 21 (0.1, 1 mg/kg) group, each containing six mice. Except for the sham-operated mice in the normal group (where only the left ureter was exposed without further manipulation), all other mice underwent unilateral ureteral ligation to establish a chronic kidney injury model as follows: The abdominal skin was disinfected with iodine solution followed by alcohol. An incision was made, and the abdominal layers were dissected sequentially. The left ureter was fully exposed and dissected. A double ligation was performed at the bladder-proximal end of the left ureter using absorbable suture material, followed by closure. On the first day of model establishment, Compound 1 (0.1, 1 mg/kg), Hydrochloride 20 (1 mg/kg), and Fumarate 21 (0.1, 1 mg/kg) were administered via oral gavage for 14 consecutive days. Mice in the normal and model groups received an equal volume of solvent (0.5% CMC-Na) via oral gavage.
Specimen Collection: One hour after the final drug administration, blood was collected from the retinal venous plexus. The blood was allowed to stand at room temperature for 2 hours, then centrifuged at 3000 rpm for 20 minutes. Serum was aspirated, aliquoted, and stored at -70 °C for future use. Following blood collection, euthanize mice by cervical dislocation. Perform laparotomy to remove kidneys, dissect out perirenal fat and capsules. Rinse twice with pre-chilled PBS, then fix in 4% paraformaldehyde for 24-48 hours. Paraffin-embedded specimens undergo HE staining; remaining tissue is stored at -70°C for future use.
Serum Creatinine (Scr) and Blood Urea Nitrogen (BUN) Level Assay: 10 µL of serum from each sample was used for creatinine and urea level detection. Samples were added sequentially strictly following the kit instructions. After incubation at 37°C, absorbance values were measured using an enzyme-linked immunosorbent assay (ELISA) reader. The concentration levels of each indicator were calculated based on the standard curve.
Q-PCR Analysis: Total RNA extraction from animal tissues, cDNA synthesis, and PCR amplification were performed as described in the preceding examples. Additional primer information is shown in Table 10:

**Table 10. Q-PCR Primer Information**

| Primers | | | Sequence (5'-3') |
|---|---|---|---|
| | | Reverse | CTGCAAGTGCATCATCGTTGTTC |
| | *Ngal* | Forward | AATGTCACCTCCATCCTGGT |
| | | Reverse | ATTTCCCAGAGTGAACTGGC |
| | *Havrcl* | Forward | CCACGCTTAGAGATGCTGACTTCC |
| | | Reverse | CCTGCTGCTACTGCTCCTTGTG |
| | *Klotho* | Forward | TCTCAAGAAGTTCATAATGGAAACC |
| | | Reverse | CAGAAAGTCAACGTAGAAGAGTCCT |

### Experimental Results

### Effects on Serum Urea and Creatinine Levels

As shown in Tables 11 and 12, compared with mice in the Normal group, serum urea and creatinine levels significantly increased in mice administered folic acid after 48 hours. However, in the treatment groups with Compound 1 (1 mg/kg) Hydrochloride 20 (1 mg/kg), and Fumarate 21 (1 mg/kg) exhibited significantly lower urea and creatinine levels than the model group, with statistically significant differences (P < 0.05). Notably, at equivalent doses, hydrochloride 20 exhibited greater potency than compound 1 and fumarate 21. These findings indicate that compound 1 and its prodrug salts provide effective protection against acute and chronic kidney injury induced by folic acid or unilateral ureteral ligation.

**Table 11. Serum Creatinine and Urea Levels-Acute Model**

| Group | *Scr* | *BUN* |
|---|---|---|
| Normal | 44.90±5.63 | 7.07±0.76 |
| FA | 124.49±12.85^{##} | 33.18±2.54^{##} |
| **1** (0.1 mg/kg) | 108.48±11.76 | 31.46±2.54 |
| 1 (1 mg/kg) | 71.95±5.74^{**} | 21.33±2.21^{**} |
| **20** (1 mg/kg) | 64.94±9.68^{**} | 16.74±1.42^{**} |
| **21** (0.1 mg/kg) | 90.69±12.83 | 30.08±3.03 |
| **21**(1 mg/kg) | 50.39±7.65^{**} | 18.95±2.58^{**} |

| | | |
|---|---|---|
| Note: Compared with the Normal group, ^{##}P < 0.01; compared with the FA group, *P < 0.05, **P < 0.01. | | |

**Table 12. Serum Creatinine and Urea Levels-Chronic Model**

| Group | Scr | BUN |
|---|---|---|
| Normal | 37.07±7.66 | 7.60±0.98 |
| UUO | 201.57±33.25^{##} | 20.36±1.44^{##} |
| **1** (0.1 mg/kg) | 176.35±17.11 | 18.41±1.82 |
| **1** (1 mg/kg) | 104.65±16.26^{*} | 11.01±1.54^{**} |
| **20** (1 mg/kg) | 64.50±7.13^{**} | 9.39±0.92^{**} |
| **21** (0.1 mg/kg) | 157.79±21.92 | 17.63±1.13 |
| **21** (1 mg/kg) | 79.87±13.44^{**} | 9.45±1.55^{**} |

| | | |
|---|---|---|
| Note: Compared with the Normal group, ^{##}P < 0.01; compared with the UUO group, *P < 0.05, **P < 0.01. | | |

### Effects on mRNA Expression of Renal Injury Markers

The effects of the parent drug 1, hydrochloride 20, and fumarate 21 on renal function in mice were evaluated by measuring changes in mRNA expression levels of renal injury markers (Ngal, Havcr1) in kidney tissues across all groups. As shown in Tables 13 and 14, compared with the Normal group, Ngal and Havcr1 mRNA levels in renal tissues were significantly elevated in mice administered folic acid for 48 h or subjected to unilateral ureteral ligation. Treatment with Compound 1 (1 mg/kg), Hydrochloride 20 (1 mg/kg), and Fumarate 21 (1 mg/kg) markedly reduced Ngal and Havcr1 mRNA levels. Notably, at equivalent doses, hydrochloride 20 exhibited greater potency than compound 1 and fumarate 21. These findings indicate that compound 1 and its prodrug salts can suppress the elevation of renal injury markers induced by folic acid or unilateral ureteral ligation.

**Table 13. mRNA Expression of Kidney Injury Markers-Acute Model**

| Group | *Havcr1* | *Ngal* |
|---|---|---|
| Normal | 1.00±0.05 | 1.00±0.04 |
| FA | 199.26±39.19^{##} | 36.25±6.78^{##} |
| **1** (0.1 mg/kg) | 175.10±17.63 | 30.52±5.05 |
| **1** (1 mg/kg) | 99.99±7.98^{*} | 20.17±2.21^{*} |
| **20** (1 mg/kg) | 62.86±12.20 | 13.05±1.82^{**} |
| **21**(0.1 mg/kg) | 170.08±33.10 | 25.75±2.80 |
| **21** (1 mg/kg) | 83.11±16.85^{*} | 13.08±2.41^{**} |

| | | |
|---|---|---|
| Note: Compared with the Normal group, ^{##}P < 0.01; compared with the FA group, *P < 0.05, **P < 0.01. | | |

**Table 14. mRNA Expression of Kidney Injury Markers-Chronic Model**

| Group | *Havcr1* | *Ngal* |
|---|---|---|
| Normal | 1.00±0.04 | 1.00±0.05 |
| UUO | 14.49±2.23^{##} | 45.13±7.89^{##} |
| **1** (0.1 mg/kg) | 12.56±1.95 | 37.82±4.85 |
| **1** (1 mg/kg) | 8.54±1.38^{*} | 21.66±6.00^{*} |
| **20** (1 mg/kg) | 6.29±2.43^{**} | 16.14±2.74^{**} |
| **21** (0.1 mg/kg) | 11.90±4.52 | 35.77±7.67 |
| **21** (1 mg/kg) | 7.25±0.80^{*} | 19.42±1.55^{**} |

| | | |
|---|---|---|
| Note: Compared with the Normal group, ^{##}P < 0.01; compared with the UUO group, ^{*}P < 0.05, **P < 0.01. | | |

### Effects on the mRNA Expression of Inflammatory Factors in Kidney Tissue

The effects of Compound 1, Hydrochloride 20, and Fumarate 21 on proinflammatory factors in mouse kidneys were evaluated by measuring changes in mRNA expression levels of inflammatory cytokines (TNF, IL-6, IL-1) in renal tissues across groups. As shown in Tables 15 and 16, compared with the Normal group, renal tissue mRNA levels of TNF, IL-6, and IL-1 significantly increased after 48 h of folic acid administration or unilateral ureteral ligation. Treatment with Compound 1 (1 mg/kg), Hydrochloride 20 (1 mg/kg), and Fumarate 21 (1 mg/kg) significantly reduced their expression. Notably, at equivalent doses, hydrochloride salt 20 exhibited greater potency than compound 1 and fumarate salt 21. These findings indicate that compound 1 and its prodrug salts can modulate folic acid- or unilateral ureteral ligation-induced renal inflammatory responses to varying degrees.

**Table 15. Renal Inflammatory Factor mRNA Expression-Acute Model**

| Group | *Tnf* | *Il1* | *Il6* |
|---|---|---|---|
| Normal | 1.00±0.06 | 1.00±0.05 | 1.00±0.02 |
| FA | 3.10±0.56^{##} | 2.94±0.35^{##} | 8.87±0.91^{##} |
| **1** (0.1 mg/kg) | 2.89±0.59 | 2.62±0.56 | 8.02±0.77 |
| **1** (1 mg/kg) | 2.76±0.71 | 1.72±0.45^{*} | 3.98±0.59^{**} |
| **20** (1 mg/kg) | 2.31±0.72 | 1.06±0.33^{**} | 1.55±0.28^{**} |
| **21** (0.1 mg/kg) | 2.60±0.62 | 2.07±0.38 | 6.47±0.53^{*} |
| **21** (1 mg/kg) | 2.42±0.35 | 1.45±0.23^{**} | 5.61±2.10^{**} |

| | | | |
|---|---|---|---|
| Note: Compared with the Normal group, ^{##}P < 0.01; compared with the FA group, ^{*}P < 0.05, **P < 0.01. | | | |

**Table 16. Renal Inflammatory Factor mRNA Expression-Chronic Model**

| Group | *Tnf* | *Il1* | *Il6* |
|---|---|---|---|
| Normal | 1.00±0.02 | 1.00±0.02 | 1.00±0.12 |
| UUO | 7.91±1.28^{##} | 7.98±1.04^{##} | 27.27±5.53^{##} |
| **1** (0.1 mg/kg) | 6.41±1.46 | 6.05±0.49 | 16.52±1.69 |
| **1** (1 mg/kg) | 6.59±1.19 | 3.99±0.31^{**} | 6.50±1.51^{**} |
| **20** (1 mg/kg) | 5.09+0.66 | 2.77±0.40^{*} | 3.69+1.26^{**} |
| **21** (0.1 mg/kg) | 7.10±1.93 | 5.32±0.56^{*} | 14.31±6.01^{*} |
| **21** (1 mg/kg) | 7.53±0.68 | 3.55±0.50^{**} | 5.61±2.10^{**} |

| | | | |
|---|---|---|---|
| Note: Compared with the Normal group, ^{##}P < 0.01; compared with the UUO group, *P < 0.05, **P < 0.01 | | | |

### Effects on mRNA Expression of Factors Associated with Renal Tissue Fibrosis

The effects of the parent drug 1, hydrochloride 20, and fumarate 21 on renal fibrosis in mice were evaluated by measuring changes in mRNA expression levels of fibrosis-related factors (α-Sma, Col1a1, Tgfb1) in kidney tissues across groups. As shown in Tables 17 and 18, compared with the Normal group, renal tissue mRNA levels of Acta2, Col1a1, and Tgfb1 were significantly elevated after 48 h of folic acid administration or unilateral ureteral ligation. Treatment with Compound 1 (1 mg/kg), Hydrochloride 20 (1 mg/kg), and Fumarate 21 (1 mg/kg) significantly reduced their expression. Notably, at equivalent doses, hydrochloride 20 exhibited stronger activity than compound 1 and fumarate 21. These results indicate that compound 1 and its prodrug salts can partially mitigate folate-induced renal fibrosis.

**Table 17. mRNA Expression of Renal Fibrosis-Related Factors-Acute Model**

| Group | *α-Sma* | *Col1a1* | *Tgfb1* |
|---|---|---|---|
| Normal | 1.00±0.06 | 1.00±0.04 | 1.00±0.03 |
| Folic acid | 2.16±0.26^{##} | 1.95±0.11^{##} | 2.11±0.29^{##} |
| **1** (0.1 mg/kg) | 1.82±0.19 | 1.64±0.16 | 1.96±0.17 |
| **1** (1 mg/kg) | 1.42±0.11^{*} | 1.32±0.15^{**} | 1.36±0.10^{*} |
| **20** (1 mg/kg) | 1.11±0.17^{**} | 1.18±0.15^{**} | 0.98±0.27^{**} |
| **21**(0.1 mg/kg) | 1.80±0.42 | 1.58±0.35 | 1.64±0.16 |
| **21** (1 mg/kg) | 0.90±0.21^{**} | 1.20±0.19^{**} | 1.09±0.15^{*} |

| | | | |
|---|---|---|---|
| Note: Compared with the Normal group, ^{##}P < 0.01; compared with the folic acid group, *P < 0.05, **P < 0.01. | | | |

**Table 18. mRNA Expression of Renal Fibrosis-Related Factors-Chronic Model**

| | *α-Sma* | *Col1a1* | *Tgfb1* |
|---|---|---|---|
| Normal | 1.00±0.04 | 1.00±0.01 | 1.00±0.02 |
| UUO | 2.37±0.26^{##} | 16.51±1.71^{#} | 2.12±0.16^{##} |
| **1** (0.1 mg/kg) | 2.32±0.54 | 16.09±3.57 | 2.04±0.24 |
| **1** (1 mg/kg) | 1.54±0.12^{*} | 9.45±1.03^{**} | 1.55±0.19^{*} |
| **20** (1 mg/kg) | 1.26±0.18^{**} | 7.30±0.90^{**} | 1.44±0.21^{*} |
| **21**(0.1 mg/kg) | 2.02±0.39 | 11.79±1.18^{*} | 1.76±0.05 |
| **21** (1 mg/kg) | 1.39±0.04^{**} | 8.27±1.15^{**} | 1.53±0.14^{*} |

| | | | |
|---|---|---|---|
| Note: Compared with the Normal group, ^{##}P < 0.01; compared with the UUO group, ^{*}P < 0.05, **P < 0.01. | | | |

### Example 8

Therapeutic Effect of Compound 20 in Lupus Nephritis:
Experimental Reagents: MCC950 (NLRP3 inhibitor), Shanghai Taoshu Biotechnology Co., Ltd.; Sodium Carboxymethylcellulose (CMC-Na), Shilong Chemical Factory, Shantou City, Guangdong Province; RNA Isolater Total RNA Extraction Reagent, Hiscript Q RT Supermix for qPCR, Nanjing Vazyme Biotech Co., Ltd.; Urea Colorimetric Test Kit, Creatinine Colorimetric Test Kit, Nanjing Jiancheng Biotechnology Co., Ltd.; Urinary Kim-1 and β-MG Content ELISA Kits, Abcam.
Experimental animals: MRL/MpJ and MRL/lpr mice, female, 10 weeks old, purchased from GemPharmatech LLC. Production license number: SCXK(Su)2023-0009. Animals had free access to food and water, were fed standard pellet diet, maintained at room temperature 22 ± 2 °C and humidity 45 ± 10%. They were acclimated for 7 days before use in experiments.

### Experimental Methods

### (1) Mouse Grouping and Drug Administration:

Normal group (MRL/MpJ), model group (MRL/lpr), positive control drug MCC950 (30 mg/kg) group, compound 20 (30 mg/kg) group, with 6 mice per group. Oral administration was performed continuously for 8 weeks. Mice in the normal and model groups received equal volumes of solvent (0.5% CMC-Na) via oral gavage.

Specimen Collection: One hour after the final drug administration, enucleate the eyeball to collect blood. Allow blood to stand at room temperature for 2 hours, then centrifuge at 3000 rpm for 20 minutes. Aspirate the serum, aliquot it, and store at -70 °C for future use. After blood collection, euthanize mice by cervical dislocation. Open the abdominal cavity, remove kidneys, and dissect out perirenal fat and capsules. Rinse twice with pre-chilled PBS, then fix in 4% paraformaldehyde for 24-48 hours. Store remaining tissues at -70 °C for future use.

Serum Creatinine (Scr) and Blood Urea Nitrogen (BUN) Assay: For each sample, 10 µL of serum was used for creatinine and urea level detection. Samples were added sequentially strictly according to the kit instructions, incubated at 37 °C, and measured for absorbance values using an enzyme-linked immunosorbent assay (ELISA) reader. The concentration levels of each indicator were calculated based on the standard curve.

Q-PCR Analysis: Total RNA extraction from animal tissues, cDNA synthesis, and PCR amplification were performed as described in the previous example. Additional primer information is as follows:

**Table 19. Q-PCR Primer Information**

| Primers | | Sequence (5'-3') |
|---|---|---|
| | Reverse | ATGTCTCAGCCTCTTCTCATTC |
| *Tnf* | Forward | GCTTGTCACTCGAATTTTGAGA |
| | Reverse | ATCTCGCAGCAGCACATCAA |
| *Il1* | Forward | ATGGGAACGTCACACACCAG |
| | Forward | TACCACTTCACAAGTCGGAGGC |
| *Il6* | Reverse | CTGCAAGTGCATCATCGTTGTTC |

### Experimental Results

**Effect on Urinary Protein:** As shown in Table 20, compared with the MpJ group, lpr mice exhibited significantly elevated 24-hour urinary protein levels. The compound 20 (30 mg/kg) treatment group demonstrated markedly lower 24-hour urinary protein content than the model group, with a statistically significant reduction in proteinuria superior to that observed in the MCC950 group (P < 0.05).

**Table 20. Urinary Protein Content**

| Group | 24-hour urine protein level |
|---|---|
| MpJ | 0.51±0.04 |
| lpr | 1.34±0.01^{##} |
| **MCC950** (30 mg/kg) | 0.89±0.11* |
| **20** (30 mg/kg) | 0.58±0.03** |

| | |
|---|---|
| Note: Compared with MpJ group, ^{##}P<0.01; Compared with lpr group, *P<0.05, ^{**}P<0.01. | |

**Effects on Renal Function Serum Markers:** As shown in Table 21, compared with MpJ group mice, serum urea and creatinine levels were significantly elevated in lpr mice. The compound 20 (30 mg/kg) treatment group exhibited markedly lower urea and creatinine levels than the model group, with overall efficacy superior to the MCC950 group. Differences were statistically significant (P<0.05). These findings indicate that Compound 20 exerts a protective effect on renal function in lupus nephritis mice.

**Table 21. Serum Creatinine and Urea Levels**

| Group | *Scr* | *BUN* |
|---|---|---|
| MpJ | 15.34±1.39 | 8.32±0.91 |
| lpr | 28.46±2.90^{##} | 17.49±1.06^{##} |
| **MCC950** (30 mg/kg) | 22.18±3.41 | 12.08±2.01* |
| **20** (30 mg/kg) | 18.55±1.08** | 9.55±0.75** |

| | | |
|---|---|---|
| Note: Compared with MpJ group, ^{##}P<0.01; Compared with lpr group, ^{*}P<0.05, ^{**}P<0.01. | | |

**Effects on urinary markers of renal injury:** The impact of compound 20 on renal function was evaluated by measuring changes in Kim-1 and β-MG expression levels in the urine of mice across groups. As shown in Table 22, compared with the control group, compound 20 suppressed the elevation of renal injury markers in the urine of lupus mice, with an overall effect superior to that of the MCC950 group, and the difference was statistically significant (P<0.05).

**Table 22. Levels of Urinary Kidney Injury Markers**

| Group | Kim-1 | β-MG |
|---|---|---|
| MpJ | 0.15±0.01 | 10.15±0.18 |
| lpr | 0.77±0.08^{##} | 18.93±0.25^{##} |
| **MCC950** (30 mg/kg) | 0.53±0.06* | 15.41±0.39 |
| **20** (30 mg/kg) | 0.41±0.02** | 13.55±0.14** |

| | | |
|---|---|---|
| Note: Compared with MpJ group, ^{##}P<0.01; Compared with lpr group, *P<0.05, ^{**}P<0.01. | | |

**Effects on mRNA expression of inflammatory factors in renal tissue:** The impact of Compound 20 on proinflammatory factors in mouse kidneys was evaluated by measuring mRNA expression levels of inflammatory factors (TNF, IL-6, IL-1) in renal tissues across groups. As shown in Table 23, compared with MpJ mice in the control group, the mRNA levels of TNF, IL-1, and IL-6 in the kidney tissues of lupus nephritis model LPR mice were significantly elevated. Treatment with Compound 20 (30 mg/kg) significantly reduced their expression, with statistically significant differences (P < 0.05). Thus, Compound 20 can reduce the inflammatory response in the kidneys of lupus nephritis mice to a certain extent.

**Table 23. mRNA Expression of Renal Inflammatory Factors**

| | Group | *Tnf* | *Il1* | *Il6* |
|---|---|---|---|---|
| | MpJ | 1.04±0.10 | 1.05±0.03 | 1.00±0.07 |
| | lpr | 6.23±0.75^{##} | 9.31±0.85^{##} | 15.44±1.07^{##} |
| **MCC950** (30 mg/kg) | | 4.33±0.52* | 5.43±0.71** | 9.52±0.84** |
| | **20** (30 mg/kg) | 4.04±0.33* | 6.01±0.99* | 11.80±1.90* |

| | | | | |
|---|---|---|---|---|
| Note: Compared with the MpJ group, ^{##}P < 0.01; compared with the lpr group, ^{*}P < 0.05, **P < 0.01. | | | | |

### Example 9

Therapeutic Effect of Compound 20 in Diabetic Nephropathy:
Experimental Reagents: Sodium Carboxymethylcellulose (CMC-Na), Xilong Chemical Factory, Shantou, Guangdong; Streptozocin (STZ), Sigma-Aldrich (Shanghai) Trading Co., Ltd.; RNA Isolater Total RNA Extraction Reagent, Hiscript Q RT Supermix for qPCR, Nanjing Vazyme Biotech Co., Ltd.; Urea Colorimetric Test Kit, Creatinine Colorimetric Test Kit, Nanjing Jiancheng Biotechnology Co., Ltd.; Urinary Kim-1 and β-MG Content ELISA Kits, Abcam.
Experimental animals: C57/BL6J mice, male, 10 weeks old, purchased from GemPharmatech LLC. Production license number: SCXK(Su)2023-0009. Animals had free access to food and water, were fed standard pellet diet, and maintained at room temperature of 22 ± 2 °C and humidity of 45 ± 10%. Animals were acclimated for 7 days before use in experiments.

### Experimental Methods

### (1) Mouse Modeling and Grouping for Drug Administration:

Administer streptozotocin (STZ, 50 mg/kg) via intraperitoneal injection for 5 consecutive days. After 72 hours, select mice with fasting blood glucose levels exceeding 16.7 mmol/L as model mice. Assign model mice to the following groups: Model Group (DKD), Compound 20 (1 mg/kg) Group, and Compound 20 (10 mg/kg) Group, with 6 mice per group. Model mice were fed a high-fat diet (HFD) providing 60% of energy from fat for 8 weeks. After 8 weeks, gavage administration commenced. Mice in the normal group (Ctrl) and model group (DKD) received equal volumes of solvent (0.5% CMC-Na) via gavage for 8 consecutive weeks.

Specimen Collection: One hour after the last drug administration, enucleate the eyeball to collect blood. Allow blood to stand at room temperature for 2 hours, then centrifuge at 3000 rpm for 20 minutes. Aspirate the serum, aliquot it, and store at -70 °C for future use. After blood collection, euthanize the mice by cervical dislocation. Open the abdominal cavity, remove the kidneys, and dissect away the perirenal fat and capsule. Store the tissue at -70 °C for future use.

Serum Creatinine (Scr) and Blood Urea Nitrogen (BUN) Assay: For each sample, 10 µL of serum was used for creatinine and urea level detection. Samples were added sequentially strictly following the kit instructions. After incubation at 37 °C, absorbance values were measured using an enzyme-linked immunosorbent assay (ELISA) reader. The concentration levels of each indicator were calculated based on the standard curve.

Q-PCR Analysis: Total RNA extraction from animal tissues, cDNA synthesis, PCR amplification, and primer information were performed as described in the preceding examples. Additional primer information is as follows:

**Table 24. Additional Information on Q-PCR Primers**

| Primers | | | Sequence (5'-3') |
|---|---|---|---|
| | | Reverse | GCTGCTACTGCTCCTTGTGA |
| | *Kim-1* | Forward | GGAAGGCAACCACGCTTAGA |
| | | Reverse | ATTTCCCAGAGTGAACTGGC |
| | *Mcp-1* | Forward | AACGCCCCACTCACCTGCTG |
| | | Reverse | GCTTCTTTGGGACACCTGCTGCT |
| | *Vimentin* | Forward | GAGCTATGTGACCACGTCCA |
| | | Reverse | CCGGGGGATGAGGAATAGAG |

### Experimental Results

**Effects on Key Renal Function Indicators:** As shown in Table 25, compared with Ctrl group mice, serum urea and creatinine levels were significantly elevated in DKD mice. In contrast, urea and creatinine levels in the Compound 20 (1 mg/kg) and Compound 20 (10 mg/kg) treatment groups were markedly lower than those in the model group, exhibiting a dose-dependent effect with statistically significant differences (P < 0.05). This indicates that Compound 20 exerts a favorable protective effect on renal function in DKD mice.

**Table 25. Serum Creatinine and Urea Levels**

| Group | Scr | BUN |
|---|---|---|
| Ctrl | 15.94±1.50 | 21.57±2.92 |
| DKD | 32.87±1.92^{##} | 63.03±4.24^{##} |
| **20** (1 mg/kg) | 17.72±1.658** | 37.01±2.50** |
| **20** (10 mg/kg) | 12.96±0.78** | 19.71±1.22** |

| | | |
|---|---|---|
| Note: Compared with the Ctrl group, ^{##}P < 0.01; compared with the DKD group, ^{**}P < 0.01. | | |

Effects on mRNA expression of key renal injury factors: The impact of Compound 20 on renal proinflammatory factors in mice was evaluated by measuring changes in mRNA expression levels of renal injury markers (Kim-1, Ngal) in kidney tissues across groups. As shown in Table 26, compared with the Ctrl group, Kim-1 and Ngal mRNA levels were significantly elevated in the kidney tissues of DKD model lpr mice. Treatment with Compound 20 (1 mg/kg) and Compound 20 (10 mg/kg) significantly reduced their expression with a dose-dependent effect, showing statistically significant differences (P<0.05). Thus, Compound 20 demonstrated efficacy in mitigating renal injury in DKD mice.

**Table 26. mRNA Expression Levels of Key Renal Injury Markers**

| Group | *Kim-1* | *Ngal* |
|---|---|---|
| Ctrl | 0.98±0.09 | 0.73±0.09 |
| DKD | 3.65±0.42^{##} | 3.76±0.55^{##} |
| **20** (1 mg/kg) | 1.68±0.17** | 1.38±0.17** |
| **20** (10 mg/kg) | 0.64±0.10** | 0.96±0.13** |

| | | |
|---|---|---|
| Note: Compared with the Ctrl group, ^{##}P < 0.01; compared with the DKD group, **P < 0.01. | | |

Effects on mRNA expression of inflammatory factors in renal tissue: The impact of Compound 20 on the expression of proinflammatory factors in mouse kidneys was evaluated by measuring changes in mRNA expression levels of inflammatory factors (MCP-1, TNF-α) in renal tissue across all groups. As shown in Table 27, compared with the Ctrl group, Mcp-1 and Tnf-α mRNA levels were significantly elevated in the kidney tissues of DKD model mice. Treatment with Compound 20 (1 mg/kg) and Compound 20 (10 mg/kg) significantly reduced their expression with a dose-dependent effect, showing statistically significant differences (P< 0.05). Thus, Compound 20 can attenuate renal inflammatory responses in DKD mice to a certain extent.

**Table 27. mRNA Expression Levels of Renal Tissue Inflammatory Factors**

| Group | *Mcp-1* | *Tnf-α* |
|---|---|---|
| Ctrl | 1.21±0.07 | 0.73±0.09 |
| DKD | 5.57±0.98^{##} | 3.76±0.55^{##} |
| **20** (1 mg/kg) | 2.33±0.20** | 1.38±0.17** |
| **20** (10 mg/kg) | 1.40±0.22** | 0.96±0.13** |

| | | |
|---|---|---|
| Note: Compared with the Ctrl group, ^{##}P < 0.01; compared with the DKD group, ^{**}P < 0.01. | | |

### Example 10

Activity Testing of Compound 1 for Treating Atopic Dermatitis

### 1. Effects of Compound 1 Ointment on Mouse Atopic Dermatitis

Preparation of Blank Ointment:
(1) Preparation of Oil Phase: Weigh white petrolatum, light liquid paraffin, glyceryl monostearate, ethylparaben, and cetyl alcohol. Heat to 65 °C while stirring until dissolved. Slowly add the dissolved active pharmaceutical ingredient (API) while continuing to stir until uniformly mixed. Set aside.
(2) Aqueous Phase Preparation: Weigh purified water and Tween 80, heat to 70 °C, stir to dissolve, and set aside;
(3) Emulsification: Slowly add the aqueous phase to the oil phase while maintaining 65 °C, homogenize, and continue stirring for 30 min;
(4) Cooling: Stop heating, continue stirring, gradually reduce to room temperature, cool to form an ointment, and fill containers.

### Preparation of 0.01%, 0.1%, and 1% Compound 1 Ointments and 1% Dexamethasone Ointment

Weigh out 0.005 g, 0.05 g, and 0.5 g of Compound 1 respectively, along with 0.5 g of dexamethasone. Dissolve these substances in a small amount of propylene glycol, then place the resulting solution into the aforementioned pre-cooled blank ointment base. Finally, add water to make the total mass up to 10 g, and grind the mixture thoroughly in a mortar. This yields 0.01%, 0.1%, and 1% Compound 1 ointments, as well as 1% dexamethasone ointment.

### Establishment of MC903-Induced Atopic Dermatitis (AD) Mouse Model and Drug Administration

Establishment of Mouse Ear Atopic Dermatitis Model and Drug Administration: Thirty-six mice were randomly divided into the Normal group, Model group (MC903), and groups treated with 0.01%, 0.1%, and 1% Compound 1, as well as Dexamethasone (Dex), with 6 mice per group. Except for the Normal group, all other groups underwent AD modeling: 20 µL of MC903 solution (also known as calcipotriol, purchased from Shanghai Yuanye Biotechnology Co., Ltd., used for inducing atopic models) was applied to each mouse's ears once daily for 14 consecutive days to induce the atopic dermatitis model. Starting 7 days post-modeling, apply 0.01%, 0.1%, or 1% Compound 1 and 20 mg dexamethasone ointment evenly to corresponding mice daily. Model and normal groups received blank ointment daily. Measure ear thickness changes every 2 days using an electronic caliper before modeling and after treatment initiation. Photographs of skin lesions were taken 24 hours after completion of modeling on day 14. Skin lesion and blood samples were collected from the mice.

Establishment of the AD Model on the Dorsum of Mice and Drug Administration: Thirty-six mice in the randomized group were divided into the Normal group, the Model group (MC903), and groups treated with 0.01%, 0.1%, and 1% Compound 1, and Dexamethasone (Dex), with 6 mice per group. Except for the Normal group, all other groups underwent AD modeling: 48 hours prior to the experiment, hair on the dorsal neck region was meticulously removed using clippers and depilatory cream. Following modeling initiation, 20 µL of MC903 solution was applied once daily to the dorsal neck region for 14 consecutive days to induce the AD model. Starting 7 days post-modeling, 0.01%, 0.1%, and 1% Compound 1 and 20 mg dexamethasone ointment were uniformly applied daily to corresponding mice. Model and control group mice received daily application of blank ointment. Twenty-four hours after completing the 14-day modeling period, photographs of skin lesions were taken, and skin lesion and blood samples were collected.

Skin Lesion Severity Score: Daily skin lesion severity was assessed in mice using the following criteria, primarily encompassing four symptoms: 1) erythema/hemorrhage; 2) dryness; 3) desquamation/erosion; 4) edema. Each symptom was scored from 0 to 3 based on lesion severity: no symptoms = 0 points, mild = 1 point, moderate = 2 points, severe = 3 points. The sum of all symptom scores constituted the total skin lesion severity score. Higher scores indicated more severe lesions, with a maximum score of 12. Statistical analysis was performed on the data. On day 14 of the experiment, AD-like skin lesion manifestations including erythema, edema, dryness, exudation, and crusting were compared among groups and documented via digital photography.

### Q-PCR Analysis

Total RNA extraction from animal tissues, cDNA synthesis, and PCR amplification were performed as described in the preceding examples. Additional primer information is as follows:

**Table 28. Q-PCR Analysis**

| Primers | | Sequence (5'-3') |
|---|---|---|
| *Tslp* | Forward | CGAGGACTGTGAGAGCAAGCC |
| | Reverse | GCAGCCAGGGATAGGATTGAGAG |
| *Il33* | Forward | GACCAGGTGCTACTACGCTACTATG |
| | Reverse | CAGCCAGATGTCTGTGTCTTTGATG |
| *Il31* | Forward | ACACAGGAACAACGAAGCCTACC |
| | Reverse | ATATTGGGGCACCGAAGGACAAG |
| *Il13* | Forward | TCTTGCTTGCCTTGGTGGTCTC |
| | Reverse | GGGGAGTCTGGTCTTGTGTGATG |
| *Il13* | Forward | GTTGTCATCCTGCTCTTCTTTCTCG |
| | Reverse | CATGGCGTCCCTTCTCCTGTG |

**Experimental results:** Compared to mice in the normal group, mice in the model group exhibited distinct AD-like skin lesions on their ears, including erythema, scaling, crusting, and edema. However, after topical application of 0.01%, 0.1%, and 1% Compound 1 ointment, the aforementioned symptoms were significantly alleviated in the mice.

As shown in Table 29, with prolonged modeling duration, ear thickness in the AD group increased significantly, but the rate of increase was markedly lower in the compound 1-treated group compared to the AD group. On the final day of the experiment, ear thickness in the AD group increased 2.4-fold compared to baseline values, while the inhibition rates for 0.01%, 0.1%, and 1% Compound 1 ointments were 71%, 71%, and 80%, respectively. This indicates that Compound 1 alleviates the severity of ear lesions in mice.

**Table 29. Compound 1 significantly reduced ear thickness in AD mice.**

| Number of days | Normal (mm) | MC903(mm) | **1**(0.01%, mm) | **1**(0.1%, mm) | **1**(1%, mm) | **Dex** (1%, mm) |
|---|---|---|---|---|---|---|
| Day 1 | 0.15±0.00 | 0.14±0.00 | 0.14±0.00 | 0.14±0.00 | 0.15±0.00 | 0.15±0.00 |
| Day 3 | 0.15±0.00 | 0.17±0.01 | 0.17±0.00 | 0.17±0.00 | 0.17±0.00 | 0.18±0.00 |
| Day 5 | 0.15±0.00 | 0.20±0.00 | 0.21±0.00 | 0.21±0.00 | 0.21±0.00 | 0.22±0.00 |
| Day 7 | 0.15±0.00 | 0.22±0.01 | 0.22±0.00 | 0.21±0.00 | 0.22±0.01 | 0.24±0.01 |
| Day 8 | 0.15±0.00 | 0.24±0.01 | 0.23±0.01 | 0.23±0.00 | 0.22±0.01 | 0.24±0.00 |
| Day 9 | 0.16±0.00 | 0.28±0.01 | 0.23±0.01 | 0.23±0.00 | 0.23±0.01 | 0.24±0.01 |
| Day 10 | 0.16±0.00 | 0.29±0.01 | 0.25±0.00 | 0.23±0.00 | 0.23±0.01 | 0.25±0.01 |
| Day 11 | 0.16±0.00 | 0.30±0.01 | 0.26±0.01 | 0.24±0.01 | 0.24±0.00 | 0.26±0.01 |
| Day 12 | 0.16±0.00 | 0.34±0.01 | 0.26±0.01 | 0.24±0.01 | 0.23±0.01 | 0.28±0.00 |
| Day 13 | 0.15±0.00 | 0.36±0.01 | 0.22±0.01 | 0.21±0.01 | 0.20±0.00 | 0.27±0.01 |
| Day 14 | 0.15±0.00 | 0.36±0.01 | 0.21±0.01^{**} | 0.21±0.01^{**} | 0.19±0.00^{**} | 0.26±0.01^{**} |

As shown in Table 30, compared to normal mice, AD mice exhibited dermatitis-like symptoms in their ears, characterized by pronounced erythema, scaling, crusting, and edema. Continuous application of 0.01%, 0.1%, and 1% Compound 1 ointment for 7 days significantly alleviated the dermatitis symptoms in mice, as evidenced by a marked reduction in dermatitis scores.

**Table 30. Compound 1 significantly downregulates ear dermatitis scores in AD mice**

| Group | Dermatitis Score |
|---|---|
| Normal | 0.00±0.00 |
| MC903 | 8.67±0.21 |
| **1** (0.01%) | 3.50±0.22^{**} |
| **1** (0.1%) | 2.67±0.21^{**} |
| **1** (1%) | 2.33±0.33** |
| **Dex** (1%) | 3.50±0.22** |

As shown in Table 31, compared to normal mice, mice in the AD group exhibited significantly increased expression of Th2-related factors Il4, 1113, and Il31 in the ear, as well as inflammatory factors Tslp, Tnf, 1113, and Il1 mRNA secreted by keratinocytes. However, administration of Compound 1 significantly suppressed the expression of these genes.

Compared to the normal group, mice in the model group exhibited distinct AD-like skin lesions on their backs, including erythema, scaling, crusting, and edema. However, after topical application of 0.01%, 0.1%, and 1% Compound 1 ointment, these symptoms were significantly alleviated.

As shown in Table 32, on the final day of the experiment, the dorsal skin thickness of mice in the AD group increased 2.4-fold compared to baseline values. The inhibition rates of 0.01%, 0.1%, and 1% Compound 1 ointment were 66%, 72%, and 83%, respectively. This indicates that Compound 1 can alleviate the severity of dorsal skin lesions in mice.

**Table 31. Compound 1 Inhibits mRNA Expression of Inflammatory Markers in the Ears of AD Mice**

| factor | Normal | MC903 | 1 (0.01%) | 1(0.1%) | **1** (1%) | Dex (1%) |
|---|---|---|---|---|---|---|
| Tnf | 1.00+0.08 | 4.91 ±0.57 | 2.35 ±0.39** | 1.34 ±0.41** | 1.01±0.40** | 1.38 ±0.22** |
| TSLP | 1.00+0.01 | 2.60 ±0.22 | 2.24 ±0.25* | 1.42 ±0.16** | 1.12±0.07** | 1.30 ±0.17** |
| Il1 | 1.00+0.08 | 124.79+28.29 | 19.67+5.26 | 9.89 ±3.44 | 5.69±1.12 | 12.69 ±4.22 |
| Il13 | 1.00+0.06 | 4.56 ±0.51 | 3.30 ±0.47* | 2.85 ±0.48** | 1.56±0.20** | 1.91 ±0.39** |
| Il4 | 1.00+0.08 | 54.18 ±10.54 | 3.62 ±0.97** | 1.95 ±0.66** | 1.17±0.37** | 1.35 ±0.30** |
| Il31 | 1.00+0.04 | 3.25 +0.28 | 2.31 ±0.28** | 1.64 ±0.25** | 1.10±0.19** | 1.86 ±0.44** |

**Table 32. Compound 1 significantly reduced the thickness of the dorsal region in AD mice.**

| Group | Skin thickness |
|---|---|
| Normal | 21.39±0.42 |
| MC903 | 45.78±1.53 |
| **1** (0.01%) | 36.06±0.91** |
| **1** (0.1%) | 33.11±0.31 |
| **1** (1%) | 29.56±0.25** |
| **Dex** (1%) | 30.44±0.62** |

As shown in Table 33, compared to normal mice, AD mice exhibited dermatitis-like symptoms on their backs, characterized by pronounced erythema, scaling, crusting, and edema. Continuous application of 0.01%, 0.1%, and 1% Compound 1 ointment for 7 days significantly alleviated the dermatitis symptoms in mice, as evidenced by a marked reduction in dermatitis scores.

**Table 33. Compound 1 significantly downregulates dermatitis scores on the backs of AD mice**

| Group | Dermatitis Score |
|---|---|
| Normal | 0.00±0.00 |
| MC903 | 8.67±0.21 |
| **1** (0.01%) | 3.67±0.33** |
| **1** (0.1%) | 2.50±0.22** |
| **1** (1%) | 2.17±0.17** |
| **Dex** (1%) | 4.17±0.31** |

### 2. Effects of Oral Administration of Compound 20 on Mouse Atopic Dermatitis

A mouse atopic dermatitis model was established as described in Section 1. Compound 20 (0.1, 1, 10 mg/kg) was administered via oral gavage for 7 consecutive days. Results are shown in Table 34. In normal mice, the skin tissue structure at the ear lesion site remained intact with clear stratification, showing no typical inflammatory responses such as vasodilation. In the MC903 group, the left ear skin tissue exhibited redness and hardening starting on day 7, by day 14, accompanied by epidermal dryness and desquamation. As the experiment progressed and the number of challenges increased, inflammatory symptoms in the skin tissue gradually worsened, manifested by a significant increase in inflammation scores. Oral administration of compound 20 (1, 10 mg/kg) significantly alleviated MC903-induced AD-like lesions in mouse ears and reduced atopic dermatitis scores.

**Table 34. Oral administration of Compound 20 significantly reduced atopic dermatitis scores in mice.**

| Days | Normal | MC903 | **20** (0.1 mg/kg) | **20** (1 mg/kg) | **20** (10 mg/kg) | **Dex** (5 mg/kg) |
|---|---|---|---|---|---|---|
| Day 1 | 0.00±0.00 | 0.00±0.00 | 0.00±0.00 | 0.00±0.00 | 0.00±0.00 | 0.00±0.00 |
| Day 3 | 0.00±0.00 | 0.66±0.21 | 0.67±0.21 | 0.50±0.22 | 0.67±0.21 | 0.67±0.21 |
| Day 5 | 0.00±0.00 | 1.33±0.21 | 1.33±0.21 | 1.17±0.17 | 1.00±0.00 | 1.50±0.22 |
| Day 7 | 0.00±0.00 | 2.17±0.17 | 2.17±0.17 | 1.67±0.21 | 2.00±0.00 | 2.00±0.26 |
| Day 8 | 0.00±0.00 | 2.83±0.31 | 2.50±0.22 | 2.17±0.17 | 2.50±0.22 | 2.83±0.31 |
| Day 9 | 0.00±0.00 | 3.50±0.34 | 3.00±0.26 | 2.83±0.17 | 2.83±0.17 | 3.00±0.00 |
| Day 10 | 0.00±0.00 | 4.00±0.26 | 3.50±0.22 | 3.00±0.00 | 2.67±0.42 | 3.17±0.17 |
| Day 11 | 0.00±0.00 | 4.50±0.22 | 3.67±0.21 | 3.17±0.17 | 3.00±0.26 | 3.50±0.22 |
| Day 12 | 0.00±0.00 | 5.17±0.31 | 3.33±0.21 | 2.67±0.21 | 2.33±0.33 | 3.00±0.26 |
| Day 13 | 0.00±0.00 | 5.66±0.21 | 3.00±0.00 | 2.50±0.22 | 1.67±0.21 | 2.67±0.21 |
| Day 14 | 0.00±0.00 | 5.83±0.40 | 2.67±0.21** | 2.00±0.00** | 1.50±0.22** | 2.00±0.26** |

As shown in Table 35, compared with the normal group mice, the ear tissue thickness of the MC903 group mice was significantly increased. Oral administration of Compound 20 (1, 10 mg/kg) significantly reduced ear thickness in AD mice.

**Table 35. Oral administration of Compound 20 significantly reduced ear thickness in mice with dermatitis.**

| Days | Normal(mm) | MC903(mm) | **20** (0.1 mg/kg; mm) | **20** (1 mg/kg; mm) | **20** (10 mg/kg; mm) | **Dex** (5 mg/kg; mm) |
|---|---|---|---|---|---|---|
| Day 1 | 0.20±0.00 | 0.20±0.00 | 0.20±0.00 | 0.20±0.00 | 0.20±0.00 | 0.20±0.00 |
| Day 3 | 0.19±0.00 | 0.25±0.00 | 0.24±0.00 | 0.23±0.00 | 0.23±0.00 | 0.23±0.00 |
| Day 5 | 0.19±0.00 | 0.26±0.00 | 0.26±0.00 | 0.24±0.00 | 0.25±0.00 | 0.25±0.00 |
| Day 7 | 0.19±0.00 | 0.27±0.00 | 0.26±0.00 | 0.26±0.00 | 0.26±0.00 | 0.26±0.00 |
| Day 8 | 0.19±0.00 | 0.28±0.00 | 0.27±0.00 | 0.27±0.00 | 0.27±0.00 | 0.26±0.00 |
| Day 9 | 0.19±0.00 | 0.30±0.00 | 0.28±0.00 | 0.28±0.00 | 0.27±0.00 | 0.27±0.00 |
| Day 10 | 0.20±0.00 | 0.31±0.00 | 0.29±0.00 | 0.28±0.00 | 0.27±0.00 | 0.28±0.00 |
| Day 11 | 0.19±0.00 | 0.33±0.00 | 0.30±0.00 | 0.28±0.00 | 0.27±0.00 | 0.27±0.00 |
| Day 12 | 0.20±0.00 | 0.34±0.00 | 0.30±0.00 | 0.27±0.00 | 0.25±0.00 | 0.27±0.00 |
| Day 13 | 0.19±0.00 | 0.36±0.00 | 0.30±0.00 | 0.27±0.00 | 0.25±0.00 | 0.27±0.00 |
| Day 14 | 0.20±0.00 | 0.37±0.00 | 0.29±0.00** | 0.27±0.00** | 0.24±0.01** | 0.26±0.00** |

As shown in Table 36, serum IgE levels were significantly elevated in the MC903 group mice, while oral administration of Compound 20 (1, 10 mg/kg) reduced IgE levels in a concentration-dependent manner. Finally, compared with the Normal group, the MC903 group exhibited markedly increased mRNA expression of inflammatory factors Tslp and Il6, as well as Th2-associated factors Il4, Il13, and Il31, in ear skin keratinocytes. However, oral administration of compound 20 (1, 10 mg/kg) significantly suppressed the expression of these genes.

**Table 36. Compound 20 Inhibits mRNA Expression of Inflammatory Markers in the Ears of AD Mice**

| Factor | Normal | MC903 | **20**(0.1 mg/kg) | **20** (1 mg/kg) | **20** (10 mg/kg) | **Dex** (5 mg/kg) |
|---|---|---|---|---|---|---|
| IgE (ng/mL) | 80.44±18.50 | 488.91±122.58 | 239.26+48.93 | 167.90±21.03** | 145.68±23.19** | 191.65±39.26* |
| Tnf | 1.00+0.03 | 4.56+0.51 | 4.04±0.50** | 2.63±0.55** | 1.22±0.18** | 2.30±0.36** |
| TSLP | 1.00+0.07 | 4.39±0.50 | 3.38+0.51 | 2.36±0.32** | 1.33±0.13** | 2.13±0.26** |
| Il13 | 1.00+0.05 | 2.80+0.21 | 2.04+0.20 | 1.82±0.21* | 1.66±0.30** | 1.89±0.20* |
| Il4 | 1.00+0.02 | 4.23+0.39 | 2.77±0.16** | 2.65±0.22** | 2.09±0.26** | 2.59±0.38** |
| Il31 | 1.00+0.03 | 3.53+0.35 | 2.64±0.22* | 2.27±0.13** | 1.68±0.13** | 2.06±0.20** |

### Example 11

### Skin Soothing Function Test of Compound 1

### (1) Testing Hyaluronic Acid and Hyaluronidase Levels in Human Keratinocytes

Human keratinocytes (Hacat) were cultured routinely in DMEM medium containing 10% FBS within a 5% CO₂ incubator. Upon reaching 70-80% confluence, cells were uniformly seeded at a density of 1×10⁵ cells/mL into 6-well plates (2 mL per well), divided into Normal, UVB, Compound 1 (0.01, 0.1, 1, 10 µM) groups. After irradiation at a dose of 90 mJ/cm², the respective concentrations of Compound 1 were added. Following incubation for 24 hours, hyaluronic acid and hyaluronidase levels were measured using a kit-based assay.

As shown in Table 37, compared to the Normal group, UVB irradiation significantly decreased hyaluronic acid levels while markedly increasing hyaluronidase levels. After 24-hour treatment with Compound 1 (0.1, 1, 10 µM), hyaluronic acid levels were significantly restored, and hyaluronidase levels were markedly reduced. This suggests that Compound 1 exerts certain soothing effects by upregulating hyaluronic acid levels.

**Table 37. Compound 1 Downregulates Hyaluronidase and Upregulates Hyaluronic Acid Levels in Human Keratinocytes**

| Group | Hyaluronidase (ng/mL) | Hyaluronic acid (ng/mL) |
|---|---|---|
| Normal | 13.98±1.28 | 31.06±1.87 |
| UVB | 138.37±9.81 | 6.92±0.70 |
| **1** (0.01 µM) | 124.41±16.49* | 8.73±1.62 |
| **1** (0.1 µM) | 88.07±10.17** | 14.17±1.30** |
| **1** (1 µM) | 56.03±5.53** | 19.51±1.87** |
| **1** (10 µM) | 37.73±11.49** | 24.82±1.13** |
| **Dex** (1 µM) | 87.14±9.63* | 14.23±1.51* |

### (2) Testing for Mast Cell Degranulation

Rat mast cells (RBL-2H3) were cultured routinely in DMEM medium containing 10% FBS within a 5% CO₂ incubator. Upon reaching 70-80% confluence, cells were uniformly seeded at a density of 1 × 10⁵ cells/mL into 6-well plates (2 mL per well), divided into the following groups: Normal, IgE+HSA, Compound 1 (0.01, 0.1, 1, 10 µM). Mast cells were sensitized with 500 µg/L anti-DNP IgE for 12 h. washed with PBS, and cultured for an additional 12 h with 100 µg/L DNP-HSA. Compound 1 at the specified concentrations was then added. After 4 h incubation, β-galactosidase levels were measured using a commercial assay kit.

As shown in Table 38, compared to the Normal group, antigen-antibody stimulation significantly enhanced mast cell degranulation, manifested as a marked increase in β-hexosaminidase levels. After 4 hours of treatment with Compound 1 (0.1, 1, 10 µM), β-hexosaminidase levels decreased significantly. This suggests that Compound 1 exerts a certain soothing effect by inhibiting mast cell degranulation.

**Table 38. Compound 1 significantly inhibits mast cell degranulation.**

| Group | β-Glucosaminidase (ng/mL) |
|---|---|
| Normal | 3.74±0.06 |
| IgE+HSA | 17.27±0.00 |
| **1** (0.01 µM) | 13.24±0.08** |
| **1** (0.1 µM) | 12.08±0.12** |
| **1** (1 µM) | 10.65±0.05** |
| **1** (10 µM) | 7.13±0.07** |
| **Dex** (1 µM) | 9.78±0.01** |

**Example 12:** Experiment on Inhibitory Effect of Compounds on MDSCs and Their Promotion of T Cell Proliferation

Mice were euthanized by cervical dislocation and immersed in 75% ethanol solution for 5 min. Under sterile conditions, the tibia was removed, both ends of the bone were cut, and bone marrow was repeatedly flushed into a 15 mL centrifuge tube using a 1 mL syringe filled with culture medium. Centrifuge at 1600 rpm for 5 min to collect bone marrow cells. Wash twice with 1×PBS, then resuspend in RPMI 1640 complete medium containing cytokines GM-CSF (10 ng/mL) and IL-4 (10 ng/mL) in a cell culture dish to stimulate differentiation of bone marrow cells into MDSCs. Subsequently, treat MDSCs with DMSO (negative control) or 1 µM Compounds in table 39 for 12 hours. Simultaneously, use forceps to separate the connective tissue beneath the spleen, remove the spleen, and place it in a sterile culture dish containing 5 mL RPMI 1640 medium. Position the spleen on a 200-mesh cell strainer and gently crush it using the needle tip of a 10 mL syringe. simultaneously aspirating a small volume of RPMI 1640 medium to rinse the cell strainer. The spleen cell suspension was then transferred into a 15 mL centrifuge tube. Centrifuge at 1600 rpm for 5 min to collect T cells. Wash twice with 1×PBS. Resuspend the centrifuged T cells in 1 mL PBS and add 1 mL of 10 µM CFDA-SE solution. Incubate at room temperature for 8 min. After incubation, add 10 mL of complete RPMI 1640 medium to terminate CFDA-SE staining. Centrifuge at 1600 rpm for 5 min, wash twice with 1×PBS, resuspend cells in RPMI 1640 complete medium containing concanavalin A (5 µg/mL), and culture in a cell culture dish at 37 °C, 5% CO₂ for 12 h to stimulate T cell activation. After drug administration, collect MDSC cells into centrifuge tubes. Centrifuge at 1600 rpm for 5 min, remove supernatant, resuspend in 1 mL fresh medium, and plate. Add 1 mL stimulated activated T cells (1:1 ratio) for 48 h co-culture. After incubation, collect cells into centrifuge tubes, wash twice with PBS, and incubate with Fc antibody at room temperature in the dark for 10 min. Add CD3-labeled flow cytometry antibody and incubate at 4 °C in the dark for 30 min. After incubation, wash cells with PBS, resuspend in 300 µL PBS, and analyze by flow cytometry.

As shown in Table 39, in the MDSC-T cell co-culture model, all compounds promoted T cell proliferation to varying degrees, suggesting that this class of compounds exhibits potent antitumor activity in tumor models with T lymphocyte infiltration (such as hepatocellular carcinoma, colorectal cancer, and lymphoma). Compounds 78, 83, and 1 significantly enhanced T-cell proliferation, with results comparable to the positive control Arg.

**Table 39. Effects of Compounds on T Cell Proliferation in a Co-culture Model of MDSCs and T Cells**

| compound | T-cell proliferation rate (%) | compound | T-cell proliferation rate (%) |
|---|---|---|---|
| **64** | +++ | **47** | ++ |
| **65** | + | **48** | ++ |
| **66** | + | **49** | +++ |
| **67** | ++ | **50** | ++ |
| **68** | + | **51** | +++ |
| **69** | ++ | **2** | ++ |
| **70** | + | **52** | + |
| **71** | +++ | **53** | + |
| **72** | ++ | **54** | + |
| **73** | ++ | **55** | ++ |
| **74** | +++ | **56** | + |
| **75** | ++ | **57** | + |
| **76** | ++ | **5** | ++ |
| **77** | + | **3** | ++ |
| **78** | +++ | **6** | + |
| **79** | ++ | **7** | + |
| **80** | ++ | **43** | + |
| **81** | ++ | **44** | + |
| **82** | + | **4** | ++ |
| **83** | +++ | **96** | + |
| **84** | ++ | **97** | + |
| **85** | + | **20** | + |
| **86** | ++ | **21** | + |
| **87** | +++ | **58** | + |
| **88** | ++ | **59** | + |
| **89** | ++ | **60** | + |
| **90** | ++ | **61** | + |
| **91** | ++ | **62** | + |
| **92** | ++ | **63** | + |
| **93** | ++ | **98** | + |
| **94** | ++ | **99** | + |
| **95** | ++ | **100** | + |
| **1** | +++ | **101** | + |
| **45** | ++ | **102** | + |
| **46** | ++ | **23** | + |
| **Arg** | +++ | **MCL** | ++ |

| | | | |
|---|---|---|---|
| Note: "+" indicates T-cell proliferation rate within the 0-20% range compared to the control group; "++" indicates T-cell proliferation rate within the 20-40% range compared to the control group; "+++" indicates T-cell proliferation rate >40% compared to the control group. | | | |

### Example 13

### Inhibition of MDSC Chemotaxis by Compounds 78, 83, and 1

Hepa1-6 mouse hepatocellular carcinoma cells were uniformly seeded into 6-well plates. After cells reached confluence, supernatant was aspirated, and 1-1.5 mL fresh medium was added to each well. Following 24 h culture, supernatant was collected, centrifuged at 1600 rpm for 5 min, aspirated, and filtered through a 0.22 µm membrane to obtain tumor-conditioned medium for subsequent use. Euthanize mice by cervical dislocation and immerse in 75% ethanol solution for 5 min. Under sterile conditions, remove the tibia, cut both ends of the bone, and aspirate the bone marrow into a 1 mL syringe. Repeat flushing into a 15 mL centrifuge tube. Centrifuge at 1600 rpm for 5 min to collect bone marrow cells. Wash twice with 1×PBS. Resuspend cells in complete RPMI 1640 medium containing cytokines GM-CSF (10 ng/mL) and IL-4 (10 ng/mL) to stimulate differentiation into MDSCs. Count cells, adjust density to 1×10⁶ cells/mL, and uniformly seed into 24-well plates at 300 µL per well. Treat MDSCs with DMSO (negative control), 1 µM, 10 µM Arg, 78, 83, 1 for 12 h. Centrifuge at 1600 rpm for 5 min, remove supernatant, resuspend cells in 300 µL fresh medium, and place into Transwell chambers. Add 600 µL normal medium (control group) or tumor-conditioned medium (model group) to the lower chamber. Co-culture for 24 h. After incubation, remove the chamber and count cells migrating into the lower chamber to investigate the chemotactic inhibitory effects of Arg, 78, 83, and 1 on MDSCs.

As shown in Table 40, in the co-culture model of MDSCs with tumor-conditioned medium, compounds 78, 83, and 1 significantly inhibited MDSC chemotaxis compared to the control group, exhibiting a concentration-dependent effect. At a concentration of 10 µM, compounds 83 and 1 demonstrated superior inhibition rates against MDSC chemotaxis compared to the positive control compound Arg.

**Table 40. Inhibitory Effects of Arg, 78, 83, and 1 on MDSC Chemotaxis**

| compound | Suppression Rate (%) | compound | Suppression Rate (%) |
|---|---|---|---|
| **78** (1 µM) | 29.0% | **1** (1 µM) | 42.3% |
| **78**(10 µM) | 67.1% | **1** (10 µM) | 83.3% |
| **83** (1 µM) | 29.4% | **Arg** (1 µM) | 42.5% |
| **83** (10 µM) | 75.9% | **Arg** (10 µM) | 73.2% |

### Example 14

### Compounds 78, 83, and 1 Downregulate CCR2 mRNA Levels

Mice were euthanized by cervical dislocation and immersed in 75% ethanol solution for 5 min. Under sterile conditions, the tibia was removed, both ends of the bone were cut, and bone marrow was repeatedly flushed into a 15 mL centrifuge tube using a 1 mL syringe filled with culture medium. Centrifuge at 1600 rpm for 5 min to collect bone marrow cells. Wash twice with 1×PBS, then resuspend in RPMI 1640 complete medium containing GM-CSF (10 ng/mL) + IL-4 (10 ng/mL) and transfer to a cell culture dish to stimulate differentiation of bone marrow cells into MDSCs. Count cells and adjust density to 1 × 10⁶ cells/mL in complete medium. Seed cells uniformly into 6-well plates at 2 mL per well. Subsequently, treat MDSCs for 2 h with DMSO (negative control), 1 µM, 10 µM Arg, 78, 83, or 1. After incubation, collect cells into centrifuge tubes, centrifuge at 1600 rpm for 5 min, wash once with 1×PBS, and add 500 µL RNA extraction reagent to each tube. After centrifugation to obtain the pellet, add 20 µL DEPC-treated water to each tube to dissolve the pellet. Determine RNA concentration and reverse transcribe to cDNA. Add the cDNA, primers, and reverse transcriptase to a 96-well plate. Centrifuge at 4000 rpm for 5 min, then place in a PCR instrument for detection. Measure the inhibitory effects of Arg, 78, 83, and 1 on CCR2 mRNA expression.

As shown in Table 41, compounds Arg, 78, 83, and 1 can all downregulate CCR2 mRNA levels in a concentration-dependent manner.

**Table 41. Compounds 78, 83, and 1 Inhibit CCR2 mRNA Levels**

| compound | Tumor Suppression Rate (%) | compound | Tumor Suppression Rate (%) |
|---|---|---|---|
| **78** (1 µM) | 23.0% | **1** (1 µM) | 35.2% |
| **78** (10 µM) | 52.7% | **1** (10 µM) | 76.5% |
| **83** (1 µM) | 17.0% | **Arg** (1 µM) | 22.3% |
| **83** (10 µM) | 70.0% | **Arg** (10 µM) | 69.7% |

### Example 15

### Antitumor Effects of Compounds 20, 99, and 102 in a Hepa1-6 Mouse Hepatocellular Carcinoma Subcutaneous Xenograft Model

Hepa1-6 cells were cultured and expanded in vitro. An appropriate amount of cells in the logarithmic growth phase was resuspended in a mixture of serum-free DMEM medium and Matrigel (1:1). under aseptic conditions to prepare a 2.5×10⁶/100 µL cell suspension. A 100 µL aliquot of this suspension was injected subcutaneously into the left forearm axilla of male C57BL/6 mice. When the tumor volume grew to 100-200 mm³, animals with appropriately sized tumors were selected and randomly divided into groups, with 6 animals per group.They are the solvent control group, the Arg group at 40 µmol/kg/day, Compound 20 group, Compound 99 group, and Compound 102 group, respectively. Daily oral administration was performed for 2 weeks. During the treatment period, mouse body weight and tumor diameter were measured daily. At the end of the experiment, mice were euthanized by cervical dislocation, and tumors were removed for weighing. Tumor volume (TV) was calculated using the formula: TV = 1/2 × a × b², where a represents the longest diameter of the tumor and b represents the shortest diameter.

As shown in Table 42, in the Hepa1-6 mouse hepatocellular carcinoma subcutaneous xenograft model, continuous administration for 2 weeks demonstrated significant tumor-inhibiting effects for Arg, 20, 99, and 102.

**Table 42. Antitumor Effects of Compounds 20, 99, and 102 in the Hepa1-6 Mouse Hepatocellular Carcinoma Xenograft Model**

| Group | Dose (µmol/kg/d) | Number of Animals Start/End | Tumor Weight (g) | Tumor Inhibition Rate (%) |
|---|---|---|---|---|
| Solvent Reference | - | 6/6 | 0.92±1.41 | - |
| **20** | 40 | 6/6 | 0.03±0.02 | 96.78% |
| **99** | 40 | 6/6 | 0.18±0.40 | 80.05% |
| **102** | 40 | 6/6 | 0.03±0.04 | 96.67% |
| **MCL** | 40 | 6/6 | 0.27±0.24 | 70.52% |
| **Arg** | 40 | 6/6 | 0.04±0.03 | 95.86% |

| | | | | |
|---|---|---|---|---|
| *, p < 0.05; **, p <0.01; ***, p < 0.001 (Compared with the solvent control). | | | | |

### Example 16

### Antitumor Effects of Compounds 20, 99, and 102 in the H22 Mouse Hepatocellular Carcinoma Subcutaneous Xenograft Model

H22 cells were cultured and expanded in vitro. An appropriate amount of cells in the logarithmic growth phase was resuspended in a mixture of serum-free RPMI 1640 medium and Matrigel (1:1). under aseptic conditions to prepare a 3×10⁵/100 µL cell suspension. A 100 µL aliquot of this suspension was injected subcutaneously into the left forelimb axilla of male BALB/c mice. When the tumor volume grew to 100-200 mm³, animals with appropriately sized tumors were selected and randomly divided into groups, with 6 animals per group.They are the solvent control group, the Arg group at 40 µmol/kg/day, Compound 20 group, Compound 99 group, and Compound 102 group, respectively. Daily oral administration was performed for 2 weeks. During the treatment period, mouse body weight and tumor diameter were measured daily. At the end of the experiment, mice were euthanized by cervical dislocation, and tumors were removed for weighing. Tumor volume (TV) was calculated using the formula: TV = 1/2 × a × b², where a represents the longest diameter of the tumor and b represents the shortest diameter.

Mice bearing tumors were euthanized by cervical dislocation and placed in a beaker containing 75% ethanol. Tumors were dissected from the mice and initially immersed in a 100 mm³ culture dish containing RPMI 1640 medium. Once all tumors were dissected, nail-sized tumor pieces were transferred to 1.5 mL Eppendorf tubes and minced with scissors. Transfer the minced tumor tissue suspension to a 15 mL centrifuge tube containing 3-5 mL of digestive enzymes (Collagenase IV 1 mg/mL, Hyaluronidase 1 mg/mL, DNase I 20 U/mL). Incubate at 200 rpm and 37°C for 1 hour. After digestion, pass the mixture through a 200-mesh sieve to obtain the tumor cell suspension. Centrifuge the resulting tumor suspension at 1600 rpm for 5 min, aspirate the supernatant, resuspend cells in 1 mL of 1 ×PBS per tube. Transfer the cell suspension to a 1.5 mL Eppendorf tube, centrifuge at 1600 rpm for 5 min, remove the supernatant, resuspend cells in 100 µL of 1×PBS per tube, add 1 µL of dead/live dye to each tube, mix thoroughly, and incubate at room temperature in the dark for 15 min. Simultaneously, remove the spleen with forceps and place it in a sterile Petri dish containing 5 mL RPMI 1640 medium. Position the spleen on a 200-mesh cell strainer. Gently press the spleen using a 10 mL syringe needle. Aspirate a small amount of RPMI 1640 medium to rinse the cell strainer. Transfer the washed splenocyte suspension into a 15 mL centrifuge tube. Centrifuge at 1600 rpm for 5 min to collect cells. Wash once with 1×PBS. Add 3 mL red blood cell lysis buffer (diluted with ddH₂O) to each tube and incubate for 5 min. After lysis, add 10 mL 1×PBS to each tube to terminate lysis. Centrifuge at 1600 rpm for 5 min. Add 1 mL 1×PBS to each 15 mL centrifuge tube, transfer to 1.5 mL EP tubes, and centrifuge at 1600 rpm for 5 min. Add 1 mL 1×PBS to each tube containing stained tumor cells and lysed spleen cells for one wash, centrifuge at 1600 rpm for 5 min, and remove the supernatant. Resuspend tumor cells and spleen cells separately in 100 µL 1×PBS. Add 1 µL FcX^{™} antibody to each tube and incubate at room temperature in the dark for 10 min. After incubation, sequentially add 1 µL each of CD45, CD11b, Gr1 antibodies. Incubate at 4°C in the dark for 30 min. After incubation, wash once with 1×PBS, discard the supernatant, resuspend cells in 300 µL 1×PBS, filter the membrane into a flow cytometry tube, and proceed to instrument analysis.

Mice bearing tumors were euthanized by cervical dislocation and placed in a beaker containing 75% ethanol. Tumors were dissected from the mice and initially immersed in a 100 mm³ culture dish containing RPMI 1640 medium. Once all tumors were dissected, nail-sized tumor pieces were transferred to 1.5 mL Eppendorf tubes and minced with scissors. Transfer the minced tumor tissue suspension to a 15 mL centrifuge tube containing 3-5 mL of digestive enzymes (Collagenase IV 1 mg/mL, Hyaluronidase 1 mg/mL, DNase I 20 U/mL). Incubate at 37°C on a shaking incubator at 200 rpm for 1 hour. After digestion, pass the mixture through a 200-mesh sieve to obtain the tumor cell suspension. Centrifuge the resulting tumor suspension at 1600 rpm for 5 min, aspirate the supernatant, resuspend cells in 1 mL of 1×PBS per tube. Transfer the cell suspension to a 1.5 mL Eppendorf tube, centrifuge at 1600 rpm for 5 min, remove the supernatant, resuspend cells in 100 µL of 1×PBS per tube, add 1 µL of dead/live dye to each tube, mix thoroughly, and incubate at room temperature in the dark for 15 min. Simultaneously, remove the spleen with forceps and place it in a sterile culture dish containing 5 mL RPMI 1640 medium. Position the spleen on a 200-mesh cell strainer. Gently press the spleen using a 10 mL syringe needle. Aspirate a small amount of RPMI 1640 medium to rinse the cell strainer. Transfer the splenocyte suspension into a 15 mL centrifuge tube. Centrifuge at 1600 rpm for 5 min to collect cells. Wash once with 1×PBS. Add 3 mL red blood cell lysis buffer (diluted with ddH₂O) to each tube and incubate for 5 min. After lysis, add 10 mL 1×PBS to each tube to terminate lysis. Centrifuge at 1600 rpm for 5 min. Add 1 mL 1×PBS to each 15 mL centrifuge tube, transfer to 1.5 mL EP tubes, and centrifuge at 1600 rpm for 5 min. Add 1×PBS to each tube containing stained tumor cells and lysed spleen cells for one wash, centrifuge at 1600 rpm for 5 min, and remove the supernatant. Resuspend tumor cells and spleen cells separately in 100 µL 1×PBS. Add 1 µL FcX^{™} antibody to each tube and incubate at room temperature in the dark for 10 min. After incubation, sequentially add 1 µL each of CD45, CD3, and CD8 antibodies to each tube. Incubate at 4°C in the dark for 30 min. After staining, wash once with 1 mL PBS. Centrifuge at 1600 rpm for 5 min. Discard the supernatant. Resuspend cells in 300 µL 1×PBS. Filter the cell suspension into a flow cytometry tube and proceed to instrument analysis.

As shown in Table 43, in the H22 mouse hepatocellular carcinoma subcutaneous xenograft model, Arg, 20, 99, and 102 all exhibited tumor-inhibiting effects following 2 weeks of continuous administration.

**Table 43. Antitumor Effects of Compounds 20, 99, and 102 in the H22 Mouse Hepatocellular Carcinoma Xenograft Model**

| Group | Dose (µmol/kg/day) | Number of Animals | Tumor weight (g) | Tumor Suppression Rate |
|---|---|---|---|---|
| | | Start/End | | (%) |
| Solvent Control | | 6/6 | 8.56± 1.72 | |
| **20** | 40 | 6/6 | 4.88+0.87** | 70.86% |
| **99** | 40 | 6/6 | 6.40±1.59 | 25.18% |
| **102** | 40 | 6/6 | 6.13±1.48* | 27.99% |
| **Arg** | 40 | 6/6 | 5.18± 0.61** | 39.43% |

| | | | | |
|---|---|---|---|---|
| *, p < 0.05; **, p <0.01; ***, p < 0.001 (Compared with solvent control). | | | | |

As shown in Table 44, after the experiment, flow cytometry was used to detect the proportion of MDSCs in tumor tissues and spleens of the H22 mouse subcutaneous xenograft model. Compared with the solvent control group, the proportions of MDSCs in tumor tissues and spleens of the Arg, 20, 99, and 102 administration groups were significantly reduced.

**Table 40. Inhibitory Effects of Compounds 20, 99, and 102 on MDSCs in the H22 Mouse Hepatocellular Carcinoma Xenograft Model**

| Group | | Dose (µmol/kg/day) | Tumor-associated MDSCs (%) | Spleen MDSCs (%) |
|---|---|---|---|---|
| Blank group | | - | - | 6.06± 0.94 |
| Solvent Control | | - | 52.9±5.11 | 48.99± 9.30*** |
| | **Arg** | 40 | 27.0±7.93*** | 27.89±4.72** |
| | **20** | 40 | 7.86±6.19*** | 23.10±5.35*** |
| | **99** | 40 | 34.85±7.99*** | 30.90±10.21* |
| | **102** | 40 | 32.11±6.33*** | 30.37±9.84* |

| | | | | |
|---|---|---|---|---|
| *, p < 0.05; **, p <0.01; ***, p < 0.001 (Compared with solvent control). | | | | |

As shown in Table 45, after the experiment, flow cytometry was employed to detect the proportion of T cells in tumor tissues and spleens of the H22 mouse subcutaneous xenograft model. Compared with the solvent control group, the proportions of T cells in tumor tissues and spleens of the Arg, 20, 99, and 102 administration groups were significantly increased.

**Table 45. Effects of Compounds 20, 99, and 102 on T Cells in H22 Mouse Hepatocellular Carcinoma Transplant Models**

| Group | | Dose (µmol/kg/day) | Tumor T cells(%) | Spleen T cells (%) |
|---|---|---|---|---|
| Blank group | | - | - | 18.22± 0.96 |
| Solvent Control | | - | 5.17±4.94 | 22.30± 3.81 |
| | **Arg** | 40 | 17.72±6.12** | 27.02±4.07 |
| | **20** | 40 | 29.44±6.07*** | 37.19±6.99** |
| | **99** | 40 | 22.81±5.33*** | 31.71±2.95* |
| | **102** | 40 | 20.86±4.15*** | 31.69±6.44* |

| | | | | |
|---|---|---|---|---|
| *, p < 0.05; **, p <0.01; ***, p < 0.001 (Compared with solvent control) | | | | |

### Example 17

### Antitumor Effects of Arg and Compound 20 in a Mouse Xenograft Model of CT26 Colon Cancer Cells

CT26 cells were cultured and expanded in vitro. An appropriate amount of cells in the logarithmic growth phase was resuspended in serum-free RPMI 1640 medium. Under aseptic conditions, a 5×10⁵/100 µL cell suspension was prepared. Using a syringe, 100 µL of the cell suspension was inoculated subcutaneously into the left forelimb axilla of male BALB/c mice. When the tumor volume grew to 50-70 mm³, animals with appropriately sized tumors were selected and randomly divided into groups, with 5 animals in each group. The groups were as follows: solvent control group, Arg group at 80 µmol/kg/day, and compound 20 group. Daily oral administration was performed for 3 weeks. During the treatment period, mouse body weight and tumor diameter were measured daily. At the end of the experiment, mice were euthanized by cervical dislocation, and tumors were removed and weighed. Tumor volume (TV) was calculated using the formula: TV = 1/2 × a × b², where a represents the longest diameter of the tumor and b represents the shortest diameter.

As shown in Table 46, in the subcutaneous tumor model of CT26 mice, both Arg and Compound 20 exhibited tumor-inhibiting effects following three consecutive weeks of administration.

**Table 46. Antitumor Effects of Arg and Compound 20 in the CT26 Mouse Xenograft Tumor Model**

| Group | | Dose (µmol/kg/day) | Number of Animals Start/End | Tumor weight (g) | Tumor inhibition rate (%) |
|---|---|---|---|---|---|
| Solvent Control | | - | 5/5 | 8.15±2.73 | - |
| | **Arg** | 80 | 5/5 | 5.31±0.51 | 34.94% |
| | **20** | 80 | 5/5 | 4.62±1.29* | 43.36%* |

### Example 18

### Antitumor Effects of Compounds 20, 99, and 102 in a 4T1 Mouse Mammary Carcinoma Xenograft Model

4T1 cells were cultured and expanded in vitro. An appropriate amount of cells in the logarithmic growth phase was resuspended in a mixture of serum-free RPMI 1640 medium and Matrigel (1:1). under aseptic conditions to prepare a 4×10⁵/100 µL cell suspension. A 100 µL aliquot of this suspension was injected subcutaneously into the left forelimb axilla of female BALB/c mice. When the tumor volume reached 100-200 mm³, animals with appropriately sized tumors were selected and randomly divided into groups, with 5 animals in each group. The groups were as follows: solvent control group, Arg group at 40 µmol/kg/day, compound 20 group, group 99, and group 102. Daily oral administration was performed for 3 weeks. During the treatment period, mouse body weight and tumor diameter were measured daily. At the end of the experiment, mice were euthanized by cervical dislocation, and tumors were removed for weighing. Tumor volume (TV) was calculated using the formula: TV = 1/2 × a × b², where a represents the longest diameter of the tumor and b represents the shortest diameter.

As shown in Table 47, in the 4T1 mouse breast cancer subcutaneous xenograft model, continuous administration for 3 weeks revealed no significant antitumor effects for Arg, 20, 99, or 102.

**Table 47. Antitumor Effects of Compounds 20, 99, and 102 in the 4T1 Mouse Breast Cancer Xenograft Model**

| Group | | Dose (µmol/kg/day) | Number of Animals Start/End | Tumor weight (g) | Tumor inhibition rate (%) |
|---|---|---|---|---|---|
| Solvent Control | | - | 5/5 | 1.81±0.16 | - |
| | **20** | 40 | 5/5 | 2.09±0.60 | -15.24% |
| | **99** | 40 | 5/5 | 1.43±0.19* | 20.94% |
| | **102** | 40 | 5/5 | 2.18±1.34 | -20.37% |
| | **Arg** | 40 | 5/5 | 1.58±0.21 | 12.87% |

| | | | | | |
|---|---|---|---|---|---|
| *, p < 0.05; **, p <0.01; ***, p < 0.001 (Compared with solvent control)_{∘} | | | | | |

### Example 19

### Anti-tumor Efficacy of Compound 20 Combined with Anti-PD-L1 Monoclonal Antibody in a Hepa1-6 Mouse Hepatocellular Carcinoma Subcutaneous Xenograft Model

Hepa1-6 cells were cultured and expanded in vitro. An appropriate amount of cells in the logarithmic growth phase was resuspended in a mixture of serum-free DMEM medium and Matrigel (1:1). Under sterile conditions, prepare a cell suspension at 2.5 × 10⁶ cells/100 µL. Using a syringe, inject 100 µL of the cell suspension subcutaneously into the left forelimb axilla of male C57BL/6 mice. When tumor volume reached 100-200 mm³, animals with tumors of appropriate size were randomly assigned to four groups (n=6 each): solvent control, compound 20 (1 mg/kg/day), anti-PD-L1 monoclonal antibody (5 mg/kg), and compound 20 combined with anti-PD-L1 monoclonal antibody. Compound 20 was administered via intraperitoneal injection daily, while anti-PD-L1 monoclonal antibody was administered intraperitoneally every three days for a total of two weeks. During the treatment period, mouse body weight and tumor diameter were measured daily. At the end of the experiment, mice were euthanized by cervical dislocation, and tumors were removed and weighed. Tumor volume (TV) was calculated using the formula: TV = 1/2 × a × b², where a represents the longest diameter of the tumor and b represents the shortest diameter.

In the Hepa1-6 mouse subcutaneous liver cancer xenograft model, the combination therapy group demonstrated a significantly higher tumor suppression rate than either compound 20 or the anti-PD-L1 monoclonal antibody alone after two weeks of continuous administration, indicating a synergistic effect (Table 48).

**Table 48. Antitumor Effects of Compound 20 Combined with Anti-PD-L1 Monoclonal Antibody in the Hepa1-6 Mouse Liver Cancer Xenograft Model**

| Group | Dose (µmol/kg/day) - | Number of Animals Start/End | Tumor weight (g) | Tumor inhibition rate (%) |
|---|---|---|---|---|
| Solvent Control | | 6/6 | 0.43± 0.14 | - |
| **20** | **1** | 6/6 | 0.12± 0.09** | 72.37% |
| Anti-PD-L1 monoclonal antibody | 5 | 6/6 | 0.08±0.05*** | 79.16% |
| Joint Group | 1+5 | 6/6 | 0.01±0.01*** | 96.99% |

| | | | | |
|---|---|---|---|---|
| *, p < 0.05; **, p <0.01; ***, p < 0.001 (Compared with solvent control)_{∘} | | | | |

### Example 20

### Antitumor Efficacy of Compound 20 Combined with Anti-PD-L1 Monoclonal Antibody in a Subcutaneous Transplanted Liver Cancer Model in H22 Mice

H22 cells were cultured and expanded in vitro. An appropriate amount of cells in the logarithmic growth phase was resuspended in a mixture of serum-free RPMI 1640 medium and Matrigel (1:1). Under aseptic conditions, prepare a 2×10⁶/100 µL cell suspension. Using a syringe, inject 100 µL of the cell suspension subcutaneously into the left forelimb axilla of male Balb/c mice. When tumor volume reached 100-200 mm³, animals with tumors of appropriate size were randomly assigned to four groups (n=5 each): solvent control, compound 20 monotherapy (2.5 mg/kg/day), anti-PD-L1 monoclonal antibody monotherapy (5 mg/kg), and compound 20 combined with anti-PD-L1 monoclonal antibody. Compound 57 was administered daily via intraperitoneal injection, while anti-PD-L1 monoclonal antibody was administered every three days via intraperitoneal injection for a 2-week treatment period. During the treatment period, mouse body weight and tumor diameter were measured daily. At the end of the experiment, mice were euthanized by cervical dislocation, and tumors were removed and weighed. Tumor volume (TV) was calculated using the formula: TV = 1/2 × a × b², where a represents the longest diameter of the tumor and b represents the shortest diameter.

In the H22 mouse hepatocellular carcinoma subcutaneous xenograft model, the combination therapy group demonstrated a significantly higher tumor suppression rate than either compound 20 or the anti-PD-L1 monoclonal antibody alone after two weeks of continuous administration, indicating a synergistic effect (Table 49).

**Table 49. Antitumor Effects of Compound 20 Combined with Anti-PD-L1 Monoclonal Antibody in the H22 Mouse Hepatocellular Carcinoma Xenograft Model**

| Group | Dose (µmol/kg/day) | Number of Animals Start/End | Tumor weight (g) | Tumor inhibition rate (%) |
|---|---|---|---|---|
| Solvent Control | - | 5/5 | 1.24± 0.24 | - |
| **20** | 2.5 | 5/5 | 0.64± 0.09* | 48.67% |
| Anti-PD-L1 monoclonal antibody | 5 | 5/5 | 0.67±0.17* | 45.65% |
| Joint Group | 2.5+5 | 5/5 | 0.30+0.32* | 75.73% |

| | | | | |
|---|---|---|---|---|
| *, p < 0.05; **, p <0.01; ***, p < 0.001 (Compared with solvent control)_{∘} | | | | |

### Example 21

### Safety Evaluation of Compound 20

1. Experimental Reagents: Compound 20 (purity > 98%), prepared according to Example 1 above; Sodium carboxymethyl cellulose (CMC-Na), China National Pharmaceutical Group Chemical Reagent Co., Ltd.; All other reagents were of analytical grade.
2. Experimental Methods
2.1 Mouse Grouping and Administration: Female and male C57BL/6 mice weighing 18-22 g were randomly divided into three groups based on body weight to evaluate the toxic effects of Compound 20: Normal group, Compound 20 (500, 1000 mg/kg) group, with 10 mice per group (equal numbers of males and females). Compound 20 was administered via oral gavage once daily for 30 consecutive days at a volume of 0.1 mL/10 g body weight. The Normal group received an equal volume of solvent (saline).
2.2 Specimen Collection: Following the final drug administration, blood was collected from the retinal venous plexus. Samples were allowed to stand at room temperature for 2 hours, then centrifuged at 3500 rpm and 4°C for 15-20 minutes. The supernatant serum was carefully aspirated, aliquoted, and stored at -80 °C for future use. Mice were euthanized by cervical dislocation after chloroform hydrate anesthesia. Carefully incise the abdominal skin, secure with pins, then dissect the abdominal and thoracic cavities. Retrieve the liver and kidneys with forceps, rinse repeatedly in prechilled PBS, and place small tissue sections in 10% neutral formalin (4% formaldehyde solution) for fixation. Store remaining tissue at -80 °C for future use.

**3. Experimental Results**
**3.1 Effect on Mouse Body Weight:** As shown in Table 50, throughout the experimental period, the body weights of all experimental mice fluctuated within the normal range. Compared with the Normal group, 20 (500, 1000 mg/kg) had no significant effect on the body weight of either female or male C57BL/6 mice (P > 0.05).

**Table 50. Effects of Compound 20 on Mouse Body Weight**

| | | female | | | male | |
|---|---|---|---|---|---|---|
| Group | Normal | 20 (500 mg/kg) | 20 (1000 mg/kg) | Normal | 20 (500 mg/kg) | 20 (1000 mg/kg) |
| day 1 | 100.00±0.00 | 100.00±0.00 | 100.00±0.00 | 100.00±0.00 | 100.00±0.00 | 100.00±0.00 |
| day 5 | 98.37±1.00 | 100.41±0.43 | 101.96±0.55 | 99.81±2.21 | 100.31±0.89 | 101.48±1.30 |
| day 10 | 98.61±0.41 | 99.01±1.34 | 101.80±2.02 | 100.45±0.91 | 101.51±4.00 | 101.22±0.84 |
| day 15 | 101.72±2.05 | 100.03±0.95 | 99.77±2.60 | 100.59±1.24 | 99.27±2.11 | 100.47±1.28 |
| day 20 | 98.10±1.17 | 100.52±0.74 | 101.04±2.13 | 100.86±0.67 | 101.29±0.40 | 100.15±2.76 |
| day 25 | 100.21±1.81 | 99.81±1.72 | 99.06±3.56 | 100.39±0.74 | 98.93±0.60 | 98.35±1.14 |
| day 30 | 100.12±1.00 | 99.79±2.55 | 100.67±0.91 | 100.15±0.92 | 100.10±1.08 | 100.71±0.53 |

**3.2 Effects on Mouse Activity Status:** Compared with the Normal group, mice in the Compound 20 group (500, 1000 mg/kg) exhibited relatively reduced activity and lethargy after each administration, recovering to normal within 1-2 hours. Over the 30-day observation period, no significant differences were observed between the Normal group and the Compound 20 group in terms of coat luster, behavioral activity, or mental state.
**3.3 Effects on Mouse Survival Rate**

**Table 51. Effect of Compound 20 on Mouse Survival Rate**

| | female | | | male | | |
|---|---|---|---|---|---|---|
| Group | Normal | **20** (500 mg/kg) | **20** (1000 mg/kg) | Normal | **20** (500 mg/kg) | **20** (1000 mg/kg) |
| Survival rate | 100% | 100% | 100% | 100% | 100% | 100.00% |

As shown in Table 51, after consecutive administration of Compound 20 at doses of 500 mg/kg and 1000 mg/kg for 30 days, there was no significant effect on the survival rate of mice.

## Claims

1. Use of guaianolide sesquiterpene lactone derivatives or pharmaceutically acceptable salts thereof in the preparation of drugs for treating tumor or inflammatory disease or for soothing the skin; wherein the guaiacol sesquiterpene lactone derivatives have the structure shown in Formula I; 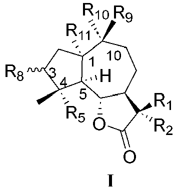
Wherein, R₁ and R₂ together form a double bond; or R₁ is hydrogen or deuterium, and R₂ is 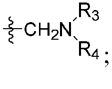 wherein R₃ and R₄ are each an alkyl group, or R₃, R₄, and the N atom to which they are attached form a 3- to 10-membered ring structure;
R₅ is selected from hydroxy, C1-C6 alkoxy, C1-C6 alkyl ester, or halogen, and - OCONR₆R₇, wherein R₆ is hydrogen or C1-C6 alkyl; R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, OH-(CH₂)n-O-substituted C1-C6 alkyl, amino-substituted C1-C6 alkyl, 5- to 6-membered nitrogen-containing heterocycle-substituted C1-C6 alkyl, or benzene ring- substituted C1-C6 alkyl; wherein the 5- to 6-membered nitrogen-containing heterocycle or benzene ring may optionally be substituted by C1-C6 alkyl; or R₆, R₇, and the N atom to which they are attached may form a 3- to 10-membered cyclic structure; or the 3rd position carbon atom may be bonded to the 4th position carbon atom to form a double bond; or the 4th position carbon atom may be bonded to the 5th position carbon atom to form a double bond;
R₈ is hydrogen or hydroxyl;
R₉ is C1-C6 alkyl, R₁₀ and R₁₁ forming a cyclopropane; or R₉ is C1-C6 alkyl, 1st position carbon atom bonded to the 10th position carbon atom forming a double bond; When R₉ is C1-C6 alkyl and the 1st position carbon atom forms a double bond with the 10th carbon atom, R₅ is not hydroxyl;
n is selected from 1, 2, 3, 4, 5, or 6;
the tumor is selected from: hepatocellular carcinoma, colorectal cancer, or lymphoma; the inflammatory disease is selected from (1) acute lung injury; (2) hepatitis; (3) sepsis caused by bacterial and/or viral infections; (4) acute or chronic kidney diseases; (5) renal fibrosis; (6) lupus nephritis; (7) diabetic nephropathy; (8) atopic dermatitis; preferably, the skin soothing is achieved by increasing skin hyaluronic acid levels and/or inhibiting mast cell degranulation.

2. The use according to claim 1, wherein the structure of the guaiacol sesquiterpene lactone derivative is as shown in Formula II; 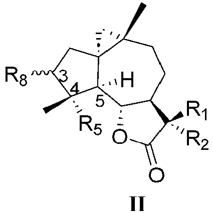
R₁ and R₂ together form a double bond;
or R₁ is hydrogen or deuterium, R₂ is 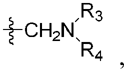
wherein R₃ and R₄ are each an alkyl group, or R₃, R₄, and N atom to which they attached form a 3- to 10-membered ring structure,
R₅ is a hydroxyl group, a C1-C6 alkoxy group, a C1-C6 alkyl ester group, or a halogen atom; or the 3rd position carbon atom is bonded to the 4th position carbon atom to form a double bond; or the 4th position carbon atom is bonded to the 5th position carbon atom to form a double bond;
R₈ is hydrogen or a hydroxyl group.

3. The use according to claim 1, wherein the structure of the guaiacol sesquiterpene lactone derivative is as shown in Formula III; 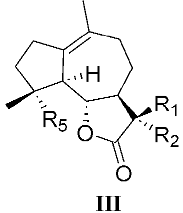
R₁ and R₂ together form a double bond;
or R₁ is hydrogen or deuterium, R₂ is 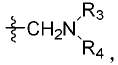 wherein R₃ and R₄ are each an alkyl group, or R₃, R₄, and N form a 3- to 10- membered ring structure;
R₅ is -OCONR₆R₇, wherein R₆ is hydrogen or C1-C6 alkyl; R₇ is C1-C6 alkyl, fluorine-substituted C1-C6 alkyl, C1-C6 cycloalkyl, hydroxy-substituted C1-C6 alkyl, C1-C6 alkoxy-substituted C1-C6 alkyl, OH-(CH₂)n-O-substituted C1-C6 alkyl, amino-substituted C 1-C6 alkyl, 5- to 6-membered nitrogen-containing heterocycle-substituted C1-C6 alkyl, or benzene ring-substituted C1-C6 alkyl; wherein the 5- to 6-membered nitrogen-containing heterocycle or benzene ring may optionally be substituted by C1-C6 alkyl; or R₆, R₇, and the N atom to which they are attached form a 3- to 10-membered cyclic structure.

4. The use according to claim 1, wherein the structure of the guaiacol sesquiterpene lactone derivative is as shown in formula IV; 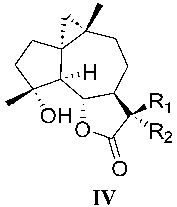
wherein R₁ and R₂ together form a double bond;
or R₁ is hydrogen or deuterium, and R₂ is 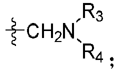 wherein R₃ and R₄ are each an alkyl group, or R₃, R₄, and the N atom to which they are attached form a 3- to 10-membered ring structure.

5. The use according to any one of claims 1 to 4, wherein R₁ and R₂ together form a double bond.

6. The use according to any one of claims 1 to 4, wherein R₁ is hydrogen, R₂ is 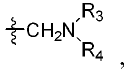 and R₃ and R₄ are each C1-C10 alkyl; preferably, R₃ and R₄ are each C1-C6 alkyl.

7. The uses according to any one of claims 1 to 4, wherein R₁ is hydrogen, R₂ is 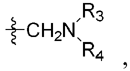 and R₃, R₄, and the N atom to which they are attached form a 4-6-membered ring structure.

8. The use according to any one of claims 1 to 4, wherein R₁ is hydrogen, R₂ is 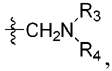 and the ring structure formed by R₃, R₄, and the N atom to which they are bonded is selected from the following structures: azetidine, tetrahydropyrrole, piperidine, piperazine, morpholine, azacyclooctane, 6-azaspiro[2,5]octane, 8-azaspiro[4,5]decane, and octahydrocyclopenta[c]pyrrole.

9. The use according to any one of claims 1 to 3, wherein R₅ is selected from hydroxyl.

10. The use according to any one of claims 1 to 3, R₅ is selected from -OCONR₆R₇, wherein R₆ is hydrogen; R₇ is 5- to 6-membered nitrogen-containing heterocycle-substituted C1-C6 alkyl ; said 5- to 6-membered nitrogen-containing heterocycle is selected from tetrahydropyrole, piperidine, piperazine, or morpholine; and optionally, the 5- to 6-membered nitrogen-containing heterocycle is substituted with C1-C6 alkyl.

11. The use according to any one of claims 1 to 3, R₅ is selected from -OCONR₆R₇, wherein R₆, R₇, and the N atom to which they are attached form a 4- to 6-membered ring structure.

12. The uses according to any one of claims 1 or 3, R₅ is selected from -OCONR₆R₇, wherein the cyclic structure formed by R₆, R₇, and the N atom to which they are bonded is selected from the following structures: azetidine, tetrahydropyrrole, piperidine, piperazine, or morpholine.

13. The use according to claim 1, wherein R₁ and R₂ together form a double bond; or R₁ is hydrogen or deuterium, and R₂ is 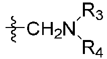 wherein R₃ and R₄ are each C1-C3 alkyl, or R₃, R₄, and N form a 5- to 6-membered ring structure, the 5- to 6-membered ring structure being selected from pyrrole, proline, piperidine, piperazine, or morpholine;
R₅ is hydroxyl, methoxy, methyl ester, halogen, or -OCONR₆R₇, wherein R₆ is hydrogen or C1-C6 alkyl; R₇ is C1-C6 alkyl, fluorine-substituted C1-C3 alkyl, cyclopropyl, hydroxyl-substituted C1-C6 alkyl, methoxy-substituted C1-C6 alkyl, dimethylaminoethyl, morpholine-substituted C1-C6 alkyl, piperazine-substituted C1-C6 alkyl, or phenyl-substituted methyl; or R₆, R₇, and the N atom form a substituted 5- or 6-membered ring structure; or a double bond formed between the 3rd position and 4th position carbon atoms; or a double bond formed between the 4th position and 5th position carbon atoms; R₈ is hydrogen or hydroxyl; R₉ is methyl, R₁₀ and R₁₁ form a cyclopropane ring; or a double bond formed between the 1st and 10th carbon atoms.

14. The use according to claim 2, R₁ and R₂ together form a double bond; or R₁ is hydrogen or deuterium, R₂ is 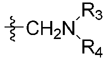 wherein R₃ and R₄ are each C1-C3 alkyl, or R₃, R₄, and N form a 5- to 6-membered ring structure, the 5- to 6-membered ring structure selected from pyrrole, proline, piperidine, piperazine, or morpholine; R₅ is hydroxyl, methoxy, methyl ester, or halogen; or the carbon atom at position 3rd is bonded to the carbon atom at position 4th to form a double bond; or the carbon atom at position 4th is bonded to the carbon atom at position 5th to form a double bond; R₈ is hydrogen or hydroxyl.

15. The use according to claim 3, R₁ and R₂ together form a double bond; or R₁ is hydrogen or deuterium, and R₂ is 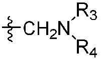 wherein R₃ and R₄ are each C1-C3 alkyl, or R₃, R₄, and N form a pyrrole; R₅ is -OCONR₆R₇, wherein R₆ is hydrogen or C1-C6 alkyl; R₇ is C1-C6 alkyl, fluorine-substituted C1-C3 alkyl, cyclopropyl, hydroxy-substituted C1-C6 alkyl, methoxy-substituted C1-C6 alkyl, dimethylaminoethyl, morpholino-substituted C1-C6 alkyl, piperazine-substituted C1-C6 alkyl, or phenyl-substituted methyl; or R₆, R₇, and the N atom form a substituted 5-6-membered ring structure.

16. The use according to claim 4, wherein R₁ and R₂ together form a double bond; or R₁ is hydrogen or deuterium, and R₂ is 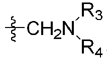 wherein R₃ and R₄ are each C1-C4 alkyl or isopropyl; or R₃, R₄, and N form a 4- to 6-membered monocyclic structure, the 4- to 6-membered monocyclic structure being selected from azetidine, pyrrole, morpholine, piperidine, piperazine, with substituents on the ring selected from ethyl, ethyl ester, morpholine, or piperidine; or R₃, R₄, and the N atom form an 8-membered cyclic structure, wherein the 8-membered cyclic structure is selected from azetinoctanes; or R₃, R₄, and the N atom form an 8-membered spiro ring structure, wherein the 8-membered spiro ring structure is selected from 6-azaspiro[2,5]octanes; or R₃, R₄, and the N atom form a 10-membered spiro ring structure, wherein the 10-membered spiro ring structure is selected from 8-azaspiro[4,5]decane; or R₃, R₄, and the N atom form an 8-membered fused ring structure, wherein the 8-membered fused ring structure is selected from octahydrocyclopenta[c]pyrrole.

17. The use according to any one of claims 1 to 16, the cyclic structure formed by R₃, R₄, and the N atom, or the cyclic structure formed by R₆, R₇, and the N atom, is substituted by a substituent selected from C1-C6 alkyl, 5- to 6-membered nitrogen-containing heterocyclic groups, and C1-C6 alkoxycarbonyl; More preferably, the 5- to 6-membered nitrogen-containing heterocyclic group is selected from tetrahydropyrolidyl, piperidyl, piperazinyl, or morpholinyl.

18. The use according to any one of claims 1 to 17, wherein the pharmaceutically acceptable salt of the guaiacol sesquiterpene lactone derivative is selected from hydrochloride, sulfate, phosphate, maleate, fumarate, or citrate.

19. The use according to any one of claims 1 to 18, wherein the guaiacol sesquiterpene lactone derivative or its pharmaceutically acceptable salt is selected from the following structures:

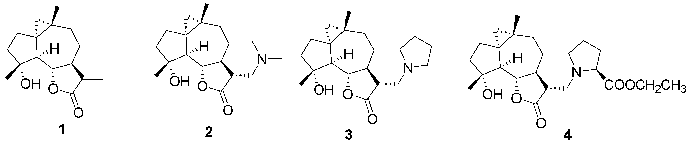

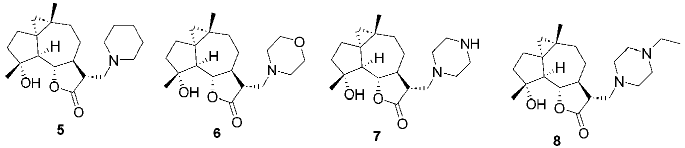

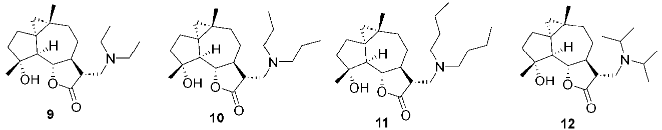

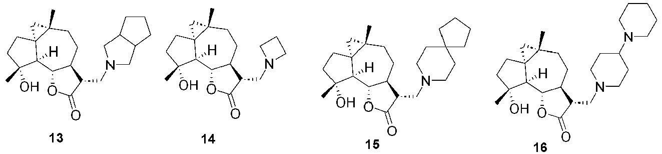

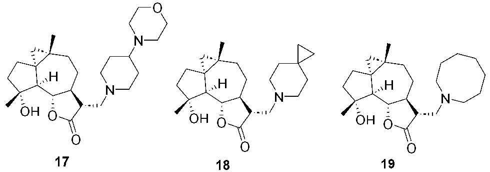

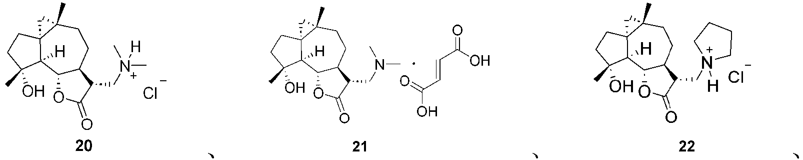

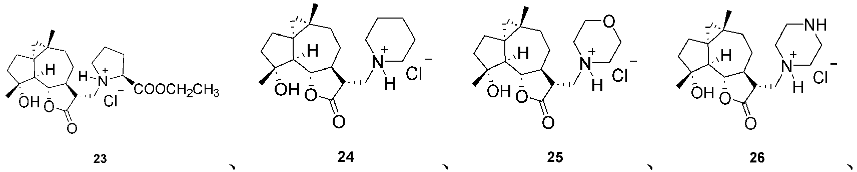

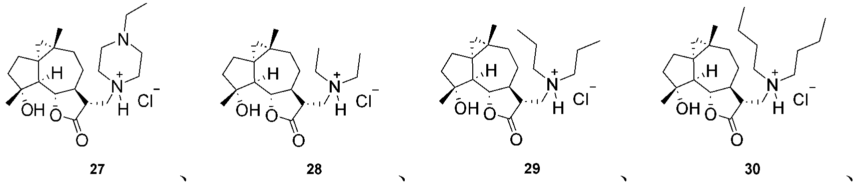

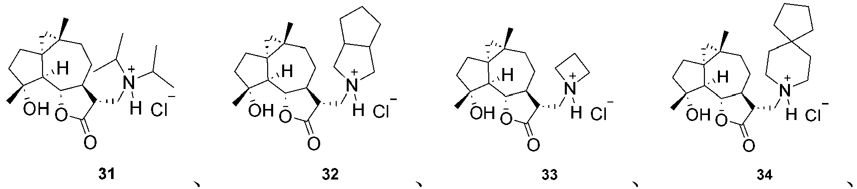

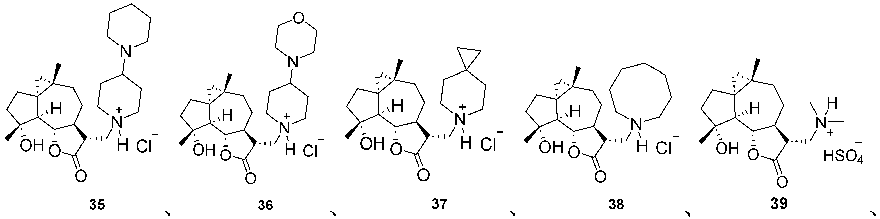

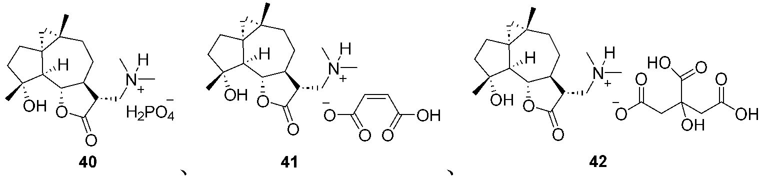

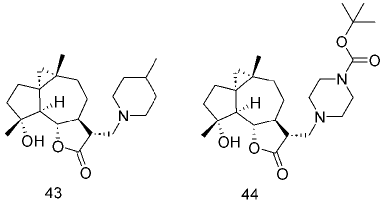

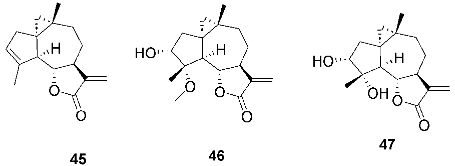

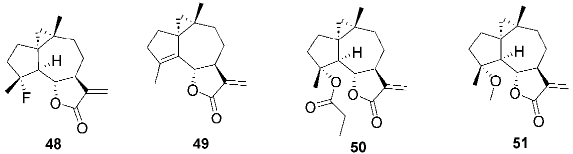

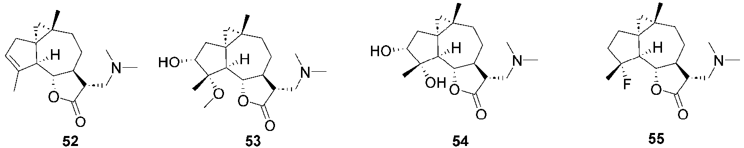

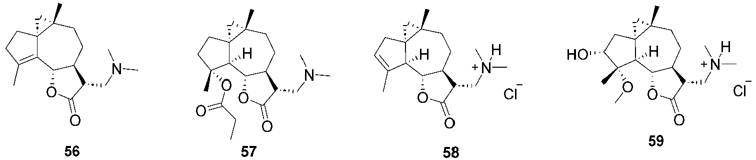

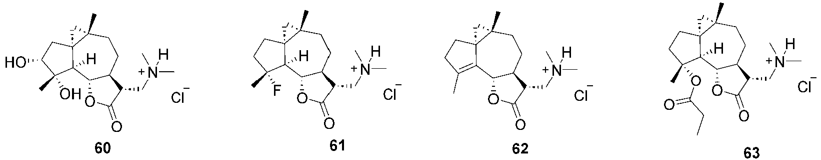

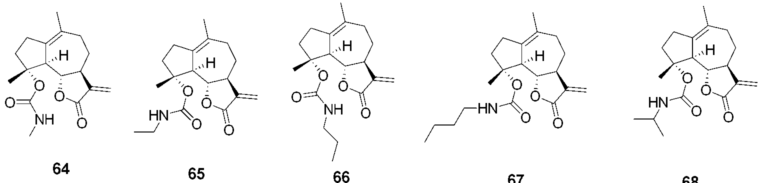

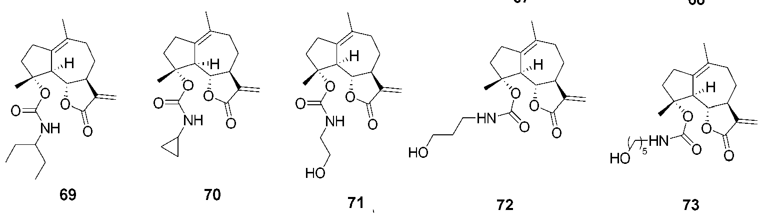

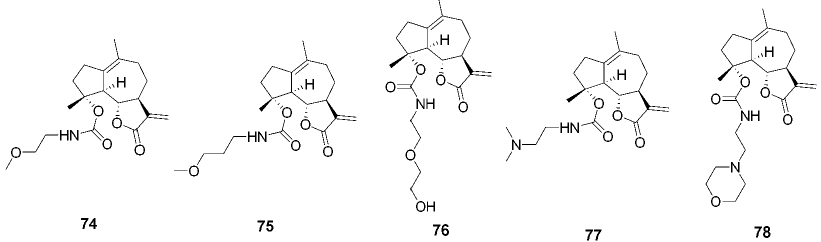

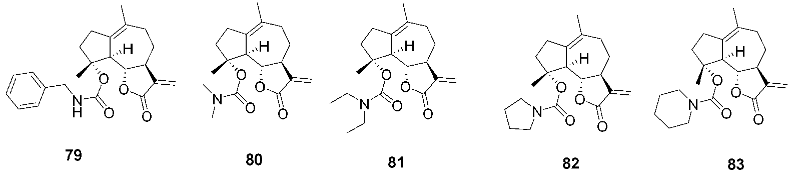

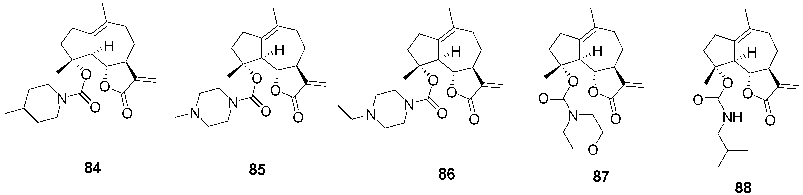

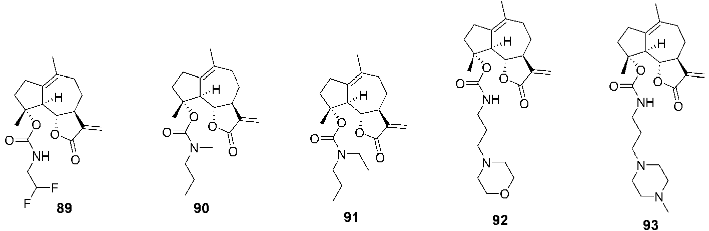

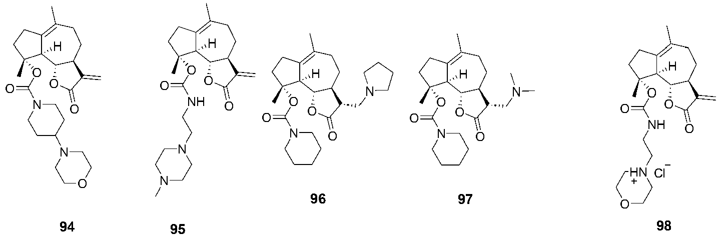

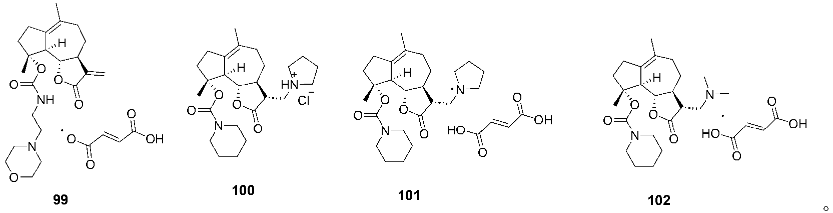

20. A pharmaceutical composition for use in the preparation of drugs for treating tumors or inflammatory diseases or for soothing the skin; said pharmaceutical composition comprises a guaiacol sesquiterpene lactone derivative as defined in claims 1 to 19 or a pharmaceutically acceptable salt thereof, and a pharmaceutically acceptable carrier; said tumors selected from: hepatocellular carcinoma, colorectal cancer, or lymphoma;the inflammatory diseases are selected from (1) acute lung injury; (2) hepatitis; (3) sepsis caused by bacterial and/or viral infections; (4) acute or chronic kidney diseases; (5) renal fibrosis; (6) lupus nephritis; (7) diabetic nephropathy; (8) atopic dermatitis.
Preferably, the soothing of the skin is achieved by increasing skin hyaluronic acid levels and/or inhibiting mast cell degranulation.

21. The use according to claim 20, the pharmaceutical composition further comprises an immune checkpoint inhibitor.

22. The use according to claim 21, wherein that the immune checkpoint inhibitor is an anti-PD-L1/PD-1 monoclonal antibody or an anti-CTLA-4 monoclonal antibody.

23. Use of guaiacol sesquiterpene lactone derivatives or pharmaceutically acceptable salts, prodrugs, solvates, or stereoisomers thereof in the preparation of drugs for treating inflammatory diseases or tumors or for soothing skin; wherein the guaiacol sesquiterpene lactone derivative is compound 1 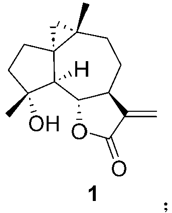
the tumors are selected from: hepatocellular carcinoma, colorectal cancer, or lymphoma; the inflammatory diseases are selected from (1) acute lung injury; (2) hepatitis; (3) sepsis caused by bacterial and/or viral infections; (4) acute or chronic kidney diseases; (5) renal fibrosis; (6) lupus nephritis; (7) diabetic nephropathy; (8) atopic dermatitis;
Preferably, the soothing of the skin is achieved by increasing skin hyaluronic acid levels and/or inhibiting mast cell degranulation.

24. The use according to claim 23, wherein the sesquiterpene lactone derivative prodrug is selected from the following structures:
